# EUROPEAN PATENT APPLICATION

(11) **EP 4 159 767 A1**
(43) Date of publication of application: **05.04.2023**
(21) Application number: 21799497.9
(22) Date of filing: 06.05.2021
(51) Int. Cl.: C07K 19/00, C12N 15/62, C12N 5/10, A61K 35/17

(54) **IMPROVED CHIMERA CONSISTING OF T-CELL RECEPTOR AND CO-STIMULATORY MOLECULES**

(30) Priority: 07.05.2020 CN 202010377726; 25.05.2020 CN 202010449454; 24.12.2020 CN 202011549176
(71) Applicant: China Immunotech (Beijing) Biotechnology Co., Ltd, Changping District, Beijing 102206 (CN); Tsinghua University, Haidian District, Beijing 100084 (CN)
(72) Inventor: WANG, Jiasheng, Beijing 100084 (CN); YU, Li, Beijing 100084 (CN); JIA, Lemei, Beijing 100084 (CN); LIU, Yue, Beijing 100084 (CN); ZHAO, Xueqiang, Beijing 102206 (CN); RUI, Wei, Beijing 102206 (CN); ZHOU, Zhixiao, Beijing 100084 (CN); LIU, Guangna, Beijing 100084 (CN); LIN, Xin, Beijing 100084 (CN)
(74) Representative: Michalski Hüttermann & Partner Patentanwälte mbB
(86) International application number: PCT/CN2021/091897
(87) International publication number: WO 2021/223707

(57) **Abstract**

The present invention relates to the field of biomedicine, to an improved T cell receptor-costimulatory molecule chimera, in particular to a T cell receptor (TCR) or TCR complex comprising a costimulatory molecule, a T cell comprising the TCR or TCR complex, and the use thereof.

## Description

### Technical Field

The present invention relates to the field of biomedicine, to an improved T cell receptor-costimulatory molecule chimera, in particular to a T cell receptor (TCR) or TCR complex comprising a costimulatory molecule, an immune cell comprising the TCR or TCR complex, and the use thereof.

### Background

Cell therapy, especially T cell-related therapy, has developed rapidly this year, among which chimeric antigen receptor T cell (CAR-T) therapy and TCR-T therapy have attracted much attention.

CAR-T therapy is based on the expression of CAR molecules in T cells. A CAR molecule consists of three parts: an ectodomain, which is an antigen recognition domain derived from an antibody and is responsible for recognizing a target antigen; a transmembrane domain; and an endodomain, which is a signal molecule and costimulatory signal molecule derived from a T cell receptor and is responsible for transducing a T cell activation signal after receiving stimulation. When CAR molecules bind to the corresponding antigens thereof, they will aggregate, start the effector function of T cells and kill target tumor cells.

TCR-T therapy is based on a T cell receptor (TCR). TCR is the identity of T cells, which can be divided into αβ T cells and γ δ T cells based on the type of TCR. In development, a T precursor cell will undergo VDJ rearrangement in TCR γ and TCR δ chains, which, if rearrangement is successful, will develop into a γδ T cell, or if rearrangement ends in failure, will undergo VDJ recombination in TCR α and TCR β chains, and then develop into a αβ T cell. αβ T cells account for 90%-95% of peripheral blood T cells, while γ δ T cells account for 5%-10% of peripheral blood T cells. The two types of T cells recognize antigens in MHC-restricted and MHC-unrestricted ways, respectively, which play an important role in the immunity to pathogens and tumors.

A T cell receptor (TCR) complex molecule contains multiple chains, in which TCR α and TCR β chains (or TCR γ and TCR δ chains) are responsible for recognizing MHC-polypeptide molecules, and the other six CD3 subunits bind to TCR α/β chains (or TCR γ/δ chains) to play the role of signal transduction. The natural TCR complex contains ten ITAM signal sequences, which can transmit stronger signals than CAR in theory. By employing the signal transduction function of natural TCR, it is possible to construct a new receptor to alleviate T cell disability, which can play a better anti-solid tumor role. The ectodomain of TCR is very similar to the Fab domain of an antibody, so the variable region sequence of TCR can be replaced by a variable region sequence of an antibody, so as to obtain a Synthetic TCR and Antigen Receptor (STAR), which not only has antibody specificity, but also has superior signal transduction function of a natural TCR on mediating T cell activation.

However, STAR-T derived from the natural TCR lacks the costimulatory signal in T cell activation, and its proliferation and activation ability are often affected. Therefore, an improved TCR and corresponding TCR-T therapy are still needed in this field.

### Brief Description of the Drawings

Fig. 1 Schematic diagram of optimization of STAR by constant region cysteine modification, transmembrane domain and endodomain modification.
Fig. 2 Schematic diagram of optimization of STAR by adding costimulatory molecule receptor endodomains to α and/or β chains.
Fig. 3 Schematic diagram of optimization of STAR by adding costimulatory molecule receptor endodomain directly or via a linker after deletion of α and/or β chain endodomains.
Fig. 4 Schematic diagram of optimization of STAR by adding costimulatory molecule receptor endodomains to CD3 subunits.
Fig. 5 Schematic diagram of optimization of STAR by adding cytokine receptor signaling transduction domains to α and/or β chains.
Fig. 6 Comparison of tumor target cell-killing ability between WT-STAR T cells and mut-STAR T cells.
Fig. 7 Effects of different costimulatory receptor endodomains on target-killing ability of STAR-T cells at different effector to target (E: T) ratios.
Fig. 8 Effects of different costimulatory receptor endodomains on target-killing ability of STAR-T cells at different co-culture time.
Fig. 9 Effects of OX40 endodomain added to α chain, β chain, α chain and β chain on target-killing ability of STAR-T cells at different E:T ratios.
Fig. 10 Effects of OX40 endodomain added to α chain, β chain, α chain and β chain on target-killing ability of STAR-T cells at different co-culture time.
Fig. 11 Effects of different costimulatory receptor endodomains on cytokine secretion of STAR-T cells.
Fig. 12 Effects of different costimulatory receptor endodomains on the proliferation of STAR-T cells.
Fig. 13 Effects of OX40 endodomain added to α chain, β chain, α chain and β chain on target-killing ability of STAR-T cells.
Fig. 14 Tumor-killing ability of mut-STAR with different costimulatory receptor endodomains connected to TCR α chain.
Fig. 15 Effects of different costimulatory receptor endodomains added to different CD3 subunits on target-killing ability of STAR-T cells.
Fig. 16 Effects of different costimulatory receptor endodomains added to different CD3 subunits on the proliferation of STAR-T cells.
Fig. 17 Effects of OX40 endodomain linked to endodomain-deleted α chain via different G4S linkers on target-killing ability of STAR-T cells.
Fig. 18 Effects of OX40 endodomain linked to endodomain-deleted β chain via different G4S linkers on target-killing ability of STAR-T cells.
Fig. 19 Effects of OX40 endodomain linked to endodomain-deleted α and/or β chains via different G4S linkers on IL-2 secretion of STAR-T cells.
Fig. 20 Effects of OX40 endodomain linked to endodomain-deleted α and/or β chains via different G4S linkers on IFN-γ secretion of STAR-T cells.
Fig. 21 Effects of OX40 endodomain linked to endodomain-deleted α chains via different G4S linkers on central memory T cell differentiation.
Fig. 22 Effects of OX40 endodomain linked to endodomain-deleted α chains via different G4S linkers on T cell differentiation.
Fig. 23 Effects of lysine modification in the transmembrane domain or endodomain on target-killing ability of STAR-T cells.
Fig. 24 Effects of lysine modification in the transmembrane domain or endodomain on IFN-y secretion of STAR-T cells.
Fig. 25 Effects of lysine modification in the transmembrane domain or endodomain on IL-2 secretion of STAR-T cells.
Fig. 26 Effects of lysine modification in the transmembrane domain or endodomain on central memory T cell differentiation.
Fig. 27 Effects of lysine modification in the transmembrane domain or endodomain on T cell differentiation.
Fig. 28 Effects of different cytokine receptor signaling transduction domains linked to α and/or β chains on target-killing ability of STAR-T cells.
Fig. 29 Comparison of the killing effects between mutant STAR with different cytokine receptor stimulatory domains connected and α-del-(G4S) 3-OX40-STAR.
Fig. 30 Effects of different cytokine receptor signaling transduction domains linked to α and/or β chains on IL-2 secretion of STAR-T cells.
Fig. 31 Effects of different cytokine receptor signaling transduction domains linked to α and/or β chains on IFN-γ secretion of STAR-T cells.
Fig. 32 Effects of different cytokine receptor signaling transduction domains linked to α and/or β chains on central memory T cell differentiation.
Fig. 33 Effects of different cytokine receptor signaling transduction domains linked to α and/or β chains on T cell differentiation.
Fig. 34 Anti-tumor in vivo effects of αβ OX40-STAR T, mut-STAR T and CAR-T in a mouse tumor model in vivo.
Fig. 35 Survival curves of mice administered with αβ OX40-STAR T, mut-STAR T and CAR-T.
Fig. 36 Proliferation in vivo of αβ OX40-STAR T, mut-STAR T and CAR-T in mice.
Fig. 37 Anti-tumor in vivo effects of different STAR structures and CAR-T in a mouse tumor model.
Fig. 38 pattern diagram of αβ TCR and constant region mutant.
Fig. 39 Exemplary pattern diagram of armored-TCR.
Fig. 40 Exemplary pattern diagram of armored-CD3.
Fig. 41 Diagram of proliferation and killing ability of armored-TCR T cells with costimulatory endodomain integrated in different TCR constant regions under long-term stimulation by excessive target cells in vitro.
Fig. 42 Diagram of proliferation and killing ability of armored-TCR T cells modified by different costimulatory domains under long-term stimulation by excessive target cells in vitro. A: E141-TCR; B: E315-TCR.
Fig. 43 Diagram of proliferation and killing ability of (G4S)n linker-containing armored-TCR T cells with endodomain of TCR molecule deleted or not under long-term stimulation by excessive target cells in vitro.
Fig. 44 Diagram of proliferation and killing ability of armored-TCR T cells with TCR α chain, TCR β chain, or TCR α and β chains connected to 4-1BB endodomain under long-term stimulation by excessive target cells in vitro. A: E141-TCR; B: E315-TCR.
Fig. 45 Diagram of proliferation and killing ability of armored-TCR T cells with TCR α chain, TCR β chain, or TCR α and β chains connected to OX40 endodomain under long-term stimulation by excessive target cells in vitro. A: E141-TCR; B: E315-TCR.
Fig. 46 Effects of OX40 and CD40 intracellular domains linked by G4S linker on TCR-T cell proliferation and target cell killing ability with or without deletion of TCR α, αβ or addition to CD3 δ molecules under long-term stimulation by excessive target cells in vitro.
Fig. 47 Experimental scheme of mouse tumor model for evaluation of armored-TCR T cell function and in vivo imaging results of tumor progression.
Fig. 48 Statistical graph of proliferation of human TCR T cells in mice.
Fig. 49. Survival curve of tumor-bearing mice after reinfusion of armored-TCR T cells.
Fig. 50 Diagram of proliferation and killing ability of TCR T cells expressing armored-CD3 molecules under long-term stimulation by excessive target cells in vitro.
Fig. 51 Detection of killing ability of TCR T cells expressing armored-CD3 molecules after 7 days of excessive target cell stimulation in vitro.
Fig. 52 IFN γ release from TCR T cells expressing armored-CD3 molecules after 7 days of excessive target cell stimulation in vitro.
Fig. 53 Experimental scheme of mouse tumor model for evaluation of function of TCR T cells expressing armored-CD3 molecules and in vivo imaging results of tumor progression.
Fig. 54 Statistical graph of proliferation of TCR T cells expressing human armored-CD3 in mice.
Fig. 55. Survival curve of tumor-bearing mice after reinfusion of armored-TCR T cells.
Fig. 56. Effects on killing ability of γ δ TCR T cells with OX40 endodomain directly linked to the constant region C-terminal of TCR γ chain, TCR δ chain, TCR γ and δ chains, and directly linked to the C-terminal of TCR γ and δ chains via G4S linker, or linked to the C-terminal of TCR γ and δ chains with intracellular amino acid deleted.
Fig. 57 Effects on IFN γ secretion of γ δ TCR T cells with OX40 endodomain directly linked to the constant region C-terminal of TCR γ chain, TCR δ chain, TCR γ and δ chains, and directly linked to the C-terminal of TCR γ and δ chains via G4S linker, or linked to the C-terminal of TCR γ and δ chains with intracellular amino acid deleted.
Fig. 58 Schematic diagram of optimization of STAR by constant region cysteine modification, transmembrane domain and N-terminal rearrangement.
Fig. 59 Example of connection position between a costimulatory molecule domain and a STAR structure. Only connection to α chain is shown.
Fig. 60 Example of STAR structure comprising a costimulatory molecule domain.
Fig. 61 Killing ability of STAR and STAR comprising a costimulatory factor.
Fig. 62 Nuclear RelB level related to proliferation signal of STAR and STARs comprising costimulatory factors.
Fig. 63 Results of anti- CD19 for different STARs
Fig. 64 Results of anti- CD19 and CD 20 for different STARs

### Summary of the Invention

Unless otherwise indicated or defined, all used terms have the common meaning in the field, which will be understood by those skilled in the field. See, for example, the standard manual, such as Sambrook et al., "Molecular cloning: a laboratory manual"; Lewin, "Genes VIII", and Roitt et al., "Immunology" (8nd edition), and the general prior art cited herein; in addition, unless otherwise stated, all the methods, steps, techniques and operations that are not specifically detailed may and have been performed in a manner known per se, which will be understood by those skilled in the art. Also see, for example, the standard manual, the above general prior art and other references cited therein.

As used herein, the term "and/or" encompasses all combinations of items connected by the term and shall be deemed to have been separately listed herein. For example, "A and/or B" covers "A", "A and B", and "B". For example, "A, B and/or C" covers "A", "B", "C", "A and B", "A and C", "B and C", and "A and B and C".

The term "comprising" is used herein to describe a sequence of a protein or nucleic acid, which may consist of said sequence, or may have additional amino acids or nucleotides at one or both ends of said protein or nucleic acid but still possess the activity described herein. In addition, those skilled in the art will understand that methionine encoded by the start codon at the N-terminal of a polypeptide is retained in certain practical situations (e.g. when expressed in a particular expression system), but does not substantially affect the function of the polypeptide. Thus, in the description of a specific polypeptide amino acid sequence, while it may not contain methionine encoded by the start codon at its N-terminal, but still covers a sequence comprising the methionine by the time, and correspondingly, the coding nucleotide sequences thereof may also contain the start codon; and vice versa.

As used herein, "the amino acid number being made reference to SEQ ID NO: x" (SEQ ID NO: x is a specific sequence listed herein) means that the position number of the particular amino acid described is the position number of the amino acid corresponding to that amino acid on SEQ ID NO: x. The amino acid correspondence between different amino acid sequences can be determined by sequence alignment methods known in the art. For example, the amino acid correspondence can be determined by an EMBL-EBI online alignment tool (https://www.ebi.ac.uk/Tools/psa/), in which two sequences can be aligned using Needleman-Wunsch algorithm with default parameters. For example, if alanine at position 46 starting from the N-terminal of a polypeptide is aligned in sequence alignment with an amino acid at position 48 of SEQ ID NO: x, then the amino acid in the polypeptide may also be described herein as "alanine at position 48 of the polypeptide, the amino acid position being made reference to SEQ ID NO: x". In the present invention, reference is made to SEQ ID NO: 3 for the amino acid position related to α chain constant region. In the present invention, reference is made to SEQ ID NO: 4 for the amino acid position related to β chain constant region.

In one aspect, a modified T cell receptor (TCR) complex is provided herein, wherein, the TCR may be αβ TCR, the αβ TCR complex comprising a TCR α chain, TCR β chain, CD3 ε, CD3 γ, CD3 δ and CD3 ζ; wherein at least one functional domain is connected to the C-terminal of at least one of TCR α chain, TCR β chain, CD3 ε, CD3 γ, CD3 δ and CD3 ζ; wherein, the TCR α chain comprises a first constant region, and the TCR β chain comprises a second constant region; or
the TCR may be a γ δ TCR, the γ δ TCR complex comprising a TCR γ chain, TCR δ chain, CD3 ε, CD3 γ, CD3 δ and CD3 ζ; wherein at least one functional domain is connected to the C-terminal of at least one of TCR γ chain, TCR δ chain, CD3 ε, CD3 γ, CD3 δ and CD3 ζ; wherein the TCR γ chain comprises a first constant region, and the TCR δ chain comprises a second constant region.

In some embodiments, the TCR α chain further comprises a first target binding region. In some embodiments, the TCR β chain further comprises a second target binding region. In some embodiments, the TCR α chain further comprises a first target binding region, and the TCR β chain further comprises a second target binding region.

In some embodiments, the TCR γ chain further comprises a first target binding region. In some embodiments, the TCR δ chain further comprises a second target binding region. In some embodiments, the TCR γ chain further comprises a first target binding region, and the TCR δ chain further comprises a second target binding region.

Generally, in TCR, the target binding region is located at the N-terminal of the constant region, both of which can be connected directly or via a linker.

In one aspect, a modified T cell receptor (TCR) is provided herein, wherein, the TCR may be a αβ TCR comprising TCR α and β chains, wherein at least one functional domain is connected to the C-terminal of the TCR α chain and/or β chain; wherein, the TCR α chain comprises a first constant region, and the TCR β chain comprises a second constant region; or
the TCR may be a γδ TCR comprising TCR γ and δ chains, wherein at least one functional domain is connected to the C-terminal of the TCR γ chain and/or δ chain; wherein, the TCR γ chain comprises a first constant region, and the TCR δ chain comprises a second constant region.

In some embodiments, the TCR α chain further comprises a first target binding region. In some embodiments, the TCR β chain further comprises a second target binding region. In some embodiments, the TCR α chain further comprises a first target binding region, and the TCR β chain further comprises a second target binding region.

In some embodiments, the TCR γ chain further comprises a first target binding region. In some embodiments, the TCR δ chain further comprises a second target binding region. In some embodiments, the TCR γ chain further comprises a first target binding region, and the TCR δ chain further comprises a second target binding region.

In some embodiments, wherein the natural endodomain of at least one of TCR α chain, TCR β chain, CD3 ε, CD3 γ, CD3 δ and CD3 ζ in the αβ TCR complex is deleted, or wherein the natural endodomain of at least one of TCR γ chain, TCR δ chain, CD3 ε, CD3 γ, CD3 δ and CD3 ζ in the γ δ TCR complex is deleted.

In some embodiments, wherein the natural endodomain of the TCR α chain and/or TCR β chain in the αβ TCR is deleted, or the natural endodomain of the TCR γ chain and/or TCR δ chain in the γδ TCR complex is deleted.

In some embodiments, wherein in the αβ TCR complex, the functional domain is connected directly or via a linker to the C-terminal of at least one of TCR α chain, TCR β chain, CD3 ε, CD3 γ, CD3 δ and CD3 ζ in which the natural endodomain is deleted.

In some embodiments, wherein in the αβ TCR complex, the functional domain is connected directly or via a linker to the C-terminal of the TCR α chain and TCR β chain with the natural endodomain deleted.

In some embodiments, wherein in the γδ TCR complex, the functional domain is connected directly or via a linker to the C-terminal of at least one of TCR α chain, TCR β chain, CD3 ε, CD3 γ, CD3 δ and CD3 ζ in which the natural endodomain is deleted.

In some embodiments, wherein in the γδ TCR complex, the functional domain is connected directly or via a linker to the C-terminal of at least one of the TCR γ chain, TCR δ chain in which the natural endodomain is deleted.

In some embodiments, wherein the linker is (G₄S)n, where n represents an integer from 1 to 10. Preferably n is an integer from 1 to 6, more preferably n is an integer from 2 to 5, and most preferably n is 3.

In some embodiments, at least one functional domain is connected to the C-terminal of one of TCR α chain, TCR β chain, CD3 ε, CD3 γ, CD3 δ and CD3 ζ in the αβ TCR complex.

In some embodiments, at least one functional domain is connected to the C-terminal of TCR α chain and/or TCR β chain in the αβ TCR.

In some embodiments, at least one functional domain is connected to the C-terminal of one of TCR y chain, TCR δ chain, CD3 ε, CD3 γ, CD3 δ and CD3 ζ in the y8 TCR complex.

In some embodiments, at least one functional domain is connected to the C-terminal of TCR γ chain and/or TCR δ chain in the γδ TCR.

In some embodiments, CD3 δ, CD3 γ, CD3 ε and CD3 ζ in the TCR complex do not comprise at least one functional domain additionally connected to the C-terminal thereof.

In some embodiments, at least one functional domain is connected to the C-terminal of TCR α chain in the αβ TCR complex.

In some embodiments, at least one functional domain is connected to the C-terminal of TCR α chain in the αβ TCR.

In some embodiments, the natural endodomain of the TCR α chain is deleted.

In some embodiments, the functional domain is connected directly or via a linker to the C-terminal of the TCR α chain in which the natural endodomain is deleted. In some embodiments, the linker is (G₄S)n, where n represents an integer from 1 to 10, preferably 1 to 6, more preferably 2 to 5, and most preferably, n is 3.

In some embodiments, at least one functional domain is connected to the C-terminal of TCR β chain in the αβ TCR complex.

In some embodiments, at least one functional domain is connected to the C-terminal of TCR β chain in the αβ TCR.

In some embodiments, the natural endodomain of the TCR β chain is deleted.

In some embodiments, the functional domain is connected directly or via a linker to the C-terminal of the TCR β chain in which the natural endodomain is deleted. In some embodiments, the linker is (G₄S)n, where n represents an integer from 1 to 10, preferably 1 to 6, more preferably 2 to 5, and most preferably, n is 3.

In some embodiments, at least one functional domain is connected to the C-terminal of TCR γ chain in the γδ TCR complex.

In some embodiments, at least one functional domain is connected to the C-terminal of TCR γ chain in the γδ TCR.

In some embodiments, the natural endodomain of the TCR γ chain is deleted.

In some embodiments, the functional domain is connected directly or via a linker to the C-terminal of the TCR γ chain in which the natural endodomain is deleted. In some embodiments, the linker is (G₄S)n, where n represents an integer from 1 to 10, preferably 1 to 6, more preferably 2 to 5, and most preferably, n is 3.

In some embodiments, at least one functional domain is connected to the C-terminal of TCR δ chain in the y8 TCR complex.

In some embodiments, at least one functional domain is connected to the C-terminal of TCR δ chain in the γδ TCR.

In some embodiments, the natural endodomain of the TCR δ chain is deleted.

In some embodiments, the functional domain is connected directly or via a linker to the C-terminal of the TCR δ chain in which the natural endodomain is deleted. In some embodiments, the linker is (G₄S)n, where n represents an integer from 1 to 10, preferably 1 to 6, more preferably 2 to 5, and most preferably, n is 3.

In some embodiments, at least one functional domain is connected to the C-terminals of two of TCR α chain, TCR β chain, CD3 ε, CD3 γ, CD3 δ and CD3 ζ in the αβ TCR complex.

In some embodiments, at least one functional domain is connected to the C-terminals of two of TCR γ chain, TCR δ chain, CD3 ε, CD3 γ, CD3 δ and CD3 ζ in the y8 TCR complex.

In some embodiments, at least one functional domain is connected to the respective C-terminals of TCR α chain and TCR β chain in the αβ TCR complex.

In some embodiments, at least one functional domain is connected to the respective C-terminals of TCR α chain and TCR β chain in the αβ TCR.

In some embodiments, the natural endodomain of each of the TCRα chain and TCR β chain is deleted.

In some embodiments, the functional domain is connected directly or via a linker to the C-terminal of each of the TCR α chain and TCR β chain in which the natural endodomain is deleted. In some embodiments, the linker is (G₄S)n, where n represents an integer from 1 to 10, preferably 1 to 6, more preferably 2 to 5, and most preferably, n is 3.

In some embodiments, at least one functional domain is connected to the respective C-terminals of TCR y chain and TCR δ chain in the y8 TCR complex.

In some embodiments, at least one functional domain is connected to the respective C-terminals of TCR γ chain and TCR δ chain in the γδ TCR.

In some embodiments, the natural endodomain of each of the TCR γ chain and TCR δ chain is deleted.

In some embodiments, the functional domain is connected directly or via a linker to the C-terminal of each of the TCR γ chain and TCR δ chain in which the natural endodomain is deleted. In some embodiments, the linker is (G₄S)n, where n represents an integer from 1 to 10, preferably 1 to 6, more preferably 2 to 5, and most preferably, n is 3.

In some embodiments, two or more of TCR α chain, TCR β chain, CD3 ε, CD3 γ, CD3 δ and CD3 ζ in the αβ TCR complex are connected with the same or different functional domain.

In some embodiments, the TCR α chain and/or TCR β chain in the αβ TCR is connected to the same or different functional domain.

In some embodiments, two or more of TCR γ chain, TCR δ chain, CD3 ε, CD3 γ, CD3 δ and CD3 ζ in the γδ TCR complex are connected to the same or different functional domain.

In some embodiments, the TCR γ chain and/or TCR δ chain in the γδ TCR are connected to the same or different functional domain.

In some embodiments, 1, 2, 3, 4, 5, 6, 7, 8, 9, 10 or more functional domains are connected to the C-terminal of at least one of TCR α chain, TCR β chain, CD3 ε, CD3 γ, CD3 δ and CD3 ζ in the αβ TCR complex.

In some embodiments, 1, 2, 3, 4, 5, 6, 7, 8, 9, 10 or more functional domains are connected to the C-terminal of TCR α chain and/or TCR β chain in the αβ TCR.

In some embodiments, 1, 2, 3, 4, 5, 6, 7, 8, 9, 10 or more functional domains are connected to the C-terminal of at least one of TCR γ chain, TCR δ chain, CD3 ε, CD3 γ, CD3 δ and CD3 ζ in the y8 TCR complex.

In some embodiments, 1, 2, 3, 4, 5, 6, 7, 8, 9, 10 or more functional domains are connected to the C-terminal of the TCR γ chain and/or TCR δ chain in the γδ TCR.

In some embodiments, at least one functional domain, such as a costimulatory molecule endodomain, is connected to the C-terminal of 1, 2, 3, 4, 5 or 6 of TCR α chain, TCR β chain, CD3 ε, CD3 γ, CD3 δ and CD3 ζ in the complex.

In some preferred embodiments, at least one functional domain, such as a costimulatory molecule endodomain is connected to the C-terminal of one of TCR α chain, TCR β chain, CD3 ε, CD3 γ, CD3 δ and CD3 ζ in the complex.

For example, in some embodiments, at least one functional domain, such as a costimulatory molecule endodomain, is connected to the C-terminal of the TCR α chain in the complex. In some preferred embodiments, the costimulatory molecule endodomain is OX40 or ICOS. In some embodiments, the TCR β chain, CD3 δ, CD3 γ, CD3 ε and CD3 ζ do not contain the at least one functional domain, such as a costimulatory molecule endodomain, additionally connected to the C-terminal thereof.

Alternatively, in some embodiments, at least one functional domain, such as a costimulatory molecule endodomain, is connected to the C-terminal of CD3δ in the complex. In some embodiments, TCR α, TCR β, CD3 γ, CD3 ε and CD3 ζ do not contain the at least one functional domain, such as a costimulatory molecule endodomain, connected to the C-terminal thereof.

In some preferred embodiments, at least one functional domain, such as a costimulatory molecule endodomain, is connected to the C-terminals of two of TCR α chain, TCR β chain, CD3 ε, CD3 γ, CD3 δ and CD3 ζ in the complex.

For example, in some embodiments, at least one functional domain, such as a costimulatory molecule endodomain, is connected to the C-terminals of the TCR α chain and TCR β chain in the complex. In some preferred embodiments, the costimulatory molecule endodomain is OX40 or ICOS. In some embodiments, CD3 δ, CD3 γ, CD3 ε and CD3 ζ do not contain the at least one functional domain, such as a costimulatory molecule endodomain, additionally connected to the C-terminal thereof.

In some embodiments, the functional domain is an exogenous functional domain. In some embodiments, the functional domain is an exogenous endodomain, such as a domain which is responsible for intracellular transduction function.

As used herein, "exogenous" means a protein or nucleic acid sequence derived from a foreign species, or, if derived from the same species, means a protein or nucleic acid sequence that has undergone significant changes in composition and/or position from its natural form by deliberate human intervention.

As used herein, a "functional domain" is selected from the endodomain of a costimulatory molecule such as CD40, OX40, ICOS, CD28, 4-1BB, CD27, and CD137; or the endodomain of a co-inhibitory molecule such as TIM3, PD1, CTLA4, and LAG3; or the endodomain of a cytokine receptor such as an interleukin receptor (such as IL-2 receptor, IL-7α receptor, or IL-21 receptor), an interferon receptor, a tumor necrosis factor superfamily receptor, a colony-stimulating factor receptor, a chemokine receptor, a growth factor receptor, or other membrane proteins, or the domain of an intracellular protein such as NIK. The functional domain may also be the fusion of a cytokine receptor endodomain with a human STATS activation moiety (the amino acid sequence shown in SEQ ID NO: 35) either directly or via a linker (e.g. (GaS) n, where n represents an integer from 1 to 10).

In some preferred embodiments, the functional domain is a costimulatory molecule endodomain, preferably OX40 or ICOS endodomain, and more preferably OX40 endodomain.

An exemplary CD40 endodomain contains the amino acid sequence shown in SEQ ID NO: 10. An exemplary OX40 endodomain contains the amino acid sequence shown in SEQ ID NO: 11. An exemplary ICOS endodomain contains the amino acid sequence shown in SEQ ID NO: 12. An exemplary CD28 endodomain contains the amino acid sequence shown in SEQ ID NO: 13. An exemplary 4-1BB endodomain contains the amino acid sequence shown in SEQ ID NO: 14. An exemplary CD27 endodomain contains the amino acid sequence shown in SEQ ID NO: 15. An exemplary IL-2β receptor endodomain contains the amino acid sequence shown in SEQ ID NO: 32. An exemplary IL-17α receptor endodomain contains the amino acid sequence shown in SEQ ID NO: 33. An exemplary IL-21 receptor endodomain contains the amino acid sequence shown in SEQ ID NO: 34. An exemplary fusion amino acid sequence of IL-2β receptor endodomain with a human STATS activation moiety is shown in SEQ ID NO: 36. An exemplary fusion amino acid sequence of IL-17α receptor endodomain with a human STAT5 activation moiety is shown in SEQ ID NO: 37.

In some embodiments, the first constant region is a native TCR α chain constant region, for example, a native human TCR α chain constant region (an exemplary human TCR α chain constant region amino acid sequence is shown in SEQ ID NO: 1) or a native mouse TCR α chain constant region (an exemplary mouse TCR α chain constant region amino acid sequence is shown in SEQ ID NO: 3); or the first constant region is a native TCR y chain constant region, for example, a native human TCR y chain constant region (an exemplary human TCR γ chain constant region amino acid sequence is shown in SEQ ID NO: 58) or a native mouse TCR γ chain constant region (an exemplary mouse TCR γ chain constant region amino acid sequence is shown in SEQ ID NO: 59).

In some embodiments, the first constant region is a modified TCR α chain constant region or a modified TCR γ chain constant region.

In some embodiments, the modified TCR α chain constant region is derived from the mouse TCR α chain constant region, in which the amino acid at position 48, such as threonine (T), is mutated to cysteine (C) as compared to the wild-type mouse TCR α chain constant region.

In some embodiments, the modified TCR α chain constant region is derived from the mouse TCR α chain constant region, in which, the amino acid at position 112, such as serine (S), is mutated to leucine (L), the amino acid at position 114, such as methionine (M), is mutated to isoleucine (I), and the amino acid at position 115, such as glycine (G), is mutated to valine(V), as compared to the wild-type mouse TCR α chain constant region.

In some embodiments, the modified TCR α chain constant region is derived from a mouse TCR α chain constant region, in which the amino acid (e.g. E) at position 6 is substituted by D, K at position 13 is substituted by R, and the amino acids at positions 15 to 18 are deleted, as compared to the wild-type mouse TCR α chain constant region.

In some embodiments, the modified TCR α chain constant region is derived from the mouse TCR α chain constant region, in which the amino acid at position 48, such as threonine (T), is mutated to cysteine (C), the amino acid at position 112, such as serine (S), is mutated to leucine (L), the amino acid at position 114, such as methionine (M), is mutated to isoleucine (I), and the amino acid at position 115, such as glycine (G), is mutated to valine(V), as compared to the wild-type mouse TCR α chain constant region.

In some embodiments, the modified TCR α chain constant region is derived from the mouse TCR α chain constant region, in which the amino acid (e.g. E) at position 6 is substituted by D, K at position 13 is substituted by R, the amino acids at positions 15 to 18 are deleted, the amino acid at position 48, such as threonine (T), is mutated to cysteine (C), the amino acid at position 112, such as serine (S), is mutated to leucine (L), the amino acid at position 114, such as methionine (M), is mutated to isoleucine (I), and the amino acid at position 115, such as glycine (G), is mutated to valine(V), as compared to the wild-type mouse TCR α chain constant region.

In some embodiments, the modified TCR α chain constant region is derived from a mouse TCR α chain constant region, in which the constant region endodomain is deleted, for example, amino acids at position 136-137 are deleted, as compared to the wild-type mouse TCR α chain constant region.

In some embodiments, the first constant region comprises the amino acid sequence shown in one of SEQ ID Nos: 1, 3, 5, 7, 8, 26, 41, 42, and 56.

In some embodiments, the second constant region is a native TCR β chain constant region, for example, a native human TCR β chain constant region (an exemplary human TCR β chain constant region amino acid sequence is shown in SEQ ID NO: 2) or a native mouse TCR β chain constant region (an exemplary mouse TCR β chain constant region amino acid sequence is shown in SEQ ID NO: 4); or the second constant region is a native TCR δ chain constant region, for example, a native human TCR δ chain constant region (an exemplary human TCR δ chain constant region amino acid sequence is shown in SEQ ID NO: 60) or a native mouse TCR δ chain constant region (an exemplary mouse TCR δ chain constant region amino acid sequence is shown in SEQ ID NO: 61).

In some embodiments, the second constant region is a modified TCR β chain constant region ; or a modified TCR δ chain constant region.

In some embodiments, the modified TCR β chain constant region is derived from the mouse TCR β chain constant region, in which the amino acid at position 56, such as threonine (S), is mutated to cysteine (C) as compared to the wild-type mouse TCR β chain constant region.

In some embodiments, the modified TCR β chain constant region is derived from the mouse TCR β chain constant region, in which the amino acid (e.g. R) at position 6 is substituted by K, the amino acid (e.g. T) at position 6 is substituted by F, K at position 9 is substituted by E, S at position 11 is substituted by A, L at position 12 is substituted by V, and the amino acids at positions 17 and 21 to 25 are deleted, as compared to the wild-type mouse TCR β chain constant region.

In some embodiments, the modified TCR β chain constant region is derived from the mouse TCR β chain constant region, in which the amino acid at position 56, such as serine (S), is mutated to cysteine (C), the amino acid (e.g. R) at position 3 is substituted by K, the amino acid (e.g. T) at position 6 is substituted by F, K at position 9 is substituted by E, S at position 11 is substituted by A, L at position 12 is substituted by V, and the amino acids at positions 17 and 21 to 25 are deleted, as compared to the wild-type mouse TCR β chain constant region.

In some embodiments, the modified TCR β chain constant region is derived from the mouse TCR β chain constant region, in which the constant region endodomain is deleted, for example, amino acids at position 167-172 are deleted, as compared to the wild-type mouse TCR β chain constant region.

In some embodiments, the modified TCR β chain constant region comprises the amino acid sequence shown in one of SEQ ID Nos: 2, 4, 6, 9, 27, 43, and 57.

As used herein, a "target binding region" refers to a domain capable of binding (preferably specifically binding) to a target molecule. In some embodiments, the target is an antigen. Therefore, in some embodiments, the target binding region is an "antigen-binding region".

In some embodiments, the target binding region (preferred antigen-binding region) alone or in combination with another target binding region (preferred antigen-binding region) may specifically bind to a target molecule (preferred target antigen).

In some embodiments, the first target binding region and the second target binding region are combined each other to specifically bind to a target antigen.

In some embodiments, the antigen-binding region is derived from an antibody that specifically binds to a target antigen. In some embodiments the antigen-binding region may also be derived from a specific receptor where the ligand of the receptor may serve as an antigen to be targeted. For example, the specific receptor may be a native T cell receptor. In some embodiments, the antigen-binding region comprises a variable region from a native T cell receptor. In some embodiments, the antigen-binding region may also be derived from a ligand particularly where the antigen to be targeted is a receptor.

In some embodiments, the antigen-binding region is derived from a native-specific T cell receptor. In some embodiments, the first antigen-binding region comprises a variable region shown in SEQ ID NO: 44 and the second antigen-binding region comprises a variable region shown in SEQ ID NO: 45. In some embodiments, the first antigen-binding region comprises a variable region shown in SEQ ID NO: 46 and the second antigen-binding region comprises a variable region shown in SEQ ID NO: 47. In some embodiments, the first antigen-binding region comprises a variable region shown in SEQ ID NO: 48 and the second antigen-binding region comprises a variable region shown in SEQ ID NO: 49.

In some embodiments, The target antigen is a disease-associated antigen, preferably a cancer-associated antigen, such as a cancer-associated antigen selected from the group consisting of: phosphatidylinositol proteoglycan 3 (GPC3), CD16, CD64, CD78, CD96, CLL1, CD116, CD117, CD71, CD45, CD71, CD123, CD138, ErbB2 ( HER2/neu), carcinoembryonic antigen (CEA), epithelial cell adhesion molecule (EpCAM), epidermal growth factor receptor (EGFR), EGFR Variant III ( EGFRvIII), CD19, CD20, CD30, CD40, disialoganglioside GD2, ductal epithelial mucin, gp36, TAG-72, glycosphingolipids, glioma-associated antigen, β-human chorionic gonadotropin, α fetal globulin (AFP), exogenous lectin reactive AFP, thyroglobulin, RAGE-1, MN- CA IX, human telomerase reverse transcriptase, RU1, RU2 ( AS), intestinal carboxylesterase, mut hsp70- 2. M-CSF, prostase, prostate-specific antigen (PSA), PAP, NY-ESO-1, LAGA-1a, p53, Prostein, PSMA, survival and telomerase, prostate cancer tumor antigen-1 (PCTA-1), MAGE, ELF2M, neutrophil elastase, ephrin B2, CD22, insulin growth factor (IGF1)-I, IGF-II, IGFI receptor, mesothelin, major histocompatibility complex (MHC) molecule presenting tumor-specific peptide epitopes, 5T4, ROR1, Nkp30, NKG2D, tumor matrix antigen, extradomain A (EDA) and extradomain B (EDB) of fibronectin, A1 domain of tenascin-C (TnC A1), fibroblast associated protein (fap), CD3, CD4, CD8, CD24, CD25, CD33, CD34, CD133, CD138, Foxp3, B7-1 (CD80), B7-2 (CD86), GM-CSF, cytokine receptor, endothelial factor, major histocompatibility complex(MHC) molecules, BCMA (CD269, TNFRSF17), TNFRSF17 (UNIPROT Q02223), SLAMF7 (UNIPROT Q9NQ25), GPRC5D (UNIPROT Q9NZD1), FKBP11 (UNIPROT Q9NYL4), KAMP3, ITGA8 (UNIPROT P53708), and FCRL5 (UNIPROT Q68SN8).

In some embodiments, the target antigen is an antigen derived from a pathogen or a surface antigen of a cell infected by a pathogen, such as RSVF (prevention of respiratory syncytial virus), PA (inhaled anthrax), CD4 (HIV infection), etc.

In some embodiments, The target antigen is disease-causing cells or molecules produced and secreted by cells, such as CD3 (involving transplant rejection), CD25 (involving acute rejection of kidney transplantation), C5 (involving paroxysmal nocturnal hemoglobinuria), IL-1 β (cryopyrin-associated periodic syndromes), RANKL (involving cancer-associated bone injury), von Willebrand factor (involving adult acquired thrombotic platelet purpura), plasma kallikrein (involving angioedema), calcitonin gene-related peptide receptor (involving migraine in adults), FGF23 (involving X-linked hypophosphatemia), etc.

The antigen-binding region may be derived from one or more known antibodies, including any commercially available antibodies, such as FMC63, rituximab, alemtuzumab, epratuzumab, trastuzumab, bivatuzumab, cetuximab, labetuzumab, palivizumab, sevirumab, tuvirumab, basiliximab, daclizumab, infliximab, om alizumab, efalizumab, keliximab, siplizumab, natalizumab, clenoliximab, pemtumomab, edrecolomab, cantuzumab, etc.

In some embodiments, the first antigen-binding region comprises a heavy chain variable region of an antibody that specifically binds to a target antigen, and the second antigen binding region comprises a light chain variable region of the antibody; alternatively, the first antigen-binding region comprises a light chain variable region of an antibody that specifically binds to a target antigen, and the second antigen-binding region comprises a heavy chain variable region of the antibody.

In some embodiments, the first antigen-binding region comprises a single chain antibody or a single domain antibody that specifically binds to a target antigen; and/or the second antigen-binding region comprises a single chain antibody or a single domain antibody that specifically binds to a target antigen.

In some embodiments, the single chain antibody comprises a heavy chain variable region and a light chain variable region linked by a linker, such as (G₄S)n, where n represents an integer from 1 to 10, preferably n is 1 or 3.

In some embodiments, the first antigen-binding region and the second antigen-binding region bind to the same target antigen.

In some embodiments, the first antigen-binding region and the second antigen-binding region bind to different regions (e.g. different epitopes) of the same target antigen.

In some embodiments, the first antigen-binding region and the second antigen-binding region bind to different target antigens.

For example, in some exemplary embodiments, the two antigen-binding regions may bind to CD19 and CD20, respectively, or to CD19 and CD22, respectively, or to CD38 and BCMA, respectively, or to PDL1 and EGFR, respectively. In some embodiments, the first antigen-binding region and/or the second antigen-binding region specifically bind to CD19.

In some embodiments, the first antigen-binding region comprises a heavy chain variable region amino acid sequence as shown in SEQ ID NO: 50, and the second antigen-binding region comprises a light chain variable region amino acid sequence as shown in SEQ ID NO: 51; alternatively, the first antigen-binding region comprises a light chain variable region amino acid sequence as shown in SEQ ID NO: 51, and the second antigen-binding region comprises a heavy chain variable region amino acid sequence as shown in SEQ ID NO: 50, whereby the TCR or TCR complex specifically binds to CD19.

In some embodiments, the first antigen-binding region comprises a light chain variable region amino acid sequence as shown in SEQ ID NO: 52, and the second antigen-binding region comprises a heavy chain variable region amino acid sequence as shown in SEQ ID NO: 53; alternatively, the first antigen-binding region comprises a heavy chain variable region amino acid sequence as shown in SEQ ID NO: 53, and the second antigen-binding region comprises a light chain variable region amino acid sequence as shown in SEQ ID NO: 52, whereby the TCR or TCR complex specifically binds to GPC3.

In some embodiments, the first antigen-binding region comprises a heavy chain variable region amino acid sequence as shown in SEQ ID NO: 54, and the second antigen-binding region comprises a light chain variable region amino acid sequence as shown in SEQ ID NO: 55; alternatively, the first antigen-binding region comprises a light chain variable region amino acid sequence as shown in SEQ ID NO: 55, and the second antigen-binding region comprises a heavy chain variable region amino acid sequence as shown in SEQ ID NO: 54, whereby the TCR or TCR complex specifically binds to CD19.

In some embodiments, the first antigen-binding region comprises a heavy chain variable region amino acid sequence as shown in SEQ ID NO: 62, and the second antigen-binding region comprises a light chain variable region amino acid sequence as shown in SEQ ID NO: 63; alternatively, the first antigen-binding region comprises a light chain variable region amino acid sequence as shown in SEQ ID NO: 63, and the second antigen-binding region comprises a heavy chain variable region amino acid sequence as shown in SEQ ID NO: 62, whereby the TCR or TCR complex specifically binds to CD20.

In some embodiments, the first antigen-binding region and/or the second antigen-binding region comprise a single chain antibody comprising a heavy chain variable region amino acid sequence as shown in SEQ ID NO: 50 and a light chain variable region amino acid sequence as shown in SEQ ID NO: 51, whereby the first antigen-binding region and/or the second antigen-binding region specifically bind to CD19. In certain embodiments, the heavy chain variable region amino acid sequence shown in SEQ ID NO: 50 and the light chain variable region amino acid sequence shown in SEQ ID NO: 51 are connected via a linker. In some embodiments, the linker is (G4S)n, where n represents an integer from 1 to 10, preferably n is 1 or 3.

In some embodiments, the first antigen-binding region and/or the second antigen-binding region comprise a single chain antibody comprising a heavy chain variable region amino acid sequence as shown in SEQ ID NO: 62 and a light chain variable region amino acid sequence as shown in SEQ ID NO: 63, whereby the first antigen-binding region and/or the second antigen-binding region specifically bind to CD20. In certain embodiments, the heavy chain variable region amino acid sequence shown in SEQ ID NO: 62 and the light chain variable region amino acid sequence shown in SEQ ID NO: 63 are connected via a linker. In some embodiments, the linker is (G4S)n, where n represents an integer from 1 to 10, preferably n is 1 or 3.

In some embodiments, the first antigen-binding region and/or the second antigen-binding region comprise a single chain antibody comprising a heavy chain variable region amino acid sequence as shown in SEQ ID NO: 52 and a light chain variable region amino acid sequence as shown in SEQ ID NO: 53, whereby the first antigen-binding region and/or the second antigen-binding region specifically bind to GPC3. In certain embodiments, the heavy chain variable region amino acid sequence shown in SEQ ID NO: 52 and the light chain variable region amino acid sequence shown in SEQ ID NO: 53 are connected via a linker. In some embodiments, the linker is (G4S)n, where n represents an integer from 1 to 10, preferably n is 1 or 3.

In some embodiments, the first antigen-binding region and/or the second antigen-binding region comprise a single chain antibody comprising a heavy chain variable region amino acid sequence as shown in SEQ ID NO: 54 and a light chain variable region amino acid sequence as shown in SEQ ID NO: 55, whereby the first antigen-binding region and/or the second antigen-binding region specifically bind to CD19. In some embodiments, the heavy chain variable region amino acid sequence shown in SEQ ID NO: 54 and the light chain variable region amino acid sequence shown in SEQ ID NO: 55 are connected via a linker. In some embodiments, the linker is (G4S)n, where n represents an integer from 1 to 10, preferably n is 1 or 3.

In some embodiments, the first antigen-binding region comprises an scFv amino acid sequence as shown in SEQ ID NO: 38, and the second antigen-binding region comprises an scFv amino acid sequence as shown in SEQ ID NO: 39; alternatively, the first antigen-binding region comprises an scFv amino acid sequence as shown in SEQ ID NO: 39, and the second antigen-binding region comprises an scFv amino acid sequence as shown in SEQ ID NO: 38, whereby the TCR or TCR complex specifically binds to both CD19 and CD20.

In some embodiments, the CD3y, CD3δ, CD3ε and/ or CD3ζ is humanized. In some embodiments, the human CD3y comprises the amino acid sequence shown in SEQ ID No: 28. In some embodiments, the human CD3y comprises the amino acid sequence shown in SEQ ID No: 29. In some embodiments, the human CD3y comprises the amino acid sequence shown in SEQ ID No: 30. In some embodiments, the human CD3y comprises the amino acid sequence shown in SEQ ID No: 31.

In another aspect, an isolated therapeutic immune cell is provided herein, which comprises the modified T cell receptor (TCR) or TCR complex of the present invention.

In some embodiments, the immune cell is a T cell. In other embodiments, the immune cell is a NK cell.

In another aspect, the present invention provides an isolated polynucleotide comprising a nucleotide sequence encoding at least one of TCR α chain, TCR β chain, TCR γ chain, TCR δ chain, CD3 ε, CD3 γ, CD3 δ and CD3 ζ as defined above, wherein at least one exogenous functional endodomain is connected to the C-terminal of at least one of the TCR α chain, TCR β chain, TCR γ chain, TCR δ chain, CD3 ε, CD3 γ, CD3 δ and CD3 ζ.

In another aspect, the present invention provides an isolated polynucleotide comprising a nucleotide sequence encoding TCR as defined above.

In some embodiments, the isolated polynucleotide comprises a nucleotide sequence encoding TCR α chain and/or TCR β chain which is connected to at least one costimulatory molecule endodomain at the C-terminal thereof.

In some embodiments, the polynucleotide comprises i) a nucleotide sequence encoding the α chain, ii) a nucleotide sequence encoding the β chain, and iii) a nucleotide sequence encoding a self-cleavage peptide located between i) and ii) in the same reading frame. The nucleotide sequence encoding the α chain may be located at the 5' end or the 3' end of the nucleotide sequence encoding the β chain.

In some embodiments, the isolated polynucleotide comprises a nucleotide sequence encoding TCR γ chain and/or TCR δ chain which is connected to at least one costimulatory molecule endodomain at the C-terminal thereof.

In some embodiments, the polynucleotide comprises i) a nucleotide sequence encoding the γ chain, ii) a nucleotide sequence encoding the δ chain, and iii) a nucleotide sequence encoding a self-cleavage peptide located between i) and ii) in the same reading frame. The nucleotide sequence encoding the γ chain may be located at the 5' end or the 3' end of the nucleotide sequence encoding the δ chain.

As used herein, the "self-cleavage peptide" means a peptide that can carry out self-cleavage in cells. For example, the self-cleavage peptide may contain a protease recognition site, so as to be recognized and specifically cleaved by proteases in cells.

Alternatively, the self-cleavage peptide may be a 2A polypeptide. The 2A polypeptide is a kind of short peptide from virus, and its self-cleavage occurs during translation. When two different target proteins are linked by 2A polypeptide and expressed in the same reading frame, the two target proteins are generated almost in a ratio of 1:1. A common 2A polypeptide may be P2A from porcine techovirus-1, T2A from Thosea asigna virus, E2A from equine rhinitis A virus, and F2A from foot-and-mouth disease virus. Among them, P2A has the highest cutting efficiency and is therefore preferred. A variety of functional variants of these 2A polypeptides are also known in the art, which can also be used in the present invention.

In another aspect, the present invention provides an expression vector comprising the polynucleotide of the present invention operably linked to a regulatory sequence.

The "expression vector" of the present invention may be a linear nucleic acid fragment, a cyclic plasmid, a viral vector, or an RNA capable of translation (e.g. mRNA). In some preferred embodiments, the expression vector is a viral vector, such as a lentiviral vector.

The term "regulatory sequence" and "regulatory element" are used interchangeably to refer to a nucleotide sequence that is located upstream (5' non-coding sequence), intermediate or downstream (3' non-coding sequence) of a coding sequence and affect the transcription, RNA processing or stability or translation of the relevant coding sequence. An expression regulatory element refers to a nucleotide sequence that can control the transcription, RNA processing or stability, or translation of a nucleotide sequence of interest. A regulatory sequence may include, but is not limited to, a promoter, a translation leader sequence, an intron, an enhancer, and a polyadenylation recognition sequence.

As used herein, the term "operably linked" means that a regulatory element (e.g., but not limited to, a promoter sequence, a transcription termination sequence, etc.) is linked to a nucleic acid sequence (e.g., a coding sequence or an open reading frame) such that the nucleotide sequence transcription is controlled and regulated by the transcriptional regulatory element. Techniques for operably linking a regulatory element region to a nucleic acid molecule are known in the art.

In another aspect, the present invention provides a method for preparing the therapeutic immune cell of the present invention, comprising introducing the polynucleotide or expression vector of the present invention into the immune cell.

The immune cell of the present invention, such as a T cell or NK cell, may be obtained by various non-limiting methods from a number of non-limiting sources, including peripheral blood mononuclear cells, bone marrow, lymph node tissue, cord blood, thymus tissue, ascite, pleural effusion, spleen tissue, and tumor. In some embodiments, the cell may be derived from a healthy donor or from a patient diagnosed as cancer. In some embodiments, the cell may be part of a mixed population of cells showing distinct phenotypic profiles. For example, T cells can be obtained by isolating peripheral blood mononuclear cells (PBMC), then activating and amplifying with a specific antibody.

In some embodiments of aspects of the invention, the immune cells, such as T cells, are derived from autologous cells of a subject. As used herein, "autologous" means that cells, cell line, or population of cells used to treat a subject is derived from the subject. In some embodiments, the immune cells, such as T cells, are derived from allogeneic cells, such as a donor compatible with the subject human leukocyte antigen (HLA). Cells from donors can be converted into non-alloreactive cells using standard protocols and replicated as needed to produce cells that can be administered to one or more patients.

In another aspect, the present invention provides a pharmaceutical composition, which comprises the therapeutic immune cell of the present invention and a pharmaceutically acceptable carrier.

As used herein, a "pharmaceutically acceptable carrier" includes any and all physiologically compatible solvents, dispersion medium, coatings, antibacterial and antifungal agents, isotonic agents and absorption retarders, etc. Preferably, the carrier is suitable for intravenous, intramuscular, subcutaneous, parenteral, spinal, or epidermal administration (e.g. by injection or infusion).

In another aspect, the present invention provides the use of the therapeutic immune cell of the present invention in the preparation of a medicine for the treatment of diseases in a subject.

As use herein, "subject" refers to an organism that suffers from or is prone to suffer from a disease (e.g., cancer) that can be treated by the cell, method, or pharmaceutical composition of the present invention. A non-limiting example includes a human, a cattle, a rat, a mouse, a dog, a monkey, a goat, a sheep, a cow, a deer, and other non-mammals. In some preferred embodiments, the subject is a human.

In another aspect, the present invention provides a method for treating a disease such as cancer in a subject, the method comprising administering to the subject an effective amount of therapeutic immune cell or pharmaceutical composition of the present invention.

As used herein, a "therapeutically effective amount" or "therapeutically effective dose" or "effective amount" refers to the amount of a substance, compound, material or cell that is at least sufficient to produce a therapeutic effect after administration to a subject. Therefore, it is an amount necessary to prevent, cure, improve, block or partially block the symptoms of disease or disorder. For example, an "effective amount" of the cell or pharmaceutical composition of the present invention may preferably result in a decrease in the severity of disorder symptoms, an increase in the frequency and duration of the asymptomatic period of the disorder, or the prevention of injury or disability as a result of suffering from the disorder. For example, for the treatment of tumor, an "effective amount" of the cell or pharmaceutical composition of the present invention may preferably inhibit tumor cell growth or tumor growth by at least about 10%, preferably at least about 20%, more preferably at least about 30%, more preferably at least about 40%, more preferably at least about 50%, more preferably at least about 60%, more preferably at least about 70%, and more preferably at least about 80%, as compared to an untreated subject. The ability to inhibit tumor growth can be evaluated in an animal model system that may predict efficacy in a human tumor. Alternatively, it is possible to perform evaluation by examining the ability to inhibit the growth of tumor cells which may be determined in vitro by tests known to those skilled in the art.

In practice, the dose level of cells in the pharmaceutical composition of the present invention may vary to obtain an amount of the active ingredient that can effectively achieve the desired therapeutic response to a specific patient, composition and administration route without toxicity to the patient. The chosen dose level depends on a variety of pharmacokinetic factors, including the activity of the applied particular composition of the invention, administration route, administration time, excretion rate of the applied particular compound, duration of treatment, applied other drugs, compounds and/or materials in combination with the applied particular composition, age, gender, weight, condition, general health and medical history of the patient to be treated, and similar factors known in the medical field.

The administration of the therapeutic immune cell or pharmaceutical composition or drug according to the present invention may be carried out in any convenient manner, such as through injection, infusion, implantation or transplantation. The administration of the cell or composition described herein may be intravenous, intralymphatic, intradermal, intratumoral, intramedullary, intramuscular, or intraperitoneal administration. In one embodiment, the cell or composition of the present invention is preferably administered by intravenous injection.

In embodiments of various aspects of the invention, the disease is, for example, cancer, examples of such cancers include, but are not limited to, lung cancer, ovarian cancer, colon cancer, rectal cancer, melanoma, kidney cancer, bladder cancer, breast cancer, liver cancer, lymphoma, malignant hematological diseases, head and neck cancer, glioma, gastric cancer, nasopharyngeal carcinoma, laryngeal cancer, cervical cancer, uterine body tumor, osteosarcoma, bone cancer, pancreatic cancer, skin cancer, prostate cancer, uterine cancer, anal cancer, testicular cancer, fallopian tube cancer, endometrial carcinoma, vaginal cancer, vulva cancer, Hodgkin's disease, non-Hodgkin's lymphoma, esophageal cancer, small intestinal cancer, endocrine system cancer, thyroid cancer, parathyroid carcinoma, adrenal cancer, soft tissue sarcoma, urethral cancer, penis cancer, chronic or acute leukemia ( including acute myeloid leukemia, chronic myeloid leukemia, acute lymphoblastic leukemia, chronic lymphocytic leukemia), solid tumors in children, lymphocytic lymphoma, bladder cancer, renal or ureteral cancer, renal pelvis cancer, central nervous system (CNS) tumor, primary CNS lymphoma, tumor angiogenesis, spinal tumor, brain stem glioma, pituitary adenoma, Kaposi's sarcoma, epidermal carcinoma, squamous cell carcinoma, T-cell lymphoma, environment-induced cancer (including asbestos-induced cancer), and combinations of thereof.

In embodiments of various aspects of the invention, the disease is of, for example, pathogen infection, examples of the pathogen include, but are not limited to, respiratory syncytial virus, Bacillus anthracis, human immunodeficiency virus, and the like.

In embodiments of various aspects of the invention the disease is of, for example, cardiovascular disease, diabetes, neurological diseases, anti-rejection after transplantation, and so on.

### Examples

### Example 1 Improvement of STAR

### 1. Wild-type T cell receptor and design of its constant region-mutated STAR molecule

### 1.1 Prototype design of STAR

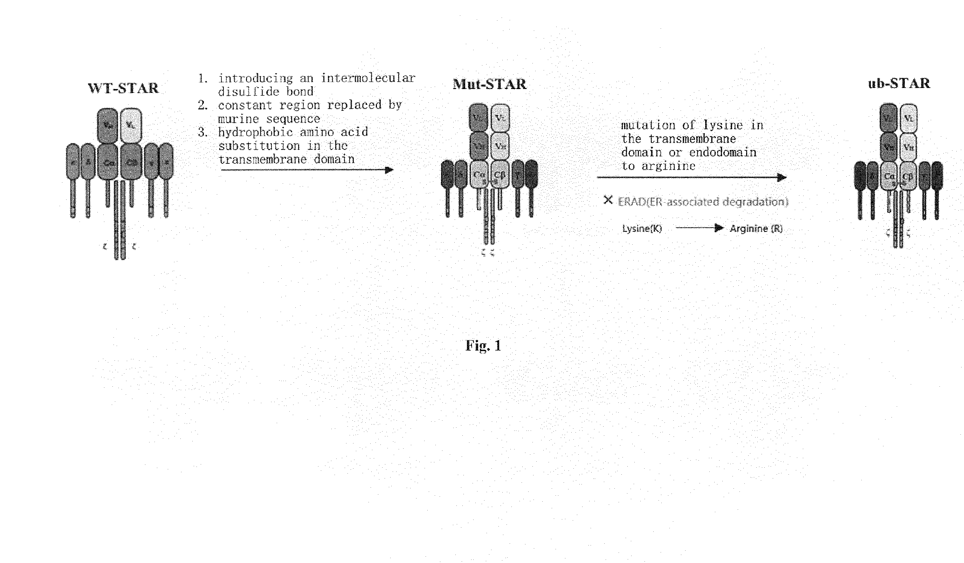

The secreted antibody (Antibody, Ab) or B-cell receptor (BCR) produced by B cells has great similarity to the T-cell receptor (TCR) in terms of genetic structure, protein structure and spatial conformation. Both the antibody and TCR consist of a variable region and a constant region, in which the variable region plays the role of antigen-recognizing and binding, while the constant region domain plays the role of structural interaction and signal transduction. By replacing the variable regions of TCR α and β chains (or TCR γ and δ chains) with the heavy chain variable region (VH) and light chain variable region (VL) of the antibody, an artificially synthetic chimeric molecule called Synthetic T-Cell Receptor and Antibody Receptor (STAR/WT-STAR) can be constructed with the structure thereof shown in Figure 1 (left).

A STAR molecule has two chains, wherein the first chain is obtained by fusing an antigen recognition sequence (such as an antibody heavy chain variable region) with a constant region (C α) of a T cell receptor α chain (TCR α), and the second chain is obtained by fusing an antigen recognition sequence (such as an antibody light chain variable region) with a constant region (C β) of a T cell receptor β chain (TCR β). The antigen recognition domain (such as VH, VL or scFv) and the constant domain (constant domain of TCR α, β, γ and δ) in the construct can be arranged and combined to form a variety of constructs with different configurations but similar functions.

The first and second chains of STAR molecule, after expressing in T cells, will combined with endogenous CD3 ε δ, CD3 γ ε and CD3 ζ chains in the endoplasmic reticulum to form a eight-subunit complex, which is present on the surface of cell membrane in the form of complex. An immunoreceptor tyrosine-based activation motif (ITAM) is a signal transduction motif in a TCR molecule, with its conserved sequence of YxxL/V. The endodomain of CD3 ε, δ, γ and ε chains comprises one ITAM sequence, and that of CD3 ζ chain comprises three ITAM sequences, so a complete STAR complex has a total of ten ITAM sequences. When the antigen recognition sequence of a STAR receptor binds to its specific antigen, the intracellular ITAM sequence will be phosphorylated successively, which then in turn activate the downstream signaling pathway, activating transcription factors such as NF-κ β, NFAT, and AP-1, to initiate the activation of T cells and produce effector functions. Previous studies by the inventor have shown that STAR can activate T cells better than a conventional chimeric antigen receptor CAR, and the background activation in the absence of antigen stimulation is significantly reduced, thus having significant advantages (see Chinese invention patent application No. 201810898720.2). However, a further improvement to STAR is still desired.

### 1.2. Design of mutant STAR (mut-STAR) and STAR with transmembrane domain and endodomain modified (ub-STAR)

The STAR prototype design used a humanzied TCR α/β chain (or TCR γ and δ chains) constant region sequence (wild-type human TCR α constant region, SEQ ID NO: 1; wild-type human TCR β constant region, SEQ ID NO: 2). Due to the constant region sequences of human, primate and murine TCR α/β chains (mouse TCRaC-WT, SEQ ID NO: 3; mouse TCRbC-WT, SEQ ID NO: 4) are highly conserved, and have the same key amino acid sequence as well, they can be replaced with each other.

After being transferred into T cells, STAR molecules will mismatch with the endogenous TCR of T cells through the constant region. On one hand, this mismatch problem may reduce the efficiency of correct pairing of STAR molecules and weakens their functions, which, on the other hand, may increase the possibility of unknown specificity due to mismatch and increases the security risk. To solve this problem, the constant region of a STAR molecule was replaced with a murine sequence by the inventors to enhance the functions of the STAR molecule after being transferred into human T cells. In order to further optimize the design of STAR molecule, cysteine mutation was carried out on the STAR molecule by the inventors to introduce an intermolecular disulfide bond, thereby enhancing mutual pairing between two chains of the STAR molecule, and reducing mismatch with an endogenous TCR. Specifically, threonine (T) at position 48 was mutated to cysteine (C) (mouse TCRaC-Cys, SEQ ID NO: 5) in TCR α chain constant region, and serine (S) at position 56 was mutated to cysteine (C) (mouse TCRbC-Cys, SEQ ID NO: 6) in the TCR β chain constant region. The two new added cysteines will form a disulfide bond between the two chains of STAR, thereby reducing mismatch between the two chains of STAR with the endogenous TCR chain, and helping STAR molecules form more stable complexes, thus obtaining better functions. In addition, in order to further optimize the design of a STAR molecule, hydrophobic amino acid substitution was performed by the inventors on the transmembrane domain of the STAR molecule to increase the stability of the STAR molecule and help it play a more lasting function. Specifically, three amino acid mutations were carried out at amino acid positions 111 to 119 in the transmembrane domain of TCR α chain constant region: serine (S) at position 112 was mutated to leucine (L), methionine (M) at position114 was mutated to isoleucine (I), and glycine (G) at position 115 was mutated to serine (V). The whole amino acid sequence in this region was changed from LSVMGLRIL to LLVIVLRIL, and this modification was called mouse TCRaC-TM9, which produced a constant region sequence of SEQ ID NO: 7. This design increased the hydrophobicity of transmembrane domain, counteracts the instability caused by positive charges carried by the TCR transmembrane domain, and makes STAR molecule more stable on the cell membrane, thus obtaining better functions, with the structure thereof shown in Figure 1 (middle).

After TCR bound to antigen and activation was completed, lysine in the endodomain and transmembrane domain of the TCR molecule was subject to ubiquitin modifications through a series of ubiquitination reactions by a ubiquitin activating enzyme, ubiquitin binding enzyme and ubiquitin ligase ubiquitinate, thereby producing T cell endocytosis, leading to the endocytosis of TCR molecules into the cells for further degradation by lysosomes, thus reducing the concentration of TCR molecules on the surface of the T cell membrane, resulting in the continuous decline of the effect of T cell activation. The amino acids in the transmembrane domain or endodomain of the α and β chains in the mut-STAR molecule were modified by the inventors, which comprising: mutating lysine in the endodomain of the α chain constant region and the transmembrane domain of the β chain constant region of the STAR molecule to arginine, producing the constant region sequence of Mouse TCR α C-Arg mut (SEQ ID NO: 8) and the constant region sequence of Mouse TCR β C-Arg mut (SEQ ID NO: 9), respectively, to reduce the endocytosis of STAR molecule caused by lysine ubiquitination. This design reduced the possibility of ubiquitination of the transmembrane domain and endodomain of a STAR molecule, thus reducing the endocytosis of STAR molecules, enabling the STAR molecules more stable on the cell membrane and obtain better functions, with the structure thereof shown in Figure 1 (right).

### 2. Design of wild-type and mutant STAR molecules comprising a costimulatory receptor endodomain

In order to improve the proliferation ability in vivo of mut-STAR cells, the effect survival time and the ability to infiltrate into the tumor microenvironment to kill the target cells efficiently, a new structure was designed by the inventors, wherein, the mut-STAR complex was modified, and an enhanced mut-STAR cell can be tailored as needed, so as to improve the clinical response of TCR-T and realize a lasting curative effect.

### 2.1. Design of mut-STAR molecules (co-STAR) comprising a costimulatory molecule receptor endodomain

TCR is a special marker on the surface of all T cells, which can be divided into αβ TCR and γ δ TCR, and the corresponding T cells thereof are αβ T cells and γ δ T cells respectively. αβ-STAR and γδ-STAR were modified with costimulatory signals, respectively, by the inventors, to improve the performance of αβ T cells and γ δ T cells, respectively.

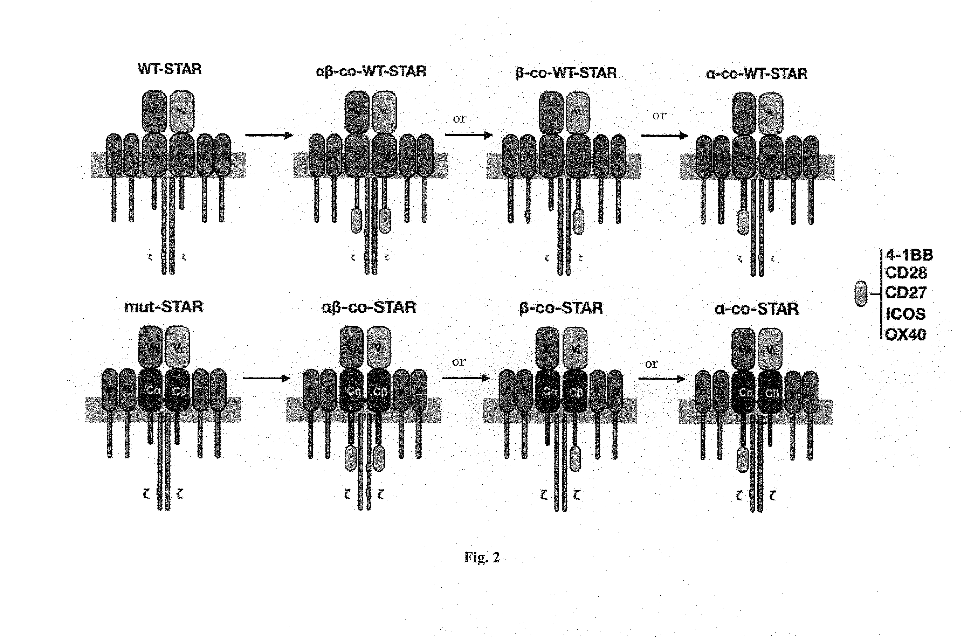

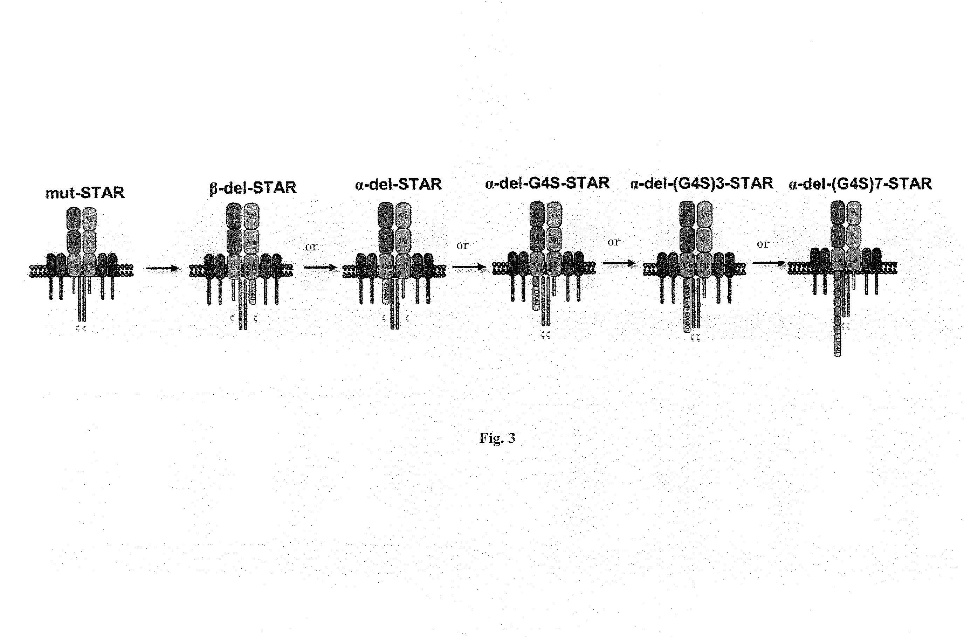

TCR of αβ T cells consists of TCR α and TCR β chains, accounting for 90%-95% of the total T cells. αβ TCR consists of a variable region and a constant region, in which the variable region has a wide diversity and plays the role of antigen recognition and binding, while the constant region domain plays the role of structural interaction and signal transduction. In order to enhance the toxicity and proliferation persistence of T cells, according to the present invention, an endodomain sequence of a humanized costimulatory receptor is introduced to the C-terminal of the αβ-STAR constant region (Fig. 2), to study the influence on STAR T cell functions. The STAR constant region of the present invention includes an unmodified WT-STAR constant region, a cys-STAR constant region containing an additional intermolecular disulfide bond, a murinized hm-STAR constant region, and a mut-STAR with a combination of the three modifications described in subsection 1. The costimulatory signal transduction structure comprises intracellular signal transduction domains of CD40, OX40, ICOS, CD28, 4-1BB or CD27, with the sequences of SEQ ID NO: 10, SEQ ID NO: 11, SEQ ID NO: 12, SEQ ID NO: 13, SEQ ID NO: 14 and SEQ ID NO: 15, respectively. The costimulatory endodomain can be connected to the C-terminal of TCR α chain or TCR β chain or both TCR α chain and β chain (co-STAR). In addition, the costimulatory endodomain can be connected directly or via a linker G4S/(G4S)n (G4S linker sequences are SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, respectively) to the C-terminal of TCR constant region, or the C-terminal of TCR constant region with the endodomain sequence of the TCR molecule deleted (endodomain-deleted TCR α chain constant region comprising both cysteine substitution and hydrophobic region modification (mouse TCRaC-del mut, SEQ ID NO: 26), endodomain-deleted TCR β chain constant region comprising cysteine substitution (mouse TCRβC-del mut, SEQ ID NO:27)) (co-linker-STAR, Fig. 3).

TCR of γ δ T cells consists of TCR γ and TCR δ chains, γ δ T cells can be divided into three subgroups: γ δ 1, γ δ 2 and γ δ 3 based on the type of TCR δ chain, with different subgroups having different distribution in human bodies. γ δ T cells recognize an antigen in an MHC-restricted way, which plays an important role in the surveillance of pathogens and tumors. Experiments showed that, CD28 or 4-1BB, and similar costimulatory signals played an important role in the activation and proliferation of γ δ T cells. The endodomain sequence of human costimulatory molecule receptor was introduced to the C terminal of TCR γ and TCR δ, respectively (Fig. 2, right), by the inventors, to improve the performance of γ δ T cells.

### 2.2. Design of CD3 molecule (co-CD3-STAR) comprising a costimulatory molecule receptor endodomain

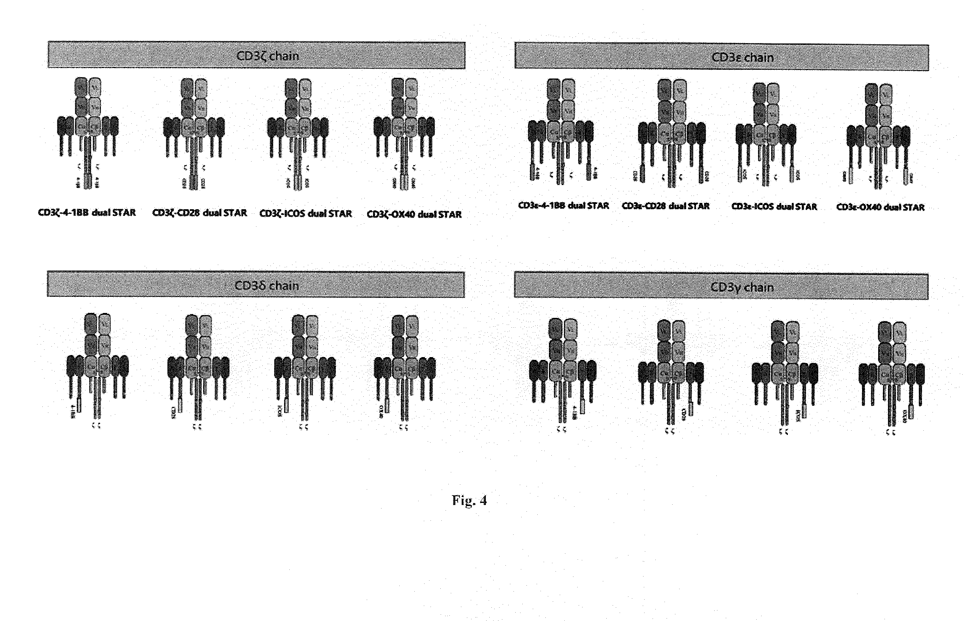

A CD3 subunit includes γ chain, δ chain, ε chain and ζ chain, and forms a T cell receptor complex with a TCR molecule, which transmits signals from the ectodomain to endodomain, so as to regulate the state of cells and response to stimuli. In order to design enhanced TCR T cells and improve the tumor killing ability, proliferation ability and survival time of T cells in vivo, a CD3 molecule was modified by the inventors by introducing a human costimulatory molecule receptor endodomain to the C terminal of CD3 γ chain (SEQ ID NO: 28), δ chain (SEQ ID NO: 29), ε chain (SEQ ID NO: 30) and ζ chain (SEQ ID NO: 31) (Fig. 4). The modified CD3 molecule was expressed in mut-STAR T cells to improve its function.

### 2.3. Design of CD3 molecule comprising the stimulatory region of cytokine receptor (cytokine-STAR, CK-STAR)

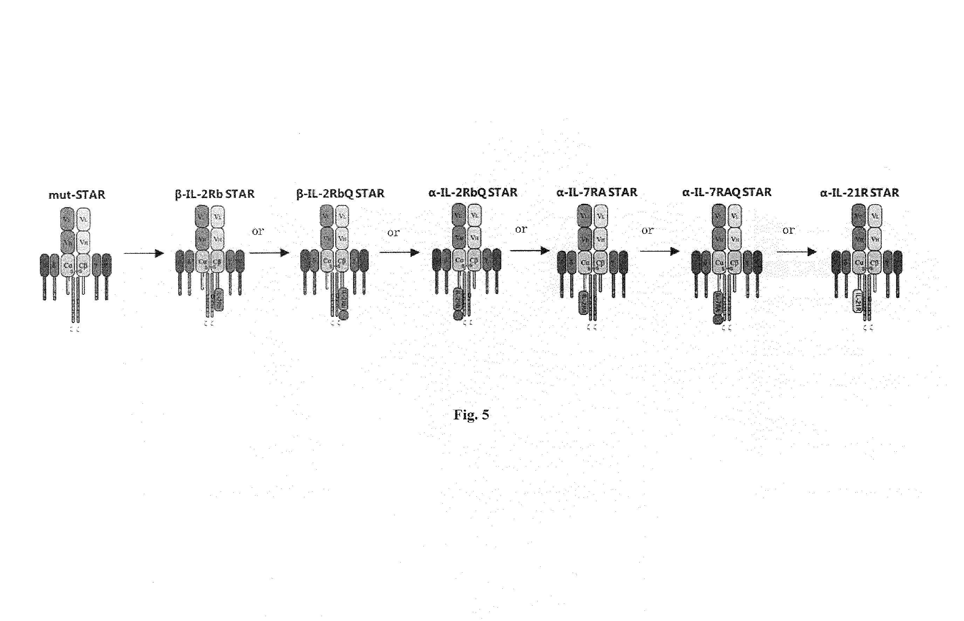

Cytokines play an important role in proliferation, anti-tumor and differentiation of T cells. Different cytokines combine with their respective receptors to transmit signals from the ectodomain to endodomain, so as to regulate the state of cells and response to stimuli. In addition, studies showed that the downstream molecule STATS (SEQ ID NO: 35) was activated by cascade reaction at the IL-2 receptor endodomain, thus enhancing the transcription of T cell proliferation-related molecules and enhancing the proliferation ability of CAR-T cells. In order to design enhanced STAR-T cells and improve the tumor-killing ability, proliferation ability and survival time of T cells in vivo, the STAR molecule was modified by the inventors by linking the intracellular signal transduction domain of a human cytokine receptor (e.g., IL-2 β receptor endodomain IL2Rb, SEQ ID NO: 32; IL-7 α receptor endodomain, SEQ ID NO: 33; IL-21 receptor endodomain, SEQ ID NO: 34, etc.)) to the C-terminal of TCR α chain or β chain or both α and β chains, or by further linking the STATS activation moiety to the IL-2 β or IL-7R α receptor endodomain via G4S (IL-2RbQ, SEQ ID NO: 36; IL-7RbQ, SEQ ID NO: 37) (Fig. 5).

### 3. Construction of vector of wild-type and mutant STAR molecules comprising the costimulatory molecule receptor endodomain

### 3.1 Vector sources

The vectors used in the present invention, including viral vectors, plasmid vectors, etc. were purchased from or synthesized by commercial companies, and the full-length sequences of these vectors are obtained, and the specific cleavage sites are known.

### 3.2. Fragment sources

The TCR mentioned in the invention can be any functional TCR, including WT-STAR, mut-STAR, ub-STAR, co-STAR, co-linker-STAR, CK-STAR, co-CD3-STAR and the like used in the invention. Gene fragments, such as the variable region of TCR, the constant region of TCR, the endodomain of a costimulatory molecule receptor, the intracellular signal transduction region of a cytokine receptor, a tag sequence, a linker and the like used in the present invention were all synthesized by commercial companies. These gene fragments were linked by PCR.

In this example, the optimization of TCR complex was validated using STAR comprising ScFv shown in SEQ ID NO: 38 (derived from a CD20-targeting antibody, OFA) and/or ScFv shown in SEQ ID NO: 39 (derived from a CD19-targeting antibody, FMC63), and was compared with a blank control group Mock expressing only RFP protein shown in SEQ ID NO: 40 (red fluorescent protein, RFP).

### 3.3. Vector construction

The lentiviral vector used herein was pHAGE-EF1 α-IRES-RFP, wherein the linear vector was obtained by restriction enzyme Not I/Nhe I, the gene fragment was obtained by synthesis and PCR, and the complete vector was obtained by homologous recombination.

### 4 Construction of cell lines:

### 4.1 Construction of plasmid pHAGE-luciferase-GFP

As a lentiviral vector, pHAGE can stably insert target genes into the genome of a target cell, which is an important way to construct a stable cell line. Luciferase was a kind of enzyme with catalytic activity, which can catalyze the chemical autoluminescence of a substrate to make the target cell express luciferase stably, the number of target cells can be indicated after adding the substrate, thus reflecting the effect of functional cells on target cells. A pHAGE-EF1A vector carrying restriction endonuclease NotI/ClaI cleavage sites was cleavaged by the two enzymes, and luciferase and GFP sequences were obtained by NCBI, the fragments were synthesized by Ruiboxingke, a commercial company, by combing the luciferase gene with GFP gene using Overlap PCR, and then the luciferase-GFP fragment was connected to the pHAGE vector by homologous recombination.

### 4.2 Construction of target cell line carrying Luciferase

After a lentiviral vector carrying with luciferase and GFP was successfully constructed, the lentivirus was packed by Lenti-X-293T, and the lentivirus solution was concentrated by PEG8000, the virus titer was measured by the gradient dilution method, and then the lymphoma cell line Raji was infected, after 72 hours of infection, whether there were GFP positive cells was observed by a fluorescence microscope, and then GFP positive cells were sorted by a flow sorter, and monoclonal cells were selected for library building and preservation. At the same time, the luciferase substrate was used to incubate with target cells, and the expression and detection levels of luciferase were detected to determine the expression level.

### 4.3 Construction of TCR α-β knockout Jurkat cell line

Based on the structure and sequence characteristics of TCR, a guide sequence was designed in the constant regions of α and β chains to construct a TCR α-β-Jurkat cell line. The exon sequences of the constant regions of TCR α and β chains were obtained in NCBI, and the exon 1 sequences of the constant regions of TCR α and β chains were submitted to the tools.genome-engineering.org website for designing guide sequences, based on the result, an oligo sequence was synthesized, and then a sgRNA-LentiCRISPR lentiviral vector (purchased from Aidi gene) was constructed. The guide sequence of α chain was linked to LentiCRISPR-puro, and the guide sequence of β chain was linked to LentiCRISPR-BSD.

Packaging of sgRNA-LentiCRISPR lentivirus: HEK-293T was planked to a 10 cm dish in advance, and when the cells grew to 80%-90%, the transfection system was added to HEK-293T, and the cells were put back into the incubator at 37 °C for culture. This time was counted as 0 hours; 12 hours after transfection, fresh 10% FBS-DMEM as added. The virus was collected 48 hours and 72 hours after transfection. The culture medium containing virus was centrifuged and filtered, mixed with PEG8000, placed at 4 °C for more than 12 hours, then centrifuged at 3500 rpm for 30 min, and resuspended and precipitated with an appropriate volume of medium after discarding the supernatant. The resultant was frozen at -80°C, or used directly.

Infection and screening of Jurkat T cells and identification of monoclonal cell line: Jurkat T cells were inoculated in a 12- or 24-well plate, followed by adding sgRNA-LentiCRISPR virus of α chain and β chain with appropriate volumes at the same time, as well as polybrene (added at a ratio of 1: 1000 based on the total volume), and mixing well. Centrifugation infection was performed at 1000 rpm at 32°C for 90 min. The resultant was placed in an incubator at 37 °C with counting as 0 hours; the liquid was changed after 10 to 12 hours; after 48 hours, Puromycin were added to the appropriate final concentration, and after treating for additional 48 hours, as shown, the cells in the uninfected control group all died. The surviving cells were sucked out, centrifuged and cultured in a complete medium to obtain a TCR α-β-Jurkat cell bank. Single cells from the TCR α-β-Jurkat cell bank were sorted into a 96-well plate by the flow sorter Aria, after two weeks of culture, the grown monoclones were sucked out for amplification culture. Monoclonal cell lines were identified with TCR α chain and β chain antibodies, respectively, and the cell lines with both chain deficient were amplified to obtain an endogenous TCR knockout Jurkat-T cell line.

### 5. Construct of virus packaging system for transducing T cells

### 5.1 Lentivirus system and packaging method (different generations)

Lentix-293T cells were inoculated to a 10 cm culture dish at 5×10⁵/mL, and cultured in an incubator at 37 °C with 5% CO₂, transfection was carried out when the cell density reached about 80% (observed under a microscope). The three plasmids were mixed with 500 µL of serum-free DMEM according to a 1: 2: 3 ratio of PMD2.G: PSPAX: transfer plasmid. 54 µL of PEI-Max and 500 uL of serum-free DMEM were mixed uniformly and left at room temperature for 5 min (in a 3:1 volume-mass ratio of PEI-Max to plasmid). A PEI-max mixture was slowly added to the plasmid mixture, blown gently, mixed evenly, and then left at room temperature for 15 min. The final mixture was slowly added to the culture medium, and evenly mixed, then put back into the incubator for another culture for12 h to16 h, then changed to a 6% FBS DMEM medium for another culture, and the virus solution was collected at 48 h and 72 h.

### 5.2 Virus titer measurement

Jurkat-C5 cells were inoculated in a flat-bottomed 96-well plate at 1.5 × 10⁵ cells /mL, and 100 uL of 1640 medium containing 10% FBS and 0.2 µ L 1000 × polybrene was added to each well. Virus dilution was carried out with a 1640 complete medium with 10 times dilution, the virus dosage in the first well was 100 µL when determining as the virus stock solution, or was 1 µL when determining as the concentrated solution. The diluted cells were added to the viral wells at 100 µ L/well, mixed at 32 °C, centrifuged at 1500 rpm for 90 min, cultured in an incubator at 37 °C with 5%CO₂ for 72 hours. Cells from the flat-bottomed 96-well plate were sucked into a round-bottomed 96-well plate, centrifuged at 4 °C and 1800 rpm for 5min, and the supernatant was discarded. After adding 200 uL of 1 × PBS, centrifugation was carried out at 4 °C and 1800 rpm for 5 min, and the supernatant was discarded. 200 uL of 4% tissue fixing solution was added, and the resultant solution was kept away from light, and was measured with a flow cytometry. The infection efficiency was measured by flow cytometry, the well with an effection rate of 2-30% was selected when calculating the titer according to the formula: titer (TU/mL) = 1.5 × 10⁴_{×} Positive rate/virus volume (µL) × 1000. Viruses of the following plasmids were packaged by the above method: pHAGE-EF1A-IRES-RFP, WT-STAR, mut-STAR, co-WT-STAR (αβ-4-1BB-WT, αβ-CD27-WT, αβ-CD28-WT, αβ-ICOS-WT, αβ-OX40-WT, αβ-OX40-WT), co-STAR (αβ-4-1BB, αβ-CD27, αβ-CD28, αβ-ICOS, αβ-OPX40, αβ-OX40), co-CD3-STAR (CD3 δ-4-1BB, CD3 δ-CD28, CD3 δ-ICOS, CD3 δ-OX40, CD3 ε-4-1BB, CD3 ε-CD28, CD3 ε-ICOS, CD3 ε-OX40, CD3 γ-OX40, CD3 γ-ICOS, CD3 γ-OX40, CD3 ζ-41BB, CD3 ζ-CD28, CD3 ζ-ICOS, CD3 ζ-OX40), co-linker-STAR (TCR β-del-OX40, TCR α-del-G4S-OX40, TCR α-del-G4S-OX40, TCR [3-del-(G4S) 3-OX40, TCR β-del-(G4S) 7-OX40), C K-STAR ([3-IL-2Rb STAR, β-IL-2RbQ STAR, α-IL-2RbQ STAR, α-IL-7RA STAR, α-IL-7RAQ STAR, α-IL-21R STAR), and so on.

### 6. Establishment of T cell culture and infection method

### 6.1 Jurkat T cell line culture

The Jurkat T cell line was cultured in a RPMI1640 medium containing 10% FBS with a culture density of 3^{∗}10⁵/ml and up to 3^{∗}10⁶/ml, and subcultured every 1 to 2 days. After cell counting, the required number of cells were taken and supplemented with the culture medium to adjust to the above density, and place in a CO₂incubator for culture.

### 6.2 Jurkat T cell line infection

Cells were counted, 1^{∗}10⁶/ml cells were taken, centrifuged and changed with the liquid, resuspended with 1 mL of RPMI 1640 medium containing 10% FBS, and added to a 24-well plate with a proper amount of virus solution added as well, centrifuged at 1500 rpm for 90 min, and placed in a CO₂ incubator for culture. The liquid was completely changed with fresh RPMI 1640 medium containing 10% FBS after 12 hours of infection, and the positive rate was detected after 72 hours.

### 6.3 Human primary T cell culture

The primary T cells were isolated by Ficoil method and cultured in an X-VIVO medium containing 10% FBS and 100 IU/mL IL-2, with the initial culture density being 1^{∗}10⁶/mL, and then added to a CD3- and RetroNectin r-Fibronectin (with a final concentration of 5ug/ml each)-pre-coated well plate. The density of anaphase culture was 5^{∗}10⁵/mL and up to 3^{∗}10⁶/mL, and subculture was carried out every 1 to 2 days.

### 6.4 Human primary T cell infection

After culture for 48 h, the primary T cells were added with the virus solution with MOI = 20, centrifuged at 1500 rpm for 90min, and then placed in a CO₂ incubator for culture. After 24 hours of infection, an X-VIVO medium containing 10% FBS and 100 IU/mL IL-2 was supplemented, and the wells were rotated, after 72 hours, the infection efficiency was detected by a tag protein or antibody.

### 6.5. Detection method of infection efficiency

After 72h of infection, the cells were blown evenly and counted, and taken at 5^{∗}10⁵/ml, centrifuged, then the supernatant was discarded, the staining solution used was PBS+2% FBS+2 mM EDTA, the corresponding antibody was added for incubation for 30 min, then PBS was added for washing twice, and detection was carried out on the computer.

### 7. In vitro functional assay for WT-STAR receptor and its mutant mut-STAR T cells

### 7.1. In vitro co-culture method for T cells and target cells

Target cells Raji-luciferase, Raji^{CD19KO}-luciferase, Raji^{CD20KO}-luciferase and primary T cells were suspension cells, for co-incubation, the corresponding number of cells were taken, and mixed with the target cell medium and centrifuged for culture. The specific steps were as follows: the primary T cells were infected with the packaged and purified WT-STAR and mut-STAR T viruses, and one day before co-culture, the infection efficiency was detected by flow cytometry, and the ratio of effector cell to target cell was determined and commonly used at a 1:1 ratio, and the total number of T cells was calculated according to the infection efficiency, the common usage of target cells was 1 × 10⁵/well (96-well plate).

### 7.2. Stimulation of T cells by target antigen

The target antigen of the present invention is generally a cell surface protein, which can be directly used for T cell activation to detect the function of T cells. Positive T cells were commonly added at 1 × 10⁵/well, centrifuged and activated for 24 hours, then T cells or target cells were collected to detect the T cell function.

### 7.3. T cell killing function validation: Luciferase assay

T cells were co-cultured with target cells for 24 h, then the cell suspension was gently blown evenly, 150 µ L of cell suspension per well was taken and added to a white 96-well plate, centrifuged at 1500 rpm/min for 5 min, and the supernatant was taken and added with a cell lysate for lysing at room temperature for 15 min, then centrifuged at 4 °C at 4000 rpm/min for 15min, then the supernatant was taken with 2 parallel wells being taken for each well, and added with a luciferase substrate (firefly luciferase assay reagent), then detected by a multifunctional microplate reader with the gain value fixing as 100 to obtain a chemiluminescence value. Cell killing calculation: killing efficiency = 100%- (effector cell-target cell value per well/control cell-target cell value per well).

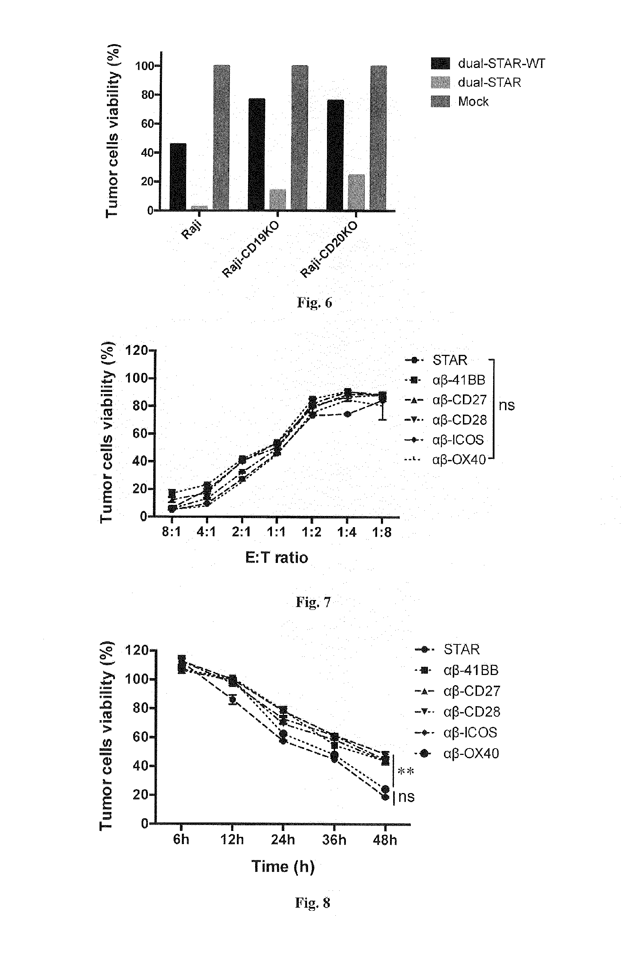

7.4. The detection results of T cell killing function test by the luciferase assay showed that mutant mut-STAR T cells exhibited a stronger T cell tumor-killing ability, as shown in Figure 6 and Table 1, after mut-STAR T cells targeting CD19 and CD20 killed Raji-luciferase, Raji^{CD19KO}-luciferase and Raji^{CD20KO}-luciferase, the survival rates of residual tumors were 2.41%, 13.94% and 24.40%, respectively, which were significantly better than those of WT-STAR by 45.73%, 77.00% and 76.16%, indicating that mutant mut-STAR had a more significant tumor-killing effect.

**Table 1 Survival rate of target cells after co-culture with wild-type-STAR and STAR (%)**

| | WT-STAR | mut-STAR | Mock |
|---|---|---|---|
| Raji | 45.73315 | 2.417751 | 100 |
| Raji-CD19KO | 77.00713 | 13.94793 | 100 |
| Raji-CD20KO | 76.16397 | 24.40734 | 100 |

### 8. Effect of costimulatory endodomain connected to the C-terminals of both TCR α chain and TCR β chain, or the C-terminal of TCR α chain or TCR β chain on the function of STAR-T cells

### 8.1. In vitro co-culture method for T cells and target cells

Target cells Raji-luciferase and primary T cells were suspension cells, for co-incubation, the corresponding number of cells were taken and mixed with the target cell culture medium, then centrifuged for culture. The specific steps were as follows: the primary T cells were infected with the packaged and purified WT-STAR and mut-STAR T viruses, and one day before co-culture, the infection efficiency was detected by flow cytometry, and the ratio of effector cell to target cell was determined, and co-incubation was carried out usually at the ratio of 8:1, 4:1, 2:1, 1:1, 1:2, 1:4 and 1:8, and the difference of co-incubation with time was also detected usually at 6 h, 12 h, 24 h, 36 h and 48 h. To detect the proliferation of co-STAR T cells, target cells Raji-luciferase were incubated with primary T cells for 7 days to observe the changes of cell proliferation number and IL-2 secretion, then positive T cells were sorted by flow cytometry and subject to resting culture without antigen stimulation for two days, which, were then cocultured again with target cells for 24 hours to detect the killing of T cells, with the common usage of target cells being 1 × 10⁵/well (96-well plate).

### 8.2. Method of stimulating T cells by target antigen

The target antigen of the present invention was generally a cell surface protein, which can be directly used for activating T cells to detect the function of T cells, in particular, the target antigen was usually added with 1 × 10⁵/well positive T cells, centrifuged and activated for 24 hours to collect the cell suspension or culture supernatant for detecting T cell function, or activated for 6 hours, 12 hours, 24 hours, 36 hours, 48 hours or 7 days to detect the killing function of T cells.

### 8.3. T cell killing function validation: Luciferase assay

T cells were co-cultured with target cells for different times, then the cell suspension was gently blown evenly, 150 µ L of cell suspension per well was taken and added to a white 96-well plate, centrifuged at 1500 rpm/min for 5 min, and the supernatant was taken and added with a cell lysate for lysing at room temperature for 15 min, then centrifuged at 4 °C at 4000 rpm/min for 15min, then the supernatant was taken with 2 parallel wells being taken for each well, and added with a luciferase substrate (firefly luciferase assay reagent), then detected by a multifunctional microplate reader with the gain value fixing as 100 to obtain a chemiluminescence value. Cell killing calculation: killing efficiency = 100%- (effector cell-target cell value per well/control cell-target cell value per well).

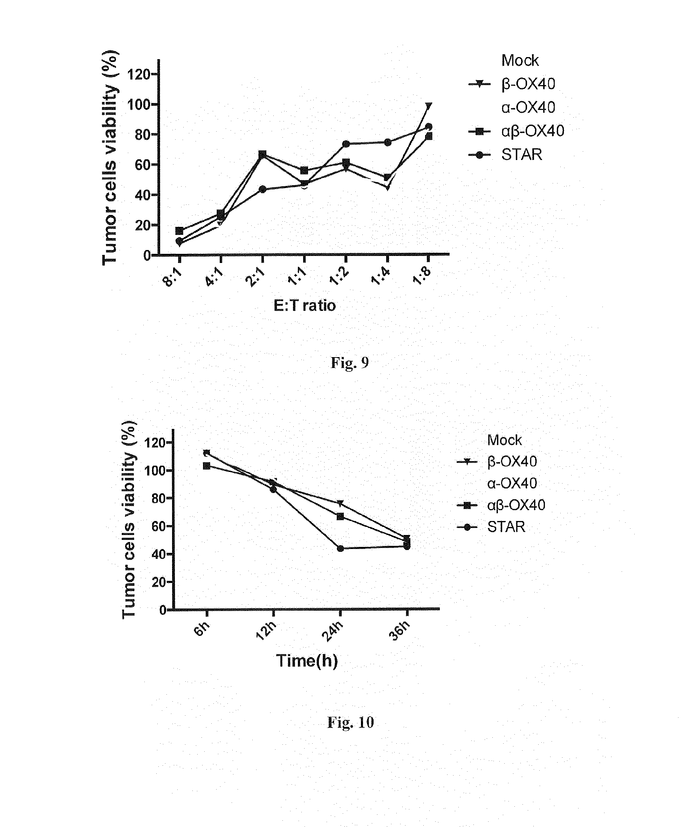

The detection results of T cell killing function test by the luciferase assay showed that mut-STAR with costimulatory endodomain connected to the C-terminals of both TCR α chain and β chain exhibited similar tumor killing effect at different E:T ratios of T cells and target cells, and there was no significant difference in residual tumor survival rate at different E:T ratios, as shown in Fig. 7 and Table 2. However, in terms of the detection results of tumor killing at different time, mut-STAR with OX40 endodomains connected to the C-terminals of both TCR α and β chains (αβ-OX40 and mut-STAR with incubation with Raji-luciferase for 48 h showed similar tumor killing effects, about 24% and 19%, respectively, which were significantly superior to mut-STARs with other costimulatory endodomains connected to the C-terminals of both TCR α and β chains (αβ-41BB, αβ-CD27, αβ-CD28, αβ-ICOS), as shown in Fig. 8 and Table 3. Based on the above results, it was found that linking the endodomain of costimulatory molecule OX40 to the C-terminals of both TCR α and β chains did not affect the killing effect of mut-STAR. In addition, based on the above results, mut-STAR with OX40 endodomain linked to the C terminal of TCR α chain (α-OX40) or β chain (β-OPX40 alone was detected on T cell killing function by the luciferase assay, and the detection results showed that, at different E:T ratios of T cell to co-incubated tumor cell or 24 hours of co-incubation, α-OX40 and β-OPX4 did not show significant difference from α-β-OX40, as shown in Figs. 9 and 10, and Tables 4 and 5. Based on the above killing results, there was no significant difference between the mut-STAR with the endodomain of costimulatory molecule OX40 linked to the C-terminals of both TCR α chain and β chain (αβ-OPX40 and mut-STAR with the endodomain of costimulatory molecule OX40 linked to the C-terminal of TCR α chain (α-OX40) or β chain (β-OPX40 alone, but their tumor killing ability was significantly better than that of mut-STAR.

### 8.4. Analysis of cytokine secretion by T cells: ELISA

During T cell activation, a large number of cytokines, such as TNF-α, IFN-γ and IL-2, were released to help T cells kill target cells or promote the expansion of T cells themselves. After T cells were stimulated by target cells or antigens, T cells were collected, centrifuged, and the supernatant was taken. The TNF-α, IFN-γ, and IL-2 ELISA kits used were Human IL-2 Uncoated ELISA, Human TNF-α Uncoated ELISA, and Human IFN-γ Uncoated ELISA (Art.No. 88-7025, 88-7346, 88-7316, respectively). The specific steps were as follows: diluting 10X Coating Buffer to 1X with ddH₂O, adding coated antibody (250X), mixing well and adding to a 96-well plate at 100 µL/well. After sealing with the plastic wrap and staying overnight at 4 °C, 1X PBST (1X PBS with 0.05% Tween 20 added) was used for washing 3 times with 260 µL/well each time, 5X ELISA/ELISPOT Diluent was diluted to 1X with ddH₂O and then added to the 96-well plate with 200 µL/well, followed by standing at room temperature for 1 hour. PBST was used for washing once, and dilution was performed according to a standard curve (ranging from: 2 to 250, 4 to 500, 4 to 500, respectively), the samples were diluted to 20 to 50 times with 1xDiluent. Samples diluted according to the standard curve were added with 100 microliters per well, two parallel wells were taken and incubated at room temperature for 2 hours, PBST was used for washing three times, then a Detection antibody diluted with 1×Diluent was added, after incubation for 1 h, PBST was used for washing three times, then HRP diluted with 1×Diluent was added, after incubation for 30 minutes, the solution was washed six times, TMB was added for color development with the color development time being less than 15min, and 2N H₂SO₄ was added for termination, light absorption at 450 nm was detected.

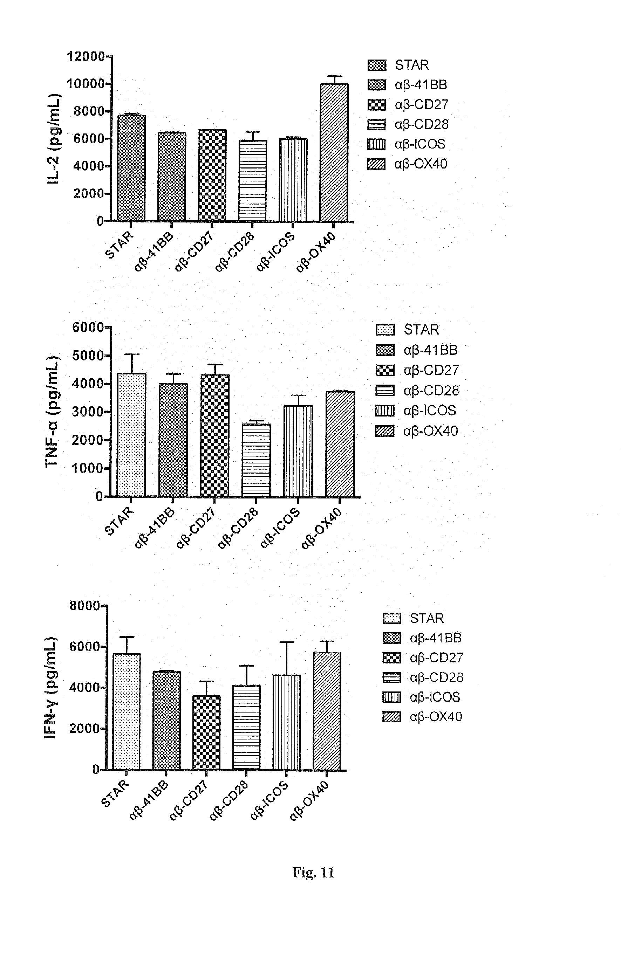

The ELISA results showed that, after T cells were co-incubated with target cells for 24 h, the IL-2 secretion of mut-STAR (αβ-OX40) with OX40 endodomain linked to the C-terminals of both TCR α chain and β chain was about 10000 pg/ml, which was significantly higher than that of STAR with other structures, in which that of mut-STAR, αβ-41BB, αβ-CD27, αβ-CD28 and αβ-ICOS was about 7700 pg/ml, 6450 pg/ml, 6690 pg/ml, 6000 pg/ml, and 6050 pg/ml, respectively; in terms of TNF α and IFN-γ secretion, αβ-OX40 also showed similar results with mut-STAR, while other structures showed different decreases, as shown in Figure 11 and Table 6. Based on the ELISA results, it was shown that the IL-2 secretion by mut-STAR with the endodomain of costimulatory molecule OX40 linked to the C-terminals of both TCR α chain and β chain (αβ-OX40) was significantly higher than that by mut-STAR.

### 8.5. Detection of T cell proliferation changes: counting by flow cytometry

During T cell activation, a large number of cytokines were released to help T cells kill target cells or promote the expansion of T cells themselves, and the most obvious occurrence in T-cell proliferation was a significant change in the number of T cells. T cells were incubated with target cells for 7 days, then centrifuged, resuspended to 200 uL with PBS, and the number of positive T cells was counted by flow cytometry. Changes of T cell proliferation: Proliferation fold = number of positive T cells after 7 days/initial number of positive T cells added.

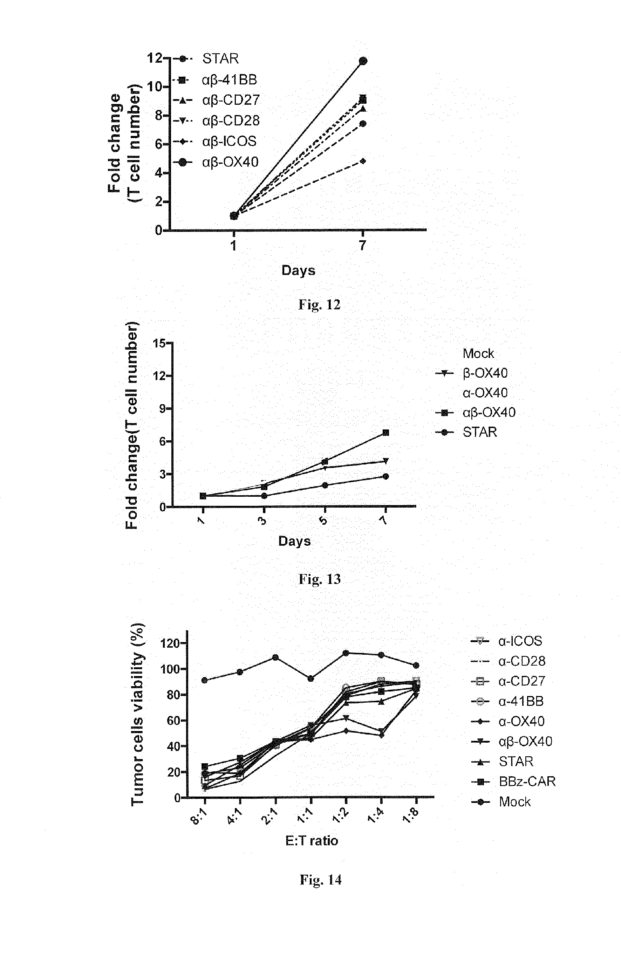

After sorting, mut-STAR-T cells with various structures were co-cultured with Raji-luciferase cells at a 1: 3 E:T ratio with counting as day 0, and then cells were collected on day 1 and day 7 for flow analysis, respectively. Among them, the medium used was 1640 complete medium without IL-2, and the initial number of TCR T cells was 1 × 10⁵ Cells, samples at each time point were incubated independently, and the remaining co-incubated samples were semi-changed with the liquid the next day, and supplemented with the target cells. The cells used for flow analysis were stained with an anti-human CD3 antibody in advance, and a specified volume thereof was collected and recorded when analysis was performed on the machine, the number and proportion of T cells in the system were known by conversion. As shown in Fig. 12 and Table 7, it can be seen from the proliferation fold curve of absolute number of mut-STAR cells, mut-STAR with OX40 endodomain connected to the C-terminals of both TCR α chain and β chain(αβ-OX40) showed better activation and proliferation after recognizing the target cell epitope, which were significantly higher that by mut-STAR. The proliferation of mut-STAR with OX40 endodomain connected to the C-terminal of TCR α chain (α-OX40) or β chain (β-OPX40 alone was shown in Fig. 13 and Table 8, wherein, the T cell proliferation of mut-STAR with OX40 endodomain connected to the C-terminal of TCR α chain (α-OX40) alone after 7 days was 11.75 times, while that of other structures like mut-STAR, β-OX40 and αβ-OX40 was 2.755 times, 4.128 times and 6.744 times, respectively, thus the proliferation effect of α-OX40 was significantly higher than that of other structures. Combining the above tumor killing results, ELISA results and T cell proliferation results, mut-STAR with OX40 endodomain connected to the C-terminal of TCR α chain (α-OX40) alone showed more enhanced proliferation and tumor killing abilities than other structures.

According to the above results, it was found that the best proliferation effect was obtained by linking the costimulatory molecule OX40 to the TCR-α chain without affecting the killing effect of T cells as well. Therefore, mut-STAR with the intracellular domains of different costimulatory molecules connected in tandem to the TCR α chain. The effector to target ratio of different T cells and target cells showed that the killing effect of the intracellular domain of costimulatory molecule OX40 linked to mut-STAR was better than that of STAR with other costimulatory domain linked. At the 1: 2 and 1: 4 E:T ratios, the effect of mut-STAR T cells with the endodomain of costimulatory molecule OX40 connected (α-OX40) was similar to that of αβ-OX40, and was better than that of mut-STAR T cells with other costimulatory molecules connected. The results were shown in Fig. 14 and Table 9. Based on the above killing results, the tumor-killing and proliferation abilities obtained by mut-STAR with OX40 endodomain linked to the TCR α chain (α-OX40) were significantly superior to that of mut-STAR.

**Table 2 Survival rate of target cells after co-culture with co-STAR at different E:T ratios (%)**

| | STAR | | αβ-41BB | | αβ-CD27 | | αβ-CD28 | | αβ-ICOS | | αβ-OX40 | |
|---|---|---|---|---|---|---|---|---|---|---|---|---|
| 8:1 | 4.799897 | 5.123116 | 18.67158 | 15.22742 | 13.44601 | 10.93907 | 6.576478 | 5.593634 | 7.02171 | 5.671445 | 5.961419 | 4.986681 |
| 4:1 | 9.421389 | 9.79921 | 23.21254 | 23.37164 | 16.94656 | 17.99448 | 12.80805 | 13.68354 | 18.94662 | 19.90719 | 7.852874 | 7.858923 |
| 2:1 | 28.26214 | 26.46111 | 42.01635 | 41.7816 | 41.09309 | 40.13039 | 32.51979 | 31.71177 | 40.25441 | 39.5446 | 25.32787 | 25.33127 |
| 1:1 | 46.26518 | 46.00616 | 54.0254 | 53.39529 | 49.70481 | 51.02658 | 49.61742 | 49.38536 | 53.68495 | 54.07269 | 45.44757 | 45.05678 |
| 1:2 | 73.39803 | 73.40839 | 85.07728 | 85.5246 | 81.83936 | 82.06654 | 80.47409 | 79.98853 | 79.0582 | 79.14856 | 75.25034 | 75.78191 |
| 1:4 | 74.8484 | 74.12886 | 90.07524 | 91.7365 | 89.8961 | 91.43537 | 86.2978 | 87,55266 | 87.96474 | 89.28484 | 84.03136 | 85.54089 |
| 1:8 | 85.01962 | 83.91612 | 87.48048 | 86.96379 | 87.89702 | 89.04024 | 89.42363 | 86,90845 | 90.04101 | 88.12827 | 87.64616 | 73.40806 |

**Table 3 Survival rate of target cells after co-culture with co-STAR for different times (%)**

| | STAR | | αβ-41BB | | αβ-CD27 | | αβ-CD28 | | αβ-ICOS | | αβ-OX40 | |
|---|---|---|---|---|---|---|---|---|---|---|---|---|
| 6h | 110.8255 | 113.647 | 105.055 | 108.66 | 111.1988 | 114.3491 | 105.0322 | 108.0125 | 110.349 | 115.3392 | 106.7645 | 107.607 |
| 12h | 88.38038 | 83.85848 | 101.3089 | 101.0553 | 98.17051 | 96.44521 | 97.93871 | 100.5614 | 101.036 | 99.22498 | 100.3137 | 99.78574 |
| 24h | 57.95153 | 57.2674 | 78.16129 | 79.72054 | 72.22607 | 73.63323 | 69.23749 | 69.64558 | 80.64219 | 76.07741 | 62.38043 | 62.93139 |
| 36h | 44.94864 | 44.96385 | 60.20564 | 49.20376 | 61.29437 | 61.55751 | 58.78605 | 58.368 | 62.44511 | 60.90037 | 48.59327 | 47.75637 |
| 48h | 19.37016 | 18.59608 | 45.55151 | 42.05922 | 45.89704 | 43.15755 | 44.77774 | 42.81658 | 49.77628 | 47.4759 | 24.93932 | 23,80572 |

**Table 4 Survival rate of target cells after co-culture with STAR with OX40 added to α chain and β chain respectively at different E:T ratios (%)**

| | STAR | αβ-OX40 | α-OX40 | β-OX40 | Mock |
|---|---|---|---|---|---|
| 8:1 | 9.44121 | 16.13121 | 19.39611 | 7.617087 | 91.18241 |
| 4:1 | 25.34729 | 27.43528 | 18.93089 | 19.52659 | 97.55484 |
| 2:1 | 43.53329 | 66.69584 | 49.92537 | 65.71404 | 108.807 |
| 1:1 | 46.13567 | 55.89357 | 44.82812 | 46.98217 | 92.18459 |
| 1:2 | 73.40321 | 61.13256 | 51.51919 | 56.88129 | 112.0738 |
| 1:4 | 74.48863 | 51.00451 | 48.09981 | 44.48893 | 110.6069 |
| 1:8 | 84.46786 | 78.43122 | 82.91417 | 98.08592 | 102.34 |

**Table 5 Survival rate of target cells after co-culture with STAR with OX40 added to α chain and β chain respectively for different times (%)**

| | STAR | αβ-OPX4 | α-OX40 | β-OX40 | Mock |
|---|---|---|---|---|---|
| 6h | 112.2362 | 103.2818 | 111.031 | 112.0466 | 117.3142 |
| 12h | 86.11943 | 91.64006 | 93.62347 | 89.51228 | 129.1662 |
| 24h | 43.53329 | 66.69584 | 59.92537 | 75.71404 | 108.807 |
| 36h | 44.95625 | 48.22791 | 48.10436 | 50.55336 | 91.45656 |

**Table 6**

| IL-2 secretion after co-culture of co-STAR with target cells | | | | | | | | | | | |
|---|---|---|---|---|---|---|---|---|---|---|---|
| STAR | | αβ-41BB | | αβ-CD27 | | αβ-CD28 | | αβ-ICOS | | αβ-OX40 | |
| 7806. 818 | 7613. 636 | 6477. 273 | 6429. 455 | 6704. 545 | 6681. 818 | 5522. 727 | 6375 | 5988. 636 | 6136. 364 | 1047 7.27 | 9659. 091 |
| | | | | | | | | | | | |

| IFN-α secretion after co-culture of co-STAR with target cells | | | | | | | | | | | |
|---|---|---|---|---|---|---|---|---|---|---|---|
| STAR | | αβ-41BB | | αβ-CD27 | | αβ-CD28 | | αβ-ICOS | | αβ-OX40 | |
| 3891. 304 | 4847. 826 | 3760. 87 | 4260. 87 | 4586. 957 | 4065. 217 | 2489. 13 | 2673. 913 | 3500 | 2978. 261 | 3717. 391 | 3782. 609 |
| | | | | | | | | | | | |

| IFN-γ secretion after co-culture of co-STAR with target cells | | | | | | | | | | | |
|---|---|---|---|---|---|---|---|---|---|---|---|
| STAR | | αβ-41BB | | αβ-CD27 | | αβ-CD28 | | αβ-ICOS | | αβ-OX40 | |
| 6238. 636 | 5058. 182 | 4750 | 4840. 909 | 4125 | 3090. 909 | 4818. 182 | 3465. 909 | 3511. 364 | 5795. 455 | 6147. 727 | 5397. 727 |

**Table 7 Proliferation after co-culture of co-STAR with target cells**

| time | **STAR** | **αβ-41BB** | **αβ-CD27** | **αβ-CD28** | **αβ-ICOS** | **αβ-OX40** |
|---|---|---|---|---|---|---|
| **1** | **1** | **1** | **1** | **1** | **1** | **1** |
| **7** | **7.420446** | **9.043757** | **8.495342** | **9.200999** | **4.818081** | **11.79747** |

**Table 8 Comparison of proliferation after co-culture of target cells with STAR comprising OX40 connected to α or β chain thereof**

| | **STAR** | **αβ-OX40** | **α-OX40** | **β-OX40** | **Mock** |
|---|---|---|---|---|---|
| **1** | **1** | **1** | **1** | **1** | **1** |
| **3** | **0.990937** | **1.829041** | **2.178261** | **2.120637** | **2.28979** |
| **5** | **1.94469** | **4.152329** | **4.461739** | **3.537936** | **3.772773** |
| **7** | **2.755287** | **6.74411** | **11.75826** | **4.128983** | **3.64965** |

**Table 9**

| | **Survival rate of target cells after co-culture of STAR with target cells at different E:T ratios (%)** | | | | | | | | |
|---|---|---|---|---|---|---|---|---|---|
| E:T ratio | **Mock** | **BBz-CAR** | **STAR** | **αβ-ox40** | **α-OPX4** | **α-41BB** | **α-CD27** | **α-CD28** | **α-ICOS** |
| **8:1** | **91.18241** | **24.26644** | **9.44121** | **16.13121** | **19.39611** | **18.67158** | **13.44601** | **6.576478** | **7.02171** |
| **4:1** | **97.55484** | **30.57931** | **25.34729** | **27.43528** | **18.93089** | **23.21254** | **16.94656** | **12.808050** | **18.94662** |
| **2:1** | **108.80700** | **43.73347** | **43.53329** | **43.73347** | **43.53329** | **42.01635** | **41.09309** | **32.519790** | **40.25441** |
| **1:1** | **92.18459** | **49.60269** | **46.13567** | **55.89357** | **44.82812** | **54.02540** | **49.70481** | **49.617420** | **53.68495** |
| **1:2** | **112.07380** | **77.94164** | **73.40321** | **61.13256** | **51.51919** | **85.07728** | **81.83936** | **80.474090** | **79.05820** |
| **1:4** | **110.60690** | **82.05241** | **74.48863** | **51.00451** | **48.09981** | **90.07524** | **89.89610** | **86.297800** | **87.96474** |
| **1:8** | **102.34000** | **84.89436** | **84.46786** | **78.43122** | **82.91417** | **87.48048** | **87.89702** | **89.423630** | **90.04101** |

### 9. Effect of costimulatory structures connected to different CD3 chains (co-CD3-STAR) on the function of STAR-T cells

### 9.1. In vitro co-culture method for T cells and target cells

Target cells Raji-luciferase and primary T cells were suspension cells, for co-incubation, the corresponding number of cells were taken and mixed with the target cell culture medium, then centrifuged for culture. The specific steps were as follows: the primary T cells were infected with the packaged and purified WT-STAR and mut-STAR T viruses, and one day before co-culture, the infection efficiency was detected by flow cytometry, and the ratio of effector cell to target cell was determined, co-incubation was carried out usually at the ratio of 1:1 or 2:1, in addition, target cells Raji-luciferase and primary T cells were co-incubated for 7 days to observe the changes in the number of cell proliferation. The total number of T cells was calculated based on the infection efficiency, and the general usage of target cells was 1 × 10⁵/well (96-well plate).

### 9.2. Stimulation of T cells by target antigen

The target antigen of the present invention is generally a cell surface protein, which can be directly used for T cell activation to detect the function of T cells. positive T cells were commonly added with 1 × 10⁵/well, centrifuged and activated for 24 hours, the cell suspension or culture supernatant was collected to detect the T cell function.

### 9.3. T cell killing function validation: Luciferase assay

T cells were co-cultured with target cells for different times, then the cell suspension was gently blown evenly, 150 µ L of cell suspension per well was taken and added to a white 96-well plate, centrifuged at 1500 rpm/min for 5 min, and the supernatant was taken and added with a cell lysate for lysing at room temperature for 15 min, then centrifuged at 4 °C at 4000 rpm/min for 15min, then the supernatant was taken with 2 parallel wells being taken for each well, and added with a luciferase substrate (firefly luciferase assay reagent), then detected by a multifunctional microplate reader with the gain value fixing as 100 to obtain a chemiluminescence value. Cell killing calculation: killing efficiency = 100%- (effector cell-target cell value per well/control cell-target cell value per well).

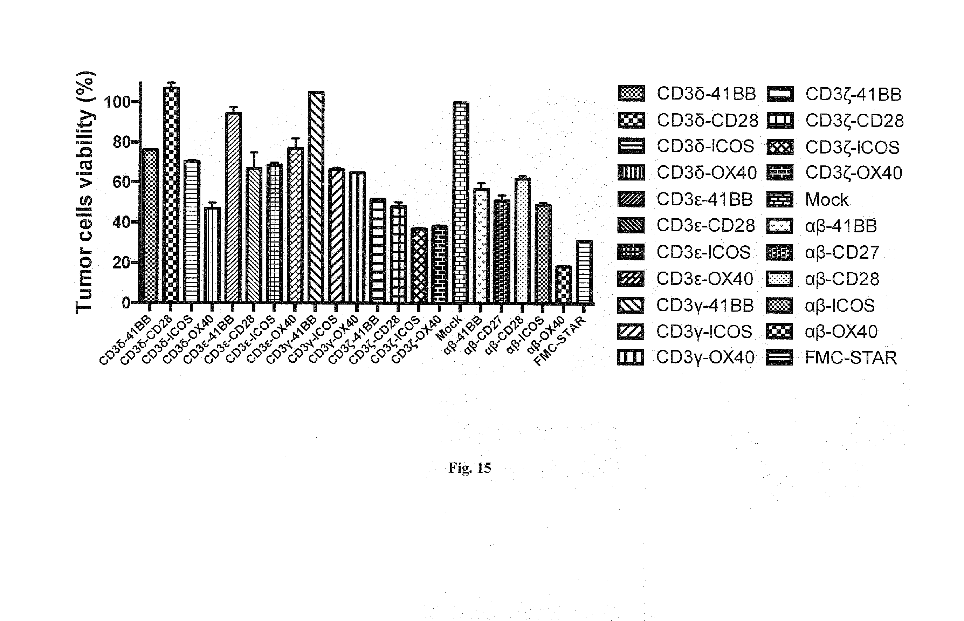

The detection results of T cell killing function test by the luciferase assay showed that when the costimulatory endodomain was connected to the C-terminal of CD3 δ or CD3 γ or CD3 ε or CD3 ζ chain and co-expressed on mut-STAR T, the results at the 1: 1 E:T ratio of different T cells to target cells showed that all co-CD3-STAR did not exhibit a better target cell-killing effect than aβ-OX40, but compared with mut-STAR, CD3δ-OX40, CD3ζ-41BB, CD3ζ-CD28, CD3ζ-ICOS, CD3ζ-OX40 exhibited similar tumor killing effects, residual tumor survival rates thereof did not differ significantly at the 1: 1 E:T ratio, as shown in Fig.15 and Table 10. Based on the above results, by linking the costimulatory endodomain to the C-terminal of different CD3 chains, the tumor killing effect of mut-STAR was not significantly improved, but the effect of mut-STAR with the costimulatory endodomain linked to the C-terminal of CD3 ζ chain was not significantly decreased.

### 9.4 Detection of T cell proliferation changes: counting by flow cytometry

During T cell activation, a large number of cytokines were released to help T cells kill target cells or promote the expansion of T cells themselves, and the most obvious occurrence in T-cell proliferation was a significant change in the number of T cells. T cells were incubated with target cells for 7 days, then centrifuged, resuspended to 200 uL with PBS, and the number of positive T cells was counted by flow cytometry. Changes of T cell proliferation: Proliferation fold = number of positive T cells after 7 days/initial number of positive T cells added.

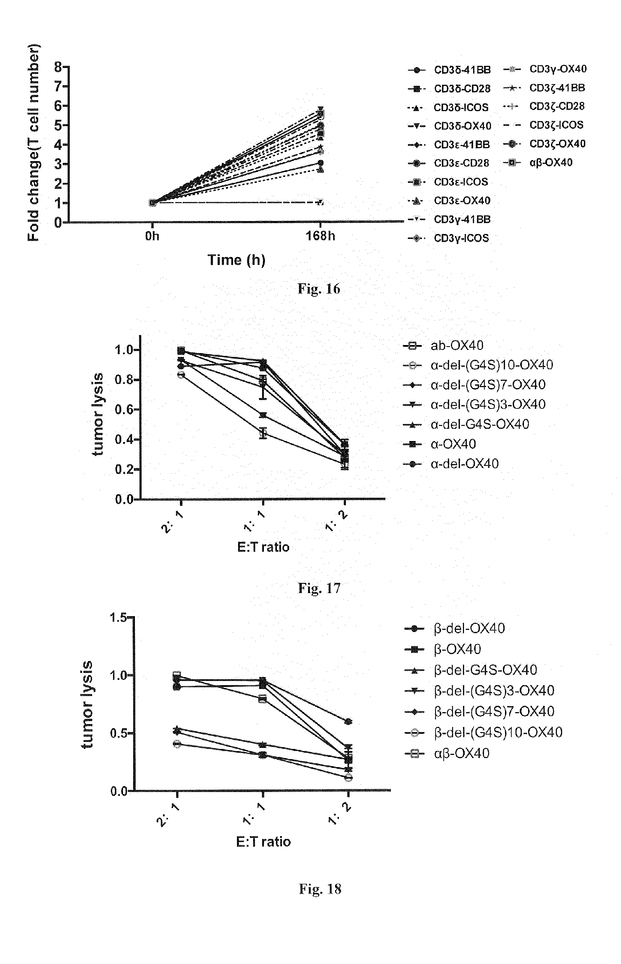

After sorting, mut-STAR-T cells with various structures were co-cultured with Raji-luciferase cells at a 1: 3 E:T ratio with counting as day 0, and then cells were collected on day 1 and day 7 for flow analysis, respectively. Among them, the medium used was 1640 complete medium without IL-2, and the initial number of TCR T cells was 1 × 10⁵ Cells, samples at each time point were incubated independently, and the remaining co-incubated samples were semi-changed with the liquid the next day, and supplemented with the target cells. The cells used for flow analysis were stained with an anti-human CD3 antibody in advance, and a specified volume thereof was collected and recorded when analysis was performed on the machine, the number and proportion of T cells in the system were known by conversion. As shown in Fig. 16 and Table 11, it can be seen from the proliferation fold curve of absolute number of mut-STAR cells that all co-CD3-STAR did not exhibit a better target cell killing effect as compared with αβ-OX40-mut-STAR, but CD3 ε-CD28, CD3 δ-OX40, CD3 ζ-CD28, CD3 ζ-ICOS, CD3 ζ-OX40 and the like exhibited similar proliferation effects. Combing the detection results of killing by the luciferase assay and T cell proliferation results, the structure with the costimulatory endodomain connected to the C terminal of CD3 δ or CD3 γ or CD3 ε or CD3 ζ chain and co-expressed on mut-STAR T, did not show better effects of tumor killing and proliferation, as compare to αβ-OX40-mut-STAR T cells.

**Table 10 Survival rate of target cells after co-culture with STAR with costimulatory domain connected to other CD3 endodomains**

| | | | | | | | | | |
|---|---|---|---|---|---|---|---|---|---|
| CD3δ-4 1BB | | CD3δ-CD 28 | | CD3δ-ICOS | | CD3δ -OX40 | | | |
| 75.94348 | 76.26161 | 108.7582 : | 104.9861 | 10.85791 | 70.03172 | 48.96212 | 45.08736 | | |
| CD3ε-41 BB | | CD3ε-CD 28 | | CD3ε-ICOS | | CD3ε-OX40 | | | |
| 96.51971 | 92.18439 | 72,59823 | 61.09674 | 69,34229 | 67.72803 | 80.5375 | 73.27773 | | |
| CD3γ-41 BB | | | | CD3γ-ICOS | | CD3γ -OX40 | | | |
| 104.8364 | 104.7641 | | | 65.88973 | 67.07659 | 65.03455 | 64.71774 | | |
| CD3ζ-41 BB | | CD3ζ-CD 28 | | CD3ζ-ICOS | | CD3ζ-OX40 | | | |
| 51.69385 | 51.38356 | 49.51813 | 46.66462 | 36.58346 | 37.27512 | 37.99206 | 38.57246 | | |
| αβ-41B B | | aβ-CD 27 | | αβ-CD28 | | αβ-ICOS | | αβ-O X40 | |
| 58.97096 | 54.71444 | 53.0418 | 48.9068 | ! 61.06239 | 63.08836 | 49 .73116 | \| 48.13088 | 18.25028 | 18.60128 |
| Mock | | FMC-ST AR | | | | | | | |
| 100 | 100 | 31.10368 | 31.41209 | | | | | | |

**Table 11 Proliferation after co-culture of STAR with costimulatory domain connected to other CD3 endodomains with target cells for 7 days**

| | CD3δ-41BB | CD3δ-CD28 | CD3δ-ICOS | CD3δ-OX40 |
|---|---|---|---|---|
| 0h | 1 | 1 | 1 | 1 |
| 168h | 3.034644 | 1 | 4.340684 | 5.782042 |
| | CD3ε-41BB | CD3ε-CD28 | CD3ε-ICOS | CD3ε-OX40 |
| 0h | 1 | 1 | 1 | 1 |
| 168h | 4.785835 | 5.396289 | 4.549294 | 2.722988 |
| | CD3γ-41BB | | CD3γ-ICOS | CD3γ-OX40 |
| 0h | 1 | | 1 | 1 |
| 168h | 1 | | 3.624776 | 3.605788 |
| | CD3ζ-41BB | CD3ζ-CD28 | CD3ζ-ICOS | CD3ζ-OX40 |
| 0h | 1 | 1 | 1 | 1 |
| 168h | 3.866649 | 4.98899 | 5.272135 | 4.948633 |
| | αβ-OX40 | | | |
| 0h | 1 | | | |
| 168h | 5.584149 | | | |

### 10. Effects of costimulatory endodomain comprising linker G₄S connected to endodomain-deleted α or β constant region on the function of STAR-T cells

### 10.1. In vitro co-culture method for T cells and target cells

Target cells Raji-luciferase and primary T cells were suspension cells, for co-incubation, the corresponding number of cells were taken and mixed with the target cell culture medium, then centrifuged for culture. The specific steps were as follows: the primary T cells were infected with the packaged and purified WT-STAR and mut-STAR T viruses, and one day before co-culture, the infection efficiency was detected by flow cytometry, and the ratio of effector cell to target cell was determined, and co-incubation was carried out usually at a 1:1 or 2:1 ratio, in addition, target cells Raji-luciferase and primary T cells were co-incubated for 7 days to observe the changes in the number of cell proliferation. The total number of T cells was calculated based on the infection efficiency, and the general usage of target cells was 1 × 10⁵/well (96-well plate).

### 10.2. Stimulation of T cells by target antigen

The target antigen of the present invention was generally a cell surface protein, which can be directly used for activating T cells to detect the function of T cells, in particular, the target antigen was usually added with 1 × 10⁵/well positive T cells, centrifuged and activated for 24 hours to collect the cell suspension or culture supernatant for detecting T cell function.

### 10.3. T cell killing function validation: Luciferase assay

T cells were co-cultured with target cells for different times, then the cell suspension was gently blown evenly, 150 µ L of cell suspension per well was taken to a white 96-well plate, centrifuged at 1500 rpm/min for 5 min, and the supernatant was taken and added with a cell lysate for lysing at room temperature for 15 min, then centrifuged at 4 °C at 4000 rpm/min for 15min, then the supernatant was taken with 2 parallel wells being taken for each well, and added with a luciferase substrate (firefly luciferase assay reagent), then detected by a multifunctional microplate reader with the gain value fixing as 100 to obtain a chemiluminescence value. Cell killing calculation: killing efficiency = 100%- (effector cell-target cell value per well/control cell-target cell value per well).

The detection results of T cell killing function test by the luciferase assay showed that, the costimulatory endodomain with different lengths of G4S linker was connected to the endodomain-deleted α or β constant region, the results at the 1: 1 E:T ratio of T cell to target cell showed that, when the linker was connected to the α constant region endodomain, α-del-OX40, α-OX40, α-del-G4S-OX40, α-del-(G4S) 3-OX40 and α-β-οx40 showed similar killing effect, which was superior to that of α-del-(G4S) 7-OX40 and α-del-(G4S) 10-OX40, and the longer linker, the weaker the T cell killing effect, when the linker was connected to the β constant region endodomain, β-del-OX40, β-ox40, β-del-(G4S) 3-OX40 and α-β-οx40 showed similar killing effect, and still, the longer the linker, the weaker the T cell killing effect. Connection to OX40 (α-del-OX40 or β-del-OX40) after removal of the constant region endodomain showed little difference in the effect compared with that without such removal, but after addition of the linker (the number of linker was not more than 3), the effect of α-del-( G4S) 1-3-OX40 or β-del-( G4S) 1-3-OX40 was better than that of α-del-OX40 or β-del-OX40. The comparison between the effect when the linker was connected to the α constant region endodomain and the effect when the linker was connected to the β constant region endodomain showed that, the effect when the linker was connected to the α constant region endodomain was better than that when the linker was connected to the β constant region endodomain. However, there was no significant difference in residual tumor survival rate at a 2: 1 E:T ratio, as shown in Figures 17 and 18, and Tables 12 and 13. Based on the above results, mut-STAR with a costimulatory endodomain comprising a G4S linker linked to the endodomain-deleted α or β constant region did not affect the tumor killing effect of the mut-STAR, and the effect when linking to the α constant region endodomain was better than that when linking to the β constant region endodomain, but the longer the linker length, the weaker the tumor killing effect instead.

### 10.4. Analysis of cytokine secretion by T cells: ELISA

During T cell activation, a large number of cytokines, such as TNF-α, lFN-γ and IL-2, were released to help T cells kill target cells or promote the expansion of T cells themselves. After T cells were stimulated by target cells or antigens, T cells were collected, centrifuged, and the supernatant was taken. The TNF-α, lFN-γ, and IL-2 ELISA kits used were Human IL-2 Uncoated ELISA, Human TNF-α Uncoated ELISA, and Human lFN-γ Uncoated ELISA (Art.No. 88-7025, 88-7346, 88-7316, respectively). The specific steps were as follows: diluting 10X Coating Buffer with ddH2O to 1X, adding coated antibody (250X), mixing well and adding to a 96-well plate at 100 µL/well. After sealing with the plastic wrap and staying overnight at 4 °C, 1X PBST (also called Wash Buffer, 1X PBS with 0.05% Tween 20 added) was used for washing 3 times with 260 µL/well each time, 5X ELISA/ELISPOT Diluent was diluted to 1X with ddH₂O and then added to the 96-well plate with 200 µL/well, followed by standing at room temperature for 1 hour. PBST was used for washing once, and dilution was performed according to a standard curve (ranging from: 2 to 250, 4 to 500, 4 to 500, respectively), the samples were diluted to 20 to 50 times with 1xDiluent. Samples according to the standard curve were added with 100 microliters per well, two parallel wells were taken and incubated at room temperature for 2 hours, PBST was used for washing three times, then a Detection antibody diluted with 1xDiluent was added, after incubation for 1 h, PBST was used for washing three times, then HRP diluted with 1xDiluent was added, after incubation for 30 minutes, the solution was washed six times, TMB was added for color development with the color development time being less than 15min, and 2N H₂SO₄ was added for termination, light absorption at 450 nm was detected.

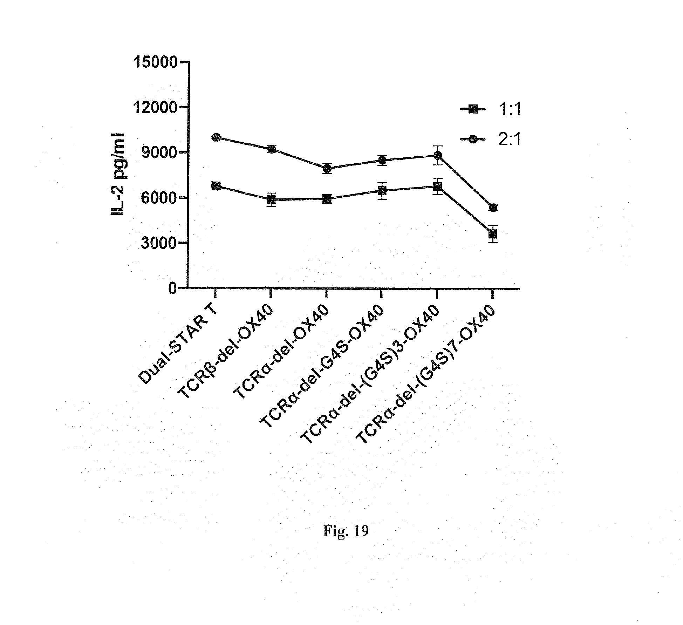

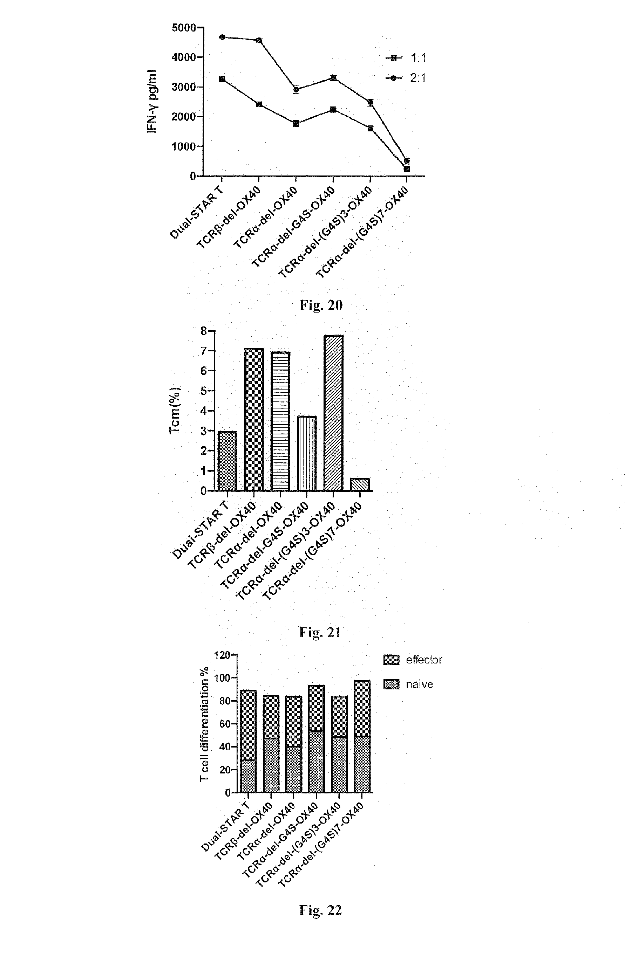

The results of ELISA showed that, after T cells were co-incubated with target cells for 24 h, the secretions of IL-2 and lFN-γ of mut-STAR with a structure of α-del-(G4S)7-OX40 OX40 were significantly lower than those of mut-STAR, while the IL-2 secretion of other structures β-del-OX40, α-del-OX40, α-del-G4S-OX40 and α-del-(G4S) 3-OX40 each was similar to that of mut-STAR; only β-del-OX40 exhibited a similar lFN-γ secretion to that of mut-STAR, while the secretion of other structures exhibited a different decrease, as shown in Figures 19 and 20, and Tables 14 and 15. According to the ELISA results, in a structure with the endodomain of α or β constant region was deleted, the deletion of the endodomain of α chain affected the secretion, but did not affect the IL-2 secretion, while the deletion of the endodomain of β chain did not affect the secretion of neither IL-2 nor IFN-y; at the same time, after the endodomain of α chain was deleted, a linker was used to link to the OX40 endodomain, which, in case of the linker with a length of less than 7, did not affect the IL-2 secretion, but decreased the secretion.

### 10.5. Detection of T cell differentiation changes: analysis by flow cytometry

During T cell activation, a large number of cytokines and other chemokines were released, and signals were transduced into the nucleus through cytokines or chemokine receptors to regulate the differentiation of T cells. T cells differentiate from primitive T cells (naive) to central memory T cells (Tcm) to effector memory T cells (Tern), and finally to effector T cells (Teff). However, the proliferation and persistence of T cells in vivo are affected by the number of T cells differentiated to central memory T cells (Tcm) to effector memory T cells (Tern). Memory T cells can be classified into stem cell T cells, central memory T cells and effector memory T cells. The differentiation ratio of central memory T cells affects the persistent killing effect of T cells in vivo. The ratio of primitive T cells to effector T cells affects the tumor-killing effect and persistence of T cells in vivo. The expression of CD45RA and CCR7 on the surface of T cells was detected by flow cytometry, thus the differentiation of T cells can be known. T cells were incubated with target cells for 7 days, centrifuged, stained with anti-human-CD45RA-Percp-cy5.5 and anti-human-CCR7-APC flow antibodies for 30 minutes, centrifuged again, washed with PBS and fixed with 4% paraformaldehyde solution, and the differentiation of T cells was detected by flow cytometry.

The results of flow cytometry showed that, when the G4S linker-containing costimulatory endodomain was linked to the structure with the endodomain of α or β constant region deleted, the obtained α-del-(G4S)3-OX40 showed significant differentiation of central memory T cells, while the structure with OX40 directly connected to the endodomain of α (α-del-OX40) or β (β-del-OX40) constant region also promoted differentiation of central memory T cells, as shown in Fig. 21 and Table 16. At the same time, although the number of effector T cells of mut-STAR T cells with various modified structures was significantly lower than that of mut-STAR, the proportion of naive T cells each was significantly higher than that of mut-STAR (28.3%), as shown in Figure 22 and Table 17. Combining tumor killing results, ELISA results and flow cytometry detection of cell differentiation, mut-STAR with the endodomain of α or β constant region deleted and connected to OX40 endodomain via a (G4S)₃ linker, can significantly improve the differentiation of memory cell population of T cells without affecting tumor killing effect and IL-2 secretion.

**Table 12 Effects of killing target cells after co-culture of target cells with STAR with OX40 connected to α chain thereof via different linkers**

| | α-del-OX40 | | α-OX40 | | α-del-G4SOX40 | | α-del-[G4S]3 -OX40 | | α-del-[G4S]7 -OX40 | | α-del-[G4S]1 0-OX40 | | ab-OX40 | |
|---|---|---|---|---|---|---|---|---|---|---|---|---|---|---|
| 2 : 1 | 0.890 328 | 0.888 4054 | 0.993 3094 | 0.992 7593 | 0.987 0744 | 0.987 2119 | 0.932 0745 | 0.926 8892 | 0.932 1665 | 0.935 556 | 0.832 1665 | 0.835 556 | 0.995 7843 | 0.996 5176 |
| 1 : 1 | 0.916 0888 | 0.915 0926 | 0.879 0945 | 0.873 1092 | 0.920 3223 | 0.928 2119 | 0.826 1465 | 0.669 2107 | 0.575 495 | 0.546 9499 | 0.475 495 | 0.406 9499 | 0.800 6691 | 0.785 0088 |
| 1 : 2 | 0.327 2268 | 0.267 4972 | 0.397 0604 | 0.330 1623 | 0.397 0604 | 0.330 1623 | 0.347 9763 | 0.248 1058 | 0.315 3948 | 0.258 3361 | 0.253 948 | 0.208 3361 | 0.360 8784 | 0.195 8023 |

**Table 13 Effects of killing target cells after co-culture of target cells with STAR with OX40 connected to β chain thereof via different linkers**

| | β-del-OX40 | | β-OX40 | | β-del-G4SOX40 | | βdel-[G4S]3-OX40 | | β-del-[G4S]7-OX40 | | β-del-[G4S]10-OX40 | | αβ-OX40 | |
|---|---|---|---|---|---|---|---|---|---|---|---|---|---|---|
| 2: 1 | 0.95 73 | 0.95 54 | 0.90 55 | 0.89 | 0.53 95 | 0.53 81 | 0.961 2 | 0.960 9 | 0.502 3 | 0.510 1 | 0.4023 | 0.4101 | 0.99 57 | 0.99 65 |
| 1: 1 | 0.95 42 | 0.95 55 | 0.90 68 | 0.91 04 | 0.38 79 | 0.40 74 | 0.947 6 | 0.950 1 | 0.288 | 0.326 7 | 0.2881 | 0.3267 | 0.80 06 | 0.78 5 |
| 1: 2 | 0.60 22 | 0.58 3 | 0.23 6 | 0.28 23 | 0.24 66 | 0.28 63 | 0.391 3 | 0.333 3 | 0.189 4 | 0.165 7 | 0.1094 | 0.1057 | 0.36 08 | 0.19 58 |

**Table 14 IL-2 secretion after co-culture of target cells with STAR with OX40 connected to α chain thereof via different linkers**

| | **2:1** | | **1:1** | |
|---|---|---|---|---|
| **Dual-START** | **10055.68** | **9922.099** | **6790.268** | **6760.583** |
| **TCRβ-del-OX40** | **9387.758** | **9066.164** | **6201.504** | **5573.159** |
| **TCRα-del-OX40** | **8200.334** | **7740.208** | **6161.923** | **5746.325** |
| **TCRα-del-G4S-OX40** | **8759.413** | **8284.444** | **6899.116** | **6112.447** |
| **TCRα-del-(G4S)3-OX40** | **9308.597** | **8413.081** | **7191.024** | **6424.146** |
| **TCRα-del-(G4S)7-OX40** | **5261.46** | **5528.63** | **3267.577** | **4064.141** |

**Table 15 IFN-α secretion after co-culture of target cells with STAR with OX40 connected to α chain thereof via different linkers**

| | **2:1** | | **1:1** | |
|---|---|---|---|---|
| **Dual-START** | **4667.302** | **4694.86** | **3292.826** | **3254.934** |
| **TCRβ-del-OX40** | **4612.185** | **4529.51** | **2373.065** | **2452.295** |
| **TCRα-del-OX40** | **3020.687** | **2820.889** | **1846.01** | **1694.439** |
| **TCRα-del-G4S-OX40** | **3372.057** | **3248.044** | **2218.049** | **2276.61** |
| **TCRα-del-(G4S)3-OX40** | **2565.974** | **2383.399** | **1580.761** | **1656.546** |
| **TCRα-del-(G4S)7-OX40** | **581.7688** | **443.9768** | **261.4024** | **226.9544** |

**Table 16 Effects of STAR with OX40 connected to α chain thereof via different linkers on the differentiation of central memory T cells**

| **Dual-START** | **TCRβ-del-OX40** | **TCRa-del-OX40** | **TCRα-del-G4S-OX40** | **TCRα-del-(G4S)3-OX40** | **TCRα-del-(G4S)7-OX40** |
|---|---|---|---|---|---|
| **2.92** | **7.1** | **6.9** | **3.71** | **7.75** | **0.59** |

**Table 17 Effects of STAR with OX40 connected to α chain thereof via different linkers on the differentiation of T cells**

| | **naive** | **effector** |
|---|---|---|
| **Dual-START** | **28.3** | **60.7** |
| **TCRβ-del-OX40** | **47.3** | **36.9** |
| **TCRα-del-OX40** | **40.1** | **43.5** |
| **TCRα-del-G4SOX40)** | **53.8** | **39.4** |
| **TCRα-del-(G4S)3-OX40** | **4.87** | **35.1** |
| **TCRα-del-(G4S)7-OX40** | **49** | **48.6** |

### 11. Effects of TCR endocytosis-related lysine modification into arginine in the transmembrane domain or endodomain on the function of mutant STAR-T cells

### 11.1. In vitro co-culture method for T cells and target cells

Target cells Raji-luciferase and primary T cells were suspension cells, for co-incubation, the corresponding number of cells were taken and mixed with the target cell culture medium, then centrifuged for culture. The specific steps were as follows: the primary T cells were infected with the packaged and purified WT-STAR and mut-STAR T viruses, and one day before co-culture, the infection efficiency was detected by flow cytometry, and the ratio of effector cell to target cell was determined, co-incubation was carried out usually at the ratio of 1:1 or 2:1, in addition, target cells Raji-luciferase and primary T cells were co-incubated for 7 days to observe the changes in the number of cell proliferation. The total number of T cells was calculated based on the infection efficiency, and the general usage of target cells was 1 × 10⁵/well (96-well plate).

### 11.2. Stimulation of T cells by target antigen

The target of the present invention was generally a cell surface protein, which can be directly used for activating T cells to detect the function of T cells, in particular, the target antigen was usually added with 1 × 10⁵/well positive T cells, centrifuged and activated for 24 hours to collect the cell suspension or culture supernatant for detecting T cell function.

### 11.3. T cell killing function validation: Luciferase assay

T cells were co-cultured with target cells for different times, then the cell suspension was gently blown evenly, 150 µ L of cell suspension per well was taken to a white 96-well plate, centrifuged at 1500 rpm/min for 5 min, and the supernatant was taken and added with a cell lysate for lysing at room temperature for 15 min, then centrifuged at 4 °C at 4000 rpm/min for 15min, then the supernatant was taken with 2 parallel wells being taken for each well, and added with a luciferase substrate (firefly luciferase assay reagent), then detected by a multifunctional microplate reader with the gain value fixing as 100 to obtain a chemiluminescence value. Cell killing calculation: killing efficiency = 100%- (effector cell-target cell value per well/control cell-target cell value per well).

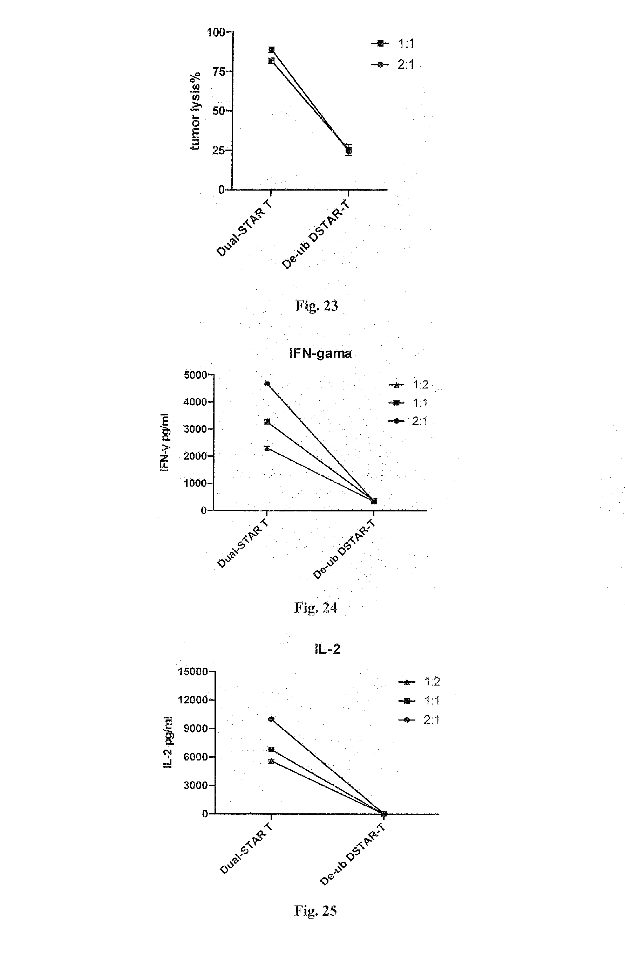

The detection results of T cell killing function test by the luciferase assay showed that, the tumor cell-killing effect of ub-STAR, a mut-STAR with TCR endocytosis-related lysine modified into arginine in the transmembrane domain or endodomain, at the 2:1 or 1:1 E:T ratio of T cell to target cell, was significantly lower than that of mut-STAR T cells, as shown in Figure 23 and Table 18.

### 11.4. Analysis of cytokine secretion by T cells: ELISA

During T cell activation, a large number of cytokines, such as TNF-α, lFN-γ and IL-2, were released to help T cells kill target cells or promote the expansion of T cells themselves. After T cells were stimulated by target cells or antigens, T cells were collected, centrifuged, and the supernatant was taken. The lFN-γ, and IL-2 ELISA kits used were Human IL-2 Uncoated ELISA, Human lFN-γ Uncoated ELISA (Art.No. 88-7025, 88-7346, 88-7316, respectively). The specific steps were as follows: diluting 10X Coating Buffer with ddH2O to 1X, adding coated antibody (250X), mixing well and adding to a 96-well plate at 100 µL/well, After sealing with the plastic wrap and staying overnight at 4 °C, 1X PBST (also called Wash Buffer, 1X PBS with 0.05% Tween 20 added) was used for washing 3 times with 260 µL/well each time, 5X ELISA/ELISPOT Diluent was diluted to 1X with ddH₂O and then added to the 96-well plate with 200 µL/well, followed by standing at room temperature for 1 hour. PBST was used for washing once, and dilution was performed according to a standard curve (ranging from: 2 to 250, 4 to 500, 4 to 500, respectively), the samples were diluted to 20 to 50 times with 1xDiluent. Samples diluted according to the standard curve were added with 100 microliters per well, two parallel wells were taken and incubated at room temperature for 2 hours, PBST was used for washing three times, then a Detection antibody diluted with 1xDiluent was added, after incubation for 1 h, PBST was used for washing three times, then HRP diluted with 1xDiluent was added, after incubation for 30 minutes, the solution was washed six times, TMB was added for color development with the color development time being less than 15min, and 2N H₂SO₄ was added for termination, light absorption at 450 nm was detected.

The ELISA results showed that, the IL-2 and lFN-γ secretions by ub-STAR, a mut-STAR with TCR endocytosis-related lysine modified into arginine in the transmembrane domain or endodomain, which was co-incubated with target cells at the 2:1 or 1:1 or 1:2 E:T ratio of T cell to target cell for 24 hours, were significantly lower than those of mut-STAR T cells, as shown in Figures 24, 25, and Tables 19, 20.

### 11.5. Detection of T cell differentiation changes: analysis by flow cytometry

During T cell activation, a large number of cytokines and other chemokines were released, and signals were transduced into the nucleus through cytokines or chemokine receptors to regulate the differentiation of T cells, the proliferation and persistence of T cells in vivo were affected by the number of T cells differentiated to memory T cells. Memory T cells can be classified into stem cell T cells, central memory T cells and effector memory T cells. The expression of CD45RA and CCR7 on the surface of T cells was detected by flow cytometry, thus the differentiation of T cells can be known. T cells were incubated with target cells for 7 days, centrifuged, stained with anti-human-CD45RA-Percp-cy5.5 and anti-human-CCR7-APC flow antibodies for 30 minutes, centrifuged again, washed with PBS and fixed with 4% paraformaldehyde solution, and the differentiation of T cells was detected by flow cytometry.

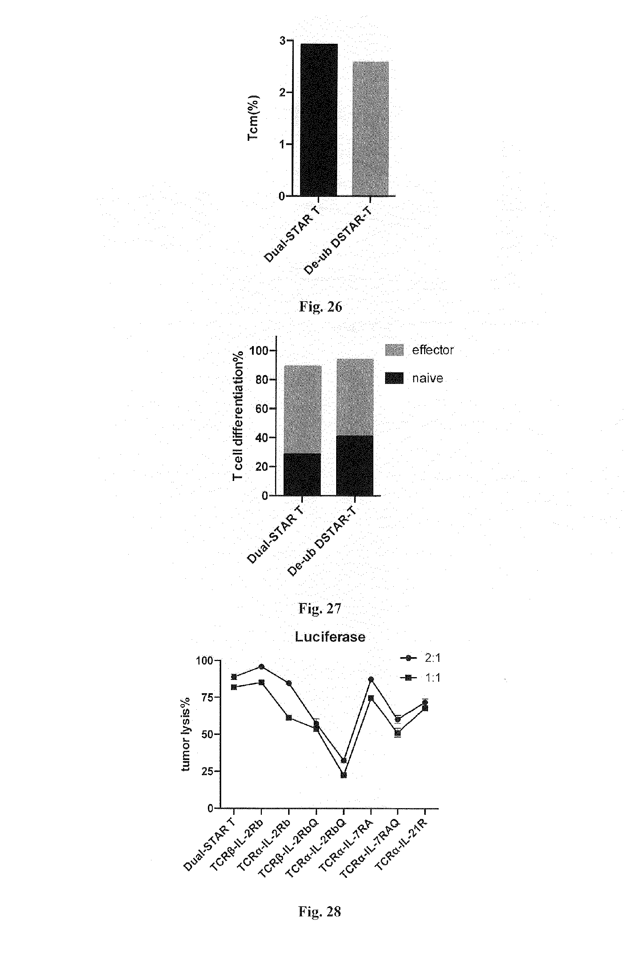

The results of flow cytometry showed that, no significant difference was found in neither the differentiation of central memory T cells nor the ratio of naive T cell to effector T cell between mut-STAR and ub-STAR, a mut-STAR with TCR endocytosis-related lysine modified into arginine in the transmembrane domain or endodomain, indicating that TCR endocytosis-related lysine modification into arginine in the transmembrane domain or endodomain had no significant effect on the differentiation of mut-STAR T cells, as shown in Figs. 26 and 27 and Tables 21 and 22. Combining tumor killing results, ELISA results and flow cytometry test of cell differentiation, ub-STAR, a mut-STAR with TCR endocytosis-related lysine modified into arginine in the transmembrane domain or endodomain, had no effective promotion effect on mut-STAR modification.

**Table 18 Killing effect after co-culture of target cells with STAR with transmembrane domain modification**

| | **2: 1** | | **1: 1** | |
|---|---|---|---|---|
| **Dual-STAR T** | **87.72091** | **90.20905** | **83.15808 :** | **80.77817** |
| **De-ub DSTAR-T** | **24.73308** | **24,27383** | **22,87242** | **27.67608** |

**Table 19 IL-2 secretion after co-culture of target cells with STAR with transmembrane domain modification**

| | **2: 1** | | **1: 1** | | **1: 2** | |
|---|---|---|---|---|---|---|
| **Dual-STAR T** | **10055.68** | **9922.099** | **6790.268** | **6760.583** | **5508.84** | **5672.111** |
| **De-ub DSTAR-T** | **46.6896** | **31.8468** | **-17.6292** | **-32.472** | **-2.7864** | **10.1132** |

**Table 20 secretion after co-culture of target cells with STAR with transmembrane domain modification**

| | **2: 1** | | **1:1** | | **1: 2** | |
|---|---|---|---|---|---|---|
| **Dual-STAR T** | **4667,302** | **4694,86** | **3292.826** | **3254.934** | **2273.166** | **2342.062** |
| **De-ub DSTAR-T** | **288.9608** | **409.5288** | **375.0808** | **354,412** | **340.6328** | **340.6328** |

**Table 21 Effects of transmembrane domain modification on the differentiation of central memory STAR-T cells**

| **Dual-STAR T** | **De-ub DSTAR-T** |
|---|---|
| **2.92** | **2.58** |

**Table 22 Effects of transmembrane domain modification on the differentiation of STAR-T cells**

| | **naive** | **effector** |
|---|---|---|
| **Dual-STAR T** | **28.3** | **60.7** |
| **De-ub DSTAR-T** | **40.8** | **52.7** |

### 12. Effects of mutant STAR with different cytokine receptor stimulatory regions connected to the α or β constant region endodomain on the function of STAR-T cells

### 12.1. In vitro co-culture method for T cells and target cells

Target cells Raji-luciferase and primary T cells were suspension cells, for co-incubation, the corresponding number of cells were taken and mixed with the target cell culture medium, then centrifuged for culture. The specific steps were as follows: the primary T cells were infected with the packaged and purified WT-STAR and mut-STAR T viruses, and one day before co-culture, the infection efficiency was detected by flow cytometry, and the ratio of effector cell to target cell was determined, co-incubation was carried out usually at the ratio of 1:1 or 2:1, in addition, target cells Raji-luciferase and primary T cells were co-incubated for 7 days to observe the changes in the number of cell proliferation. The total number of T cells was calculated based on the infection efficiency, and the general usage of target cells was 1 × 10⁵/well (96-well plate).

### 12.2. Stimulation of T cells by target antigen

The target of the present invention was generally a cell surface protein, which can be directly used for activating T cells to detect the function of T cells, in particular, the target antigen was usually added with 1 × 10⁵/well positive T cells, centrifuged and activated for 24 hours to collect the cell suspension or culture supernatant for detecting T cell function.

### 12.3. T cell killing function validation: Luciferase assay

T cells were co-cultured with target cells for different times, then the cell suspension was gently blown evenly, 150 µ L of cell suspension per well was taken and added to a white 96-well plate, centrifuged at 1500 rpm/min for 5 min, and the supernatant was taken and added with a cell lysate for lysing at room temperature for 15 min, then centrifuged at 4 °C at 4000 rpm/min for 15min, then the supernatant was taken with 2 parallel wells being taken for each well, and added with a luciferase substrate (firefly luciferase assay reagent), then detected by a multifunctional microplate reader with the gain value fixing as 100 to obtain a chemiluminescence value. Cell killing calculation: killing efficiency = 100%- (effector cell-target cell value per well/control cell-target cell value per well).

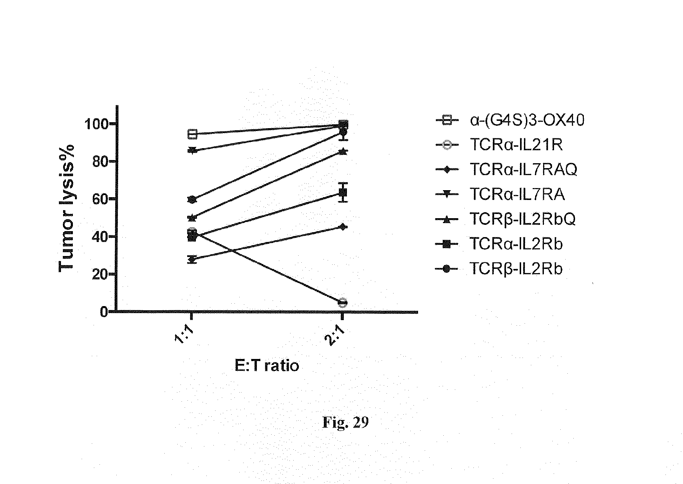

The detection results of T cell killing function test by the luciferase assay showed that, when different cytokine receptor stimulatory regions were connected to the α or β region endodomain of a mutant STAR, at the 2:1 E:T ratio of T cell to target cell, β-IL-2Rb, α-IL-2Rb, α-IL-7RA, α-IL21R all exhibited a killing effect similar to that of mut-STAR, while β-IL2RbQ, α-IL2RbQ and α-IL7RAQ exhibited a tumor-killing effect significantly lower than that of mut-STAR, as shown in Figure 28 and Table 23. In addition, the killing effect of mutant STARs with different cytokine receptor stimulatory regions linked were detected and compared with that of α-del-(G4S) 3-OX40-STAR, as shown in Fig. 29 and Table 24, at the 2:1 and 1:1 E:T ratios, the killing effect of α-IL7RA was slightly lower than that of α-del-(G4S) 3-OX40-STAR, while the killing effect of STARs with other cytokine receptor structures linked was significantly lower than that of α-del-(G4S) 3-OX40-STAR.

### 12.4. Analysis of cytokine secretion by T cells: ELISA

During T cell activation, a large number of cytokines, such as TNF-α, lFN-γ and IL-2, were released to help T cells kill target cells or promote the expansion of T cells themselves. After T cells were stimulated by target cells or antigens, T cells were collected, centrifuged, and the supernatant was taken. The IFN-γ, and IL-2 ELISA kits used were Human IL-2 Uncoated ELISA, Human lFN-γ Uncoated ELISA (Art.No. 88-7025, 88-7346, 88-7316, respectively). The specific steps were as follows: diluting 10X Coating Buffer with ddH2O to 1X, adding coated antibody (250X), mixing well and adding to a 96-well plate at 100 µL/well. After sealing with the plastic wrap and staying overnight at 4 °C, 1X PBST (also called Wash Buffer, 1X PBS with 0.05% Tween 20 added) was used for washing 3 times with 260 µL/well each time, 5X ELISA/ELISPOT Diluent was diluted to 1X with ddH₂O and then added to the 96-well plate with 200 µL/well, followed by standing at room temperature for 1 hour. PBST was used for washing once, and dilution was performed according to a standard curve (ranging from: 2 to 250, 4 to 500, 4 to 500, respectively), the samples were diluted to 20 to 50 times with 1xDiluent. Samples diluted according to the standard curve were added with 100 microliters per well, two parallel wells were taken and incubated at room temperature for 2 hours, PBST was used for washing three times, then a Detection antibody diluted with 1xDiluent was added, after incubation for 1 h, PBST was used for washing three times, then HRP diluted with 1xDiluent was added, after incubation for 30 minutes, the solution was washed six times, TMB was added for color development with the color development time being less than 15min, and 2N H₂SO₄ was added for termination, light absorption at 450 nm was detected.

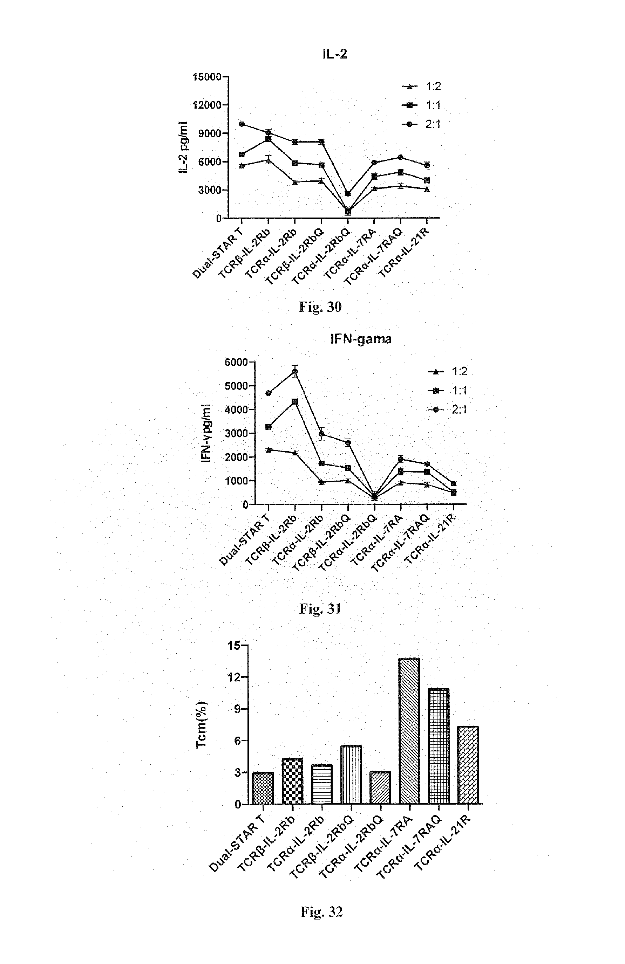

The ELISA results showed that when T cells were incubated with target cells for 24 hours, at a 1: 1 or 1: 2 E:T ratio, the IL-2 secretion of β-IL-2Rb was significantly higher than that of mut-STAR, while α-IL-2Rb, β-IL-2RbQ and α-IL-7RAQ exhibited similar IL-2 secretion, however, the IL-2 secretion of α-IL-2RbQ was significantly lower than that of mut-STAR, as shown in Figure 30 and Table 25. The lFN-γ secretion of β-IL-2Rb was significantly higher than that of mut-STAR, while the lFN-γ secretion of other structures was significantly lower than that of mut-STAR, as shown in Figure 31 and Table 26.

### 12.5. Detection of T cell differentiation changes: analysis by flow cytometry

During T cell activation, a large number of cytokines and other chemokines were released, and signals were transduced into the nucleus through cytokines or chemokine receptors to regulate the differentiation of T cells, the proliferation and persistence of T cells in vivo were affected by the number of T cells differentiated to memory T cells. Memory T cells can be classified into stem cell T cells, central memory T cells and effector memory T cells. The expression of CD45RA and CCR7 on the surface of T cells was detected by flow cytometry, thus the differentiation of T cells can be known. T cells were incubated with target cells for 7 days, centrifuged, stained with anti-human-CD45RA-Percp-cy5.5 and anti-human-CCR7-APC flow antibodies for 30 minutes, centrifuged again, washed with PBS and fixed with 4% paraformaldehyde solution, and the differentiation of T cells was detected by flow cytometry.

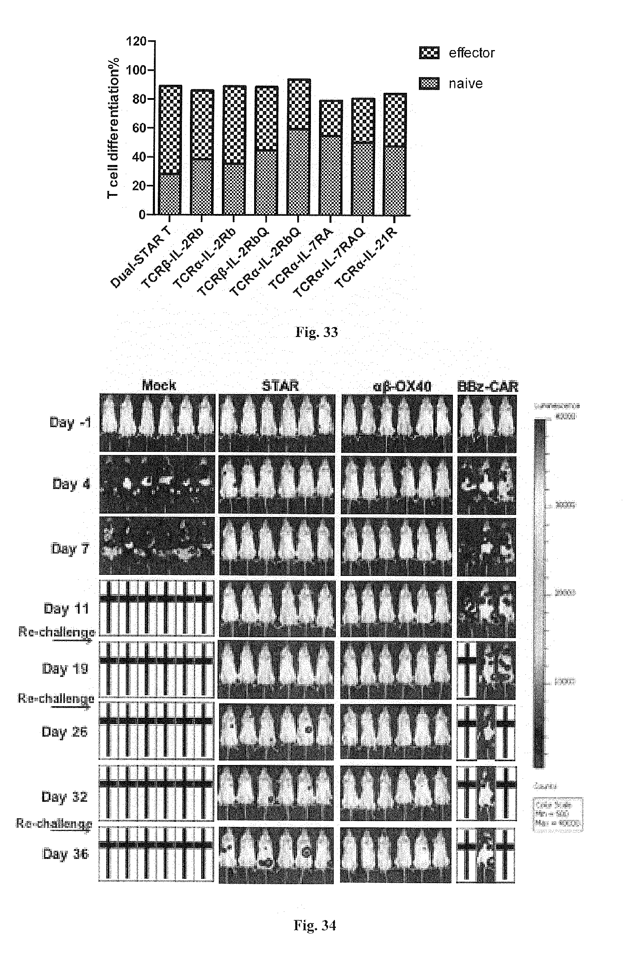

Mutant STARs with different cytokine receptor stimulatory regions linked to the α or β constant region endodomain showed various differences in the differentiation of central memory T cells and the ratio of naive T cell to effector T cell, wherein, α-IL-2Rb, β-IL-2Rb, α-IL-2RbQ, β-IL-2RbQ showed no significant difference as compared with mut-STAR, while α-IL-7RA, α-IL-7RAQ and α-IL-21R showed no significant difference as compared with mut-STAR, as shown in Figs. 32 and 33, and Tables 27 and 28. Based on the above results, the differentiation of mut STAR T cells was significantly influenced when different cytokine receptor stimulatory regions were connected to the α or β constant region endodomain of the mutant STAR.

**Table 23 Killing effect after co-culture of target cells with STAR with different cytokine receptor stimulatory domains connected to α chain thereof**

| | **2:1** | | **1:1** | |
|---|---|---|---|---|
| **Dual-STAR T** | **87.72091** | **90.20905** | **83.15808** | **80.77817** |
| **TCRβ-IL-2Rb** | **95.99994** | **95,97402** | **85,92993** | **84.68406** |
| **TCRα-lL-2Rb** | **85.12384** | **84.23454** | **62.18076** | **60.55779** |
| **TCRβ-IL-2RbQ** | **59.64616** | **55.11133** | **55.08403** | **52.52938** |
| **TCRα-IL-2RbQ** | **31.2389** | **33.48873** | **22.60999** | **22.34022** |
| **TCRα-IL-1RA** | **87.16662** | **87.63236** | **75.65523** | **73.73458** |
| **TCRα-IL-7RAQ** | **58.51033** | **62.29213** | **53.40511** | **49,22452** |
| **TCRα-IL-21R** | **70.18611** | **73.61224** | **67.44026** | **68.39847** |

**Table 24 Killing effect after co-culture of target cells with STAR with different cytokine receptor stimulatory domains connected to α chain thereof**

| | 1:1 | | 2:1 | |
|---|---|---|---|---|
| TCRβ-IL2Rb | 60.7618 | 58.6998 | 100 | 91.6018 |
| TCRa-IL2Rb | 39.7891 | 39.5275 | 68.4886 | 58.68 |
| TCRβ-IL2RbQ | 50.5957 | 50.0097 | 85.8178 | 85.7268 |
| TCRa-IL7RA | 86.0663 | 85.1918 | 99.1655 | 99.045 |
| TCRa-IL7RAQ | 29.6873 | 26.0614 | 45.2829 | 45.442 |
| TCRa-IL21R | 43.2581 | 41.9443 | 5.2928 | 4.6471 |
| a-(G4S)3-OX40 | 94.645 | 94.5233 | 99.6704 | 99.6625 |

**Table 25 IL-2 secretion after co-culture of target cells with STAR with different cytokine receptor stimulatory domains connected to α chain thereof**

| | 2: 1 | | 1: 1 | | 1: 2 | |
|---|---|---|---|---|---|---|
| Dual-START | 10055.68 | 9922.099 | 6790.268 | 6760.583 | 5508.84 | 5672-111 |
| TCRβ-IL-2Rb | 9333.335 | 8808.889 | 8418.029 | 8333.92 | 5884.858 | 6513.203 |
| TCRα-IL-2Rb | 8269.601 | 7893.583 | 5899,7 | 5840.329 | 3693.071 | 3989.927 |
| TCRβ-IL-2RbQ | 8304.234 | 7923.269 | 5652.32 | 5657.268 | 3782.128 | 4163.093 |
| TCRα-IL-2RbQ | 2748.079 | 2475.961 | 407.8644 | 1065.895 | 600.8208 | 838.3056 |
| TCRα-IL-7RA | 5855,172 | 5914,543 | 4182.883 | 4638.062 | 3039.988 | 3242.839 |
| TCRα-IL-7RAQ | 6438.989 | 6414,251 | 4633.115 | 5043.766 | 3223.049 | 3574.328 |
| TCRα-IL-21R | 5825.486 | 5296.093 | 3782.128 | 4187.831 | 2847.031 | 3287.368 |

**Table 26 secretion after co-culture of target cells with STAR with different cytokine receptor stimulatory domains connected to α chain thereof**

| | 2: 1 | | 1: 1 | | 1: :2 | |
|---|---|---|---|---|---|---|
| Dual-START | 4667.302 | 4694.86 | 3292.826 | 3254.934 | 2273,166 | 2342,062 |
| TCRβ-IL-2Rb | 5783.417 | 5435.492 | 4422.721 | 4257.37 | 2159.487 | 2204.27 |
| TCRα-IL-2Rb | 3158.479 | 2782.996 | 1732.332 | 1701.329 | 936.5832 | 967.5864 |
| TCRβ-IL-2RbQ | 2710.655 | 2472.964 | 1580.761 | 1480.862 | 946.9176 | 1057.151 |
| TCRα-IL-2RbQ | 237.2888 | 492.204 | 306.1848 | 357.8568 | 264.8472 | 251.068 |
| TCRα-IL-7RA | 2014.806 | 1797.783 | 1480.862 | 1281.063 | 867.6872 | 957.252 |
| TCRα-IL-7RAQ | 1742.666 | 1642.767 | 1322.401 | 1387.852 | 754.0088 | 902.1352 |
| TCRα-IL-21R | 936.5832 | 798.7912 | 533.5416 | 4$8,7592 | 406.084 | 564.5448 |

**Table 27 Effects of STAR with different cytokine receptor stimulatory domains connected to α chain thereof on the differentiation of central memory T cells**

| **Dual-STAR T** | **TCRβ-IL-2Rb** | **TCRα-IL-2Rb** | **TCRβ-IL-RbQ** | **TCRα-IL-2RbQ** | **TCRα-IL-7RA** | **TCRα-IL-7RAQ** | **TCRα-IL-21R** |
|---|---|---|---|---|---|---|---|
| **2.92** | **4.26** | **3.64** | **5.44** | **2.99** | **13.7** | **10.8** | **7.26** |

**Table 28 Effects of STAR with different cytokine receptor stimulatory domains connected to α chain thereof on the differentiation of T cells**

| | **naive** | **effector** |
|---|---|---|
| **Dual-START** | **28.3** | **60.7** |
| **TCRβ-IL-2Rb** | **38.4** | **47.4** |
| **TCRa-IL-2Rb** | **35.4** | **53.4** |
| **TCRβ-IL-2RbQ** | **44.8** | **43.8** |
| **TCRα-IL-2RbQ** | **59.3** | **34.3** |
| **TCRα-IL-7RA** | **54.7** | **24.5** |
| **TCRα-IL-7RAQ** | **50.2** | **30.4** |
| **TCRα-IL-21R** | **47.8** | **36.4** |

### 13. Evaluation of in vivo proliferation and anti-tumor ability of various STAR-like T cells

### 1) Experimental animal model

In this experiment, an NSG immunodeficient mouse was used as a model. The mouse genotype was NOD-Prkdcem26Il2rgem26/Nju with lack of T cells, B cells and NK cells, and macrophages and dendritic cells thereof were also deficient. The NSG mouse was the most completely immunodeficient mouse strain at present, which can be widely used in preclinical research of T cell therapy due to that it will not reject transplanted tumors and T cells. In this experiment, female NSG mice aged 6 ~ 8 weeks were used, and the weight difference of mice in each batch was controlled within 2 g. Mice were kept in independent ventilated cages within a specific pathogen free (SPF) barrier, and provided with normal diet and drinking water with pH slightly acidic to prevent pathogen contamination.

### 2) Construct of tumor model

In constructing a hematological tumor model, human Burkitt's lymphoma cell line Raji cells were used for xenotransplantation. Raji cells were cell strains expressing luciferase gene by the lentiviral vector, and the development and changes of Raji tumor were monitored in real time by fluorescein chemiluminescence and in vivo imaging in mice. In this model, different doses (generally about 1 to 3 ×10⁶ cells) of Raji-luciferase cells were mixed with matrix gel and inoculated into female NSG mice aged 6 to 8 weeks by intraperitoneal injection. Three days later, mice were intraperitoneally injected with the fluorescein potassium salt solution, and the fluorescence signals of tumor cells in vivo were detected by in vivo imaging. Raji cells grew rapidly in mice, which produced solid tumors in abdominal cavity, causing symptoms such as weight loss in mice; in the absence of therapeutic treatment, Raji tumor burdens led to death in mouse in about 40 days.

### 3) Animal experiment operation

All animal operations were carried out after the approval of the Animal protocol.

### 4) Means for monitoring tumor growth

In this experiment, in vivo fluorescence imaging was basically used by: injecting tumor cells with luciferase gene into animals for colonization. Mice were intraperitoneally injected with the fluorescein potassium salt solution, which, as a substrate, emitted light with a specific wavelength in the presence of enzyme, and the fluorescence signals of tumor cells in vivo were detected by in vivo imaging. Quantitative analysis of fluorescence signals was performed and a heat map was drawn to quantitatively reflect tumor growth.

### 5) Method for detecting T cell activity and amplification in animals

The survival and expansion of T cells in vivo are directly related to their final anti-tumor effect. In order to detect the activity and proliferation of T cells in animals, blood samples were collected from mice regularly, and the proportion, cell state and cell grouping of STAR-T cells in peripheral blood were analyzed. The specific operation was as follows: every 3-4 days, mice were anesthetized with isoflurane, and about 100 uL of blood was collected from the mouse orbit. The blood sample underwent anticoagulation, plasma collection and erythrocyte cleavage, then the remaining cells were subject to flow staining to detect the ratio of CD4 to CD8 and the molecules, such as CCR7, CD45RA, PD-1, LAG-3 and TIM-3, which were used for T cell subset analysis and cell state analysis. At the same time, the absolute number of STAR-T cells in peripheral blood of mice was obtained by flow cytometry or digital PCR. In addition, at the end of the experiment, mice can be dissected to detect the proportion of T cells in other immune organs of mice.

### 6) Method for evaluating T cell safety in animals

In order to evaluate the toxicity and safety of STAR-T cells, whether side effects have been caused by the STAR-T cells on experimental animals was examined. By observing the behavior state of mice, analyzing the pathology of mice, and analyzing the sections taken from important organs of mice, whether the reinfused T cells have significant toxicity could be evaluated. At the same time, by analyzing the infiltration of T cells in non-tumor tissues of mice, whether T cells have off-target killing effect on non-tumor tissues of mice can be determined. In addition, by detecting the level of cytokines, such as IL-2, IFN-γ, TNF α or IL-6 in the mouse blood, whether T cells may cause systematic cytokine storm can be determined.

### 7) method for evaluating T cell tumor infiltration ability

The ability of T cells to infiltrate tumors is the core ability thereof to challenge solid tumors. In order to detect the infiltration ability of T cells, tumor tissues can be separated firstly, followed by digestion and grinding to obtain single cells, which were subject to flow staining to detect the proportion of T cells in tumor tissues. At the same time, tumor cells, tumor stromal cells and immune cells in the tumor suspension can be further separated by density gradient centrifugation (such as Percoll gradient, Ficoll gradient, etc.), so as to obtain purified tumor-infiltrating T cells, and the characteristics thereof, such as chemokine receptor expression, T cell depletion and so on, can be analyzed in detail by sequencing and other methods.

### 8) Results

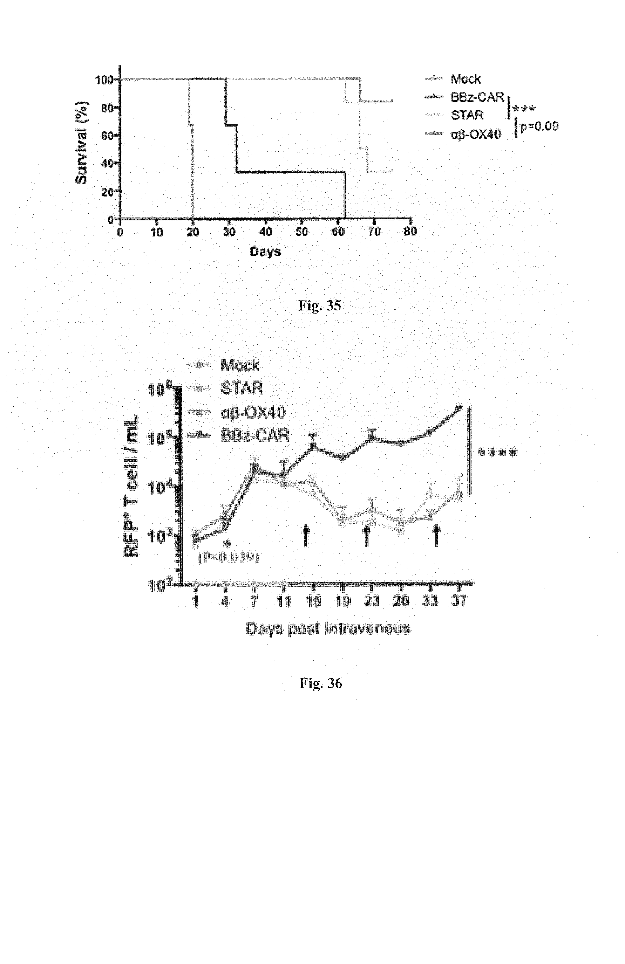

According to the results of the above in vitro function, 5 × 10⁵ Raji-luciferase tumor cells were inoculated into NCG female mice aged 6-8 weeks via tail vein, to construct a mouse tumor model (Figure 34), and the in vivo function of expressing αβ OX40-STAR or mut-STAR T cells was further evaluated. On day 6, the tumor-bearing mice were divided into four groups: A: PBS injection group (injected with equal volume of PBS); B: mut-STAR T cell injection group; C: αβ OX40-STAR T cell injection group; D: BBz-CAR-T cell injection group. Mice in B/C/D group were injected with 5 × 10⁵ TCR T cells by tail vein, and mice in Group A were injected with equal volume of 200 µL PBS. In the next few weeks, tumor cell growth, TCR T cell proliferation in vivo and mouse survival were monitored. As shown in Figures 34 and 35, compared with the control group, The αβ OX40-STAR T and mut-STAR T cells constructed in this example can significantly prolong the survival time of tumor-bearing mice, although after the tumor disappeared and tumor cells were reinfused into mice at different time points, αβ OX40-STAR T and mut-STAR T can properly eliminate tumor cells, and the effect of αβ OX40-STAR T is better than that of mut-STAR T, and the survival time of mice in αβ OX40-STAR T group was higher than that in the STAR-T group and CAR-T group. In addition, as shown in Fig. 36, compared with STAR-T group, the in vivo proliferation effect of αβ OX40-STAR T cells was better than that of STAR-T cells, and in the case of tumor reinfusion, a slight proliferation occurred in αβ OX40-STAR T cells, which, however, did not occur in STAR-T cells. Based on the results of the above animal experiments, it can be found that the in vitro and in vivo effect of the αβ OX40-STAR structure was better than that of mut-STAR.

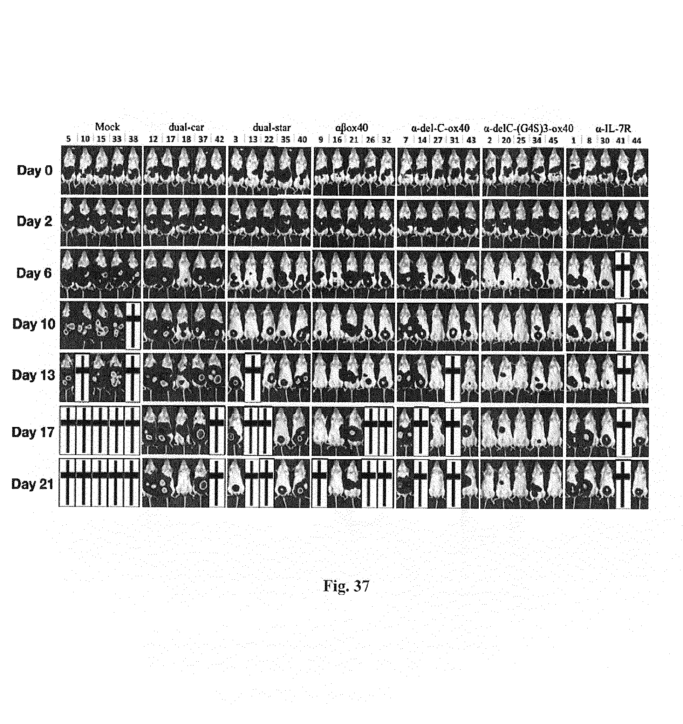

According to the above results of the in vitro function, 2×10⁶ Raji-luciferase tumor cells were inoculated intraperitoneally into NCG female mice aged 6-8 weeks, to construct a mouse tumor model (Figure 37), and the in vivo function of mut-STAR T cells expressing different effects was further evaluated. On day 8, the tumor-bearing mice were divided into seven groups: A: PBS injection group (injected with equal volume of PBS); B: dual-car; C: dual-STAR-T cell injection group; D: αβOX40-STAR T cell injection group; E: α-del- OX40-STAR-T cell injection group; F: α-del-(G4S)3-OX40-STAR-T cell injection group; and G: α-IL7R-STAR-T cell injection group. Mice in group B/C/D/E/F/G were injected with 2 × 10⁶ TCR T cells by tail vein, Mice in group A were injected with equal volume of 200 µ L PBS. In the next few weeks, tumor cell growth, STAR-T cell proliferation in vivo and mouse survival were monitored. As shown in Fig. 37, compared with the control group, the α-del-(G4S) 3-OX40-STAR-T cells constructed in this example can significantly kill tumor cells of tumor-bearing mice, and the effect thereof was superior to other groups. Based on the results of the animal experiments, it can be found that the in vitro and in vivo effect of the α-del-(G4S)3-OX40-STAR structure was better than that of mut-STAR.

### Example2 Improved TCR

### 1. Design of T cell receptor and mutant thereof with constant region mutation

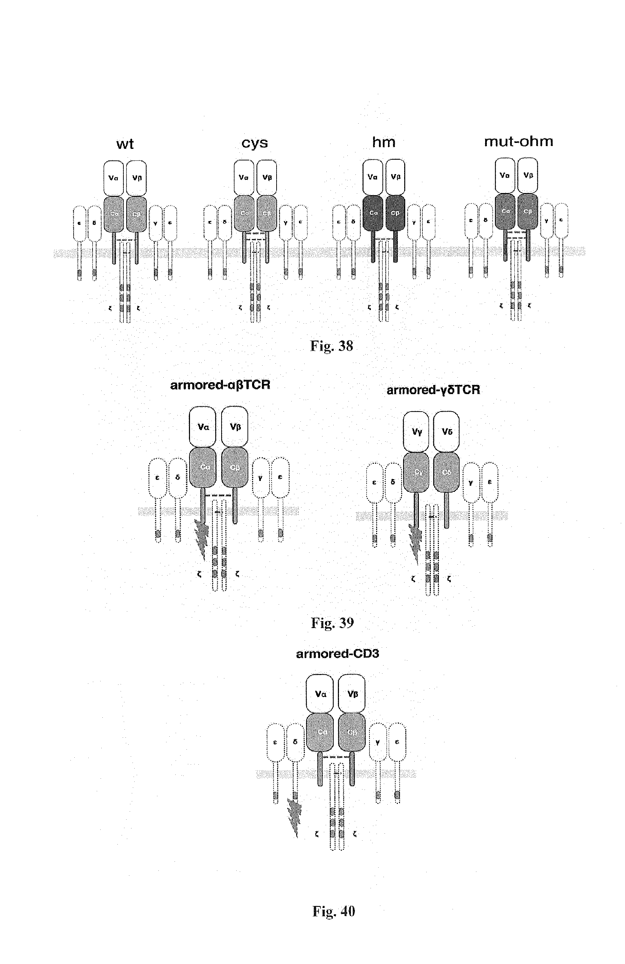

After being transferred into T cells, exogenous TCR molecules may mismatch with the endogenous TCR of T cells to form a mismatch, which, on one hand, may reduce the efficiency of correct pairing of TCR molecules and weaken the function of TCR T cells; on the other hand, may lead to potential off-target toxicity and increase the risk of treatment. In order to solve this problem, according to the present invention, the constant region of the wild-type αβ TCR sequence (wtTCR, left one in Fig. 38) was mutation-modified, resulting in the designs of cysTCR, hmTCR and mut-ohmTCR, respectively (Figure 38).

### 1.1. Design of cysTCR with intermolecular disulfide bond introduced in the constant region

Threonine (Thr) at position 48 in the TCR α chain constant region was mutated to cysteine (Cys), and serine (Ser) at position56 in the TCR β chain constant region was mutated to cysteine (Cys) by the inventors. The two new added cysteines would form a disulfide bond between the two chains of the exogenous TCR (cysTCR, left two in Fig. 38), thereby helping the TCR molecule form a more stable complex, thus obtaining better functions.

### 1.2. Design of murine hmTCR

The constant region sequences of TCR α chain and β chain are highly conserved in different species such as humans and mice. Studies have shown that the murine TCR constant region is less susceptible to form a mismatch with the humanized TCR constant region, and the murine constant region has a higher affinity with a human CD3 molecule, which can form a more stable complex in human T cells and greatly improve the function of TCR T cells. Therefore, the constant region sequence of humanized TCR was replaced by the constant region sequence of murine TCR to construct a TCR molecule with a murine constant region, i.e., hmTCR (right two in Fig. 38).

### 1.3. Design of murine mut-ohmTCR with transmembrane hydrophobic amino acid substitution and additional intermolecular disulfide bond

In order to further optimize the design of TCR molecule, the murine constant region designed for hmTCR was codon humanized to adapt to the expression of TCR molecules in human T cells. At the same time, Threonine (Thr) at position 48 in the murine TCR α chain constant region was mutated to cysteine (Cys), and serine (Ser) at position 56 in the TCR β chain constant region was mutated to cysteine (Cys), so as to form an additional intermolecular disulfide bond to help the TCR molecule form a more stable complex. In addition, the research showed that, the amino acid region from 111 to 119 in the TCR α chain transmembrane region was changed from LSVMGLRIL to LLVIVLRIL, that is, serine (Ser) at position 112 was mutated to leucine (Leu), methionine (Met) at position 114 was mutated to isoleucine (Ile), and glycine (Gly) at position 115 was mutated to valine (Val), which can increase the hydrophobicity of the transmembrane region, offset the instability caused by positive charges carried by the TCR transmembrane region, and make TCR molecules more stable on the cell membrane, thus obtaining better functions. Therefore, the mut-ohmTCR structure was designed by combining these three ideas (right one, in Fig.38) to improve the functions of TCR T cells.

### 2. Design of TCR-CD3 molecule comprising a costimulatory molecule receptor endodomain

In order to improve the proliferation ability in vivo, the effect survival time and the ability to infiltrate into the tumor microenvironment to kill the target cells efficiently, of TCR T cells, a new structure was designed by modifying the TCR-CD3 complex by the present inventors, thereby enabling tailored enhanced TCR-CD3 cell as needed, so as to improve the clinical response of TCR-T to achieve a lasting curative effect.

### 2.1. Design of TCR molecules (armored-TCR) comprising a costimulatory molecule receptor endodomain

TCR was a special marker of all T cell surfaces, which was divided into αβTCR and γδ TCR, which had the corresponding T cells: αβ T cells and γδ T cells. αβ TCR and γδ TCR were modified with costimulatory signals, respectively, by the inventors, to improve the performance of αβ T cells and γ δ T cells, respectively.

TCR of αβ T cells consisted of TCR α and TCR β chains, accounting for 90%-95% of the total T cells, αβ TCR consisted of a variable region and a constant region, in which the variable region had a wide diversity and played the role of antigen recognition and binding, while the constant region domain played the role of structural interaction and signal transduction. In order to enhance the toxicity and proliferation persistence of T cells, according to the present invention, an endodomain sequence of a humanized costimulatory molecule receptor was introduced to the C-terminal of the αβ TCR constant region (left, Fig. 39), to study the effects on the function of STAR T cells. The αβ TCR constant region of the present invention comprised an unmodified wtTCR constant region, a cysTCR constant region containing an additional intermolecular disulfide bond, a murine hmTCR constant region, and a mut-STAR with a combination of the three modifications described in subsection 1. The costimulatory signal transduction structure comprised an intracellular signal transduction domain of CD40/OX40/ICOS/CD28/4-1BB/CD27. The costimulatory endodomain could be connected to the C-terminal of TCR α chain or TCR β chain or both TCR α chain and β chain. In addition, the costimulatory molecular domain could be connected directly or via G4S/(G4S)n to the C terminal of TCR constant region, or to the C terminal of TCR constant region after deleting the endodomain sequence of the TCR molecule.

TCR of yδ T cells consisted of TCR γ and TCR δ chains, γδ T cells can be divided into three subgroups: γδ1, γδ2 and γδ3 based on the type of TCR δ chain, with different subgroups having different distribution in human bodies. γ δ T cells recognized a non-peptide antigen in an MHC-restricted way, which played an important role in the surveillance of pathogens and tumors. Experiments showed that, CD28/4-1BB and similar costimulatory signals played an important role in the activation and proliferation of γ δ T cells. The endodomain sequence of a human costimulatory molecule receptor was introduced to the C terminal of TCR γ and TCR δ, respectively (right, Fig. 39), by the inventors, to improve the performance of γ δ T cells.

### 2.2. Design of CD3 molecule (armored-CD3) comprising a costimulatory molecule receptor endodomain

A CD3 subunit included γ chain, δ chain, ε chain and ζ chain, and formed a T cell receptor complex with a TCR molecule, which transmitted signals from the ectodomain to endodomain, so as to regulate the state of cells and response to stimuli. In order to design enhanced TCR T cells and improve the tumor killing ability, proliferation ability and survival time of T cells in vivo, a CD3 molecule was modified by the inventors by introducing a human costimulatory molecule receptor endodomain to the C terminal of CD3 γ chain, δ chain, ε chain and ζ chain (Fig. 40), and the modified CD3 molecule was expressed in the TCR T cell to improve the function thereof.

### 3. Plasmid construction of TCR comprising the costimulatory molecule receptor endodomain and mutant thereof

### 3.1 Plasmid source

The vectors used in the present invention, including lentivirus vectors, retrovirus vectors, protein expression vectors, phagocytes, lentivirus packaging plasmid, retrovirus packaging plasmid, etc., were purchased from or synthesized by commercial companies, and the full-length sequences of these vectors were obtained, and the specific cleavage sites were known.

### 3.2. Fragment sources

The TCR mentioned in the present invention may be any functional TCR, including TCR-E 141, TCR-E 315, TCR-E 316 as used in the present invention. The gene fragments used in the present invention included the variable region of TCR-E141/TCR-E315/TCR-E316, wtTCR constant region, cysTCR constant region, hmTCR constant region, mut-ohmTCR constant region, costimulatory receptor endodomain, tag sequence and linker, etc., all of which were synthesized from commercial companies. One or more target fragments were connected by PCR with synthesizing primers to obtain corresponding functional sequences.

### 3.3 Plasmid construction

The lentiviral vector used herein was pHAGE-EF1 α-IRES-RFP, wherein the linear vector was obtained by a restriction enzyme Not I/Nhe I, the gene fragment was obtained by synthesis and PCR, and the complete vector was obtained by homologous recombination.

### 4. Construction of human primary TCR T cells and target cell lines

### 4.1 Lentivirus packaging

The aseptically extracted target gene plasmid and packaging plasmids psPAX2 and pMD2.G in a certain proportion were mixed with PEI (Polyethylenimine, PEI) to transfect Lenti-X 293T cells, the cell culture supernatant was collected at 48 hour and 72 hour, which was then filtered and mixed with PEG8000 (Polyethylene glycol, PEG), followed by standing overnight at 4 °C, centrifuged at 3500 rpm for 30 min, after discarding the supernatant, then resuspended with a small volume of medium to obtain concentrated lentivirus.

### 4.2 Isolation, culture, and lentivirus infection of human primary T cells

Peripheral blood mononuclear cells (PBMC) were isolated from peripheral blood of volunteers with Ficoll, a solution used for separation lymphocytes, then T cells were obtained from PBMC by negative selection according to the product instruction of EasySep Human T cell isolation kit (stem cell technologies), which were resuspended to 1 × 10⁶ cells/mL with 1640 complete medium containing IL-2 (100U/mL), and then activated in a culture dish coated with the anti-CD3/CD28 antibody. After 48 hours of activation, T cells were infected with viral particles loaded with TCR or/and CD3 using a lentiviral system by centrifugation at 1500 rpm for 2 hours at 32°C, and then taken out and incubated in a 37°C cell culture incubator for 10 hours, then the infection was terminated by addition of medium and the cells were continuously cultured in a 37°C cell culture incubator. TCR-positive cells were sorted by flow cytometry three days after infection.

### 4.3 Construction of target cell lines

Raji cells in the logarithmic growth phase were infected with viral particles loaded with LMP2-RFP, HLA-A^{∗}1101-BSD and Luciferase-GFP, respectively, using a lentiviral system. By drug screening and flow sorting, a Raji cell stably expressing LMP2, HLA-A^{∗}1101 molecules and Luciferase simultaneously was obtained, which was denominated as Raji-HLA-A^{∗}1101-LMP2-luciferase cell.

### 5. Effects of costimulatory signals on the function of TCR T cell with different constant regions

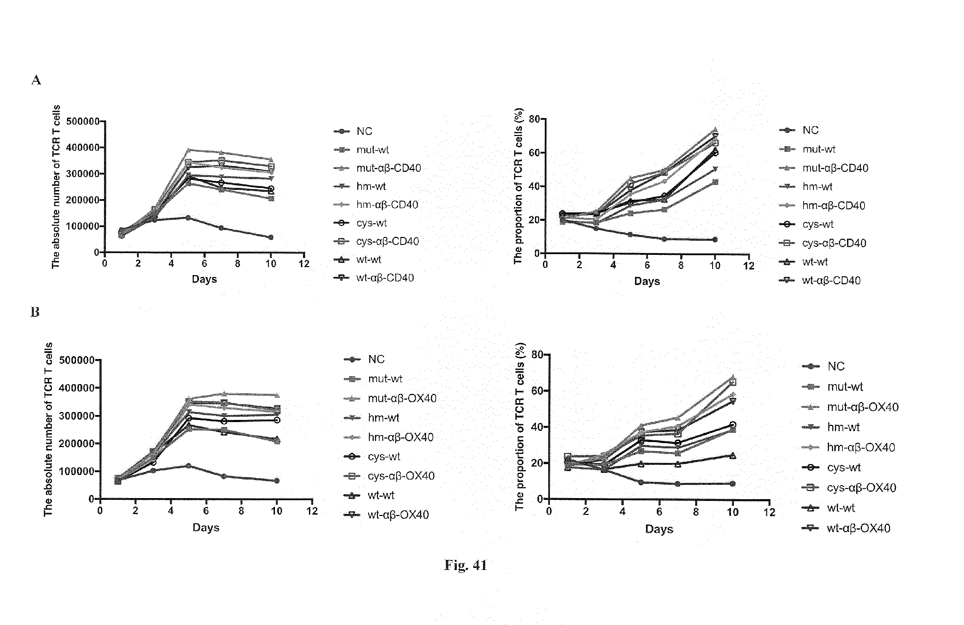

In order to investigate whether a costimulatory signal can improve the proliferation in vivo and enhance the tumor killing effect of TCR T cells, OX40, CD40 and ICOS endodomain were added to the C-terminals of TCR α chain and β chain (wtE141, cysE141, hmE141, mut-ohmE141) with four different constant domains, respectively), thus plasmids wtE141-αβ-OX40, cysE141-αβ-OX40, hmE141-αβ-OX40, mut-ohmE141-αβ-CD40, cysE141-αβ-CD40, hmE141-αβ-CD40, mut-ohmE141-αβ-CD40, wtE141-αβ-ICOS, cysE141-αβ-ICOS, hmE141-αβ-ICOS, mut-ohmE141-αβ-ICOS and mut-ohmE141-αβ-ICOS were constructed. In addition, plasmids were also constructed by adding OX40, CD40 or ICOS to the C-terminal of α and β chains of TCR-E315 and TCR-E316 with different constant regions. The virus was packaged with the second-generation packaging plasmid, which was then used for infecting the primary T cells and sorting for the positive cells. The sorted TCR T cells were co-cultured with Raji-HLA-A*1101-LMP2-luciferase cells according to the scheme in section 5, with counting as day 0, and then cells were collected on day 1, day 3, day 5, day 7 and day 10, respectively, for flow analysis. Among them, the medium used was 1640 complete medium without IL-2, and the initial number of TCR T cells was 1 × 10⁵ Cells, samples at each time point were incubated independently, and the remaining co-incubated samples were semi-changed with the liquid the next day, and supplemented with the target cells. The cells used for flow analysis were stained with an anti-human CD3 antibody in advance, and a specified volume thereof was collected and recorded when analysis was performed on the machine, the number and proportion of T cells in the system were obtained by conversion (Fig.41A). As seen from the proliferation curve of absolute number of E141 TCR T cells (left, Fig. 41A), TCR T cells with an OX40 receptor added at the C-terminal of TCR constant region showed better activation and proliferation after recognizing target cell epitopes. In addition, the proportion of T cells and residual target cells in the system (right, Fig. 41A) was analyzed, and it was found that TCR T cells with OX40 added showed stronger tumor clearance ability, especially for mut-ohmE141 TCR T cells, the costimulatory signal significantly improved the function of T cells. In addition, long-term co-culture in vitro for TCR-E141with CD40 or ICOS added also showed that chimeric costimulatory signals could significantly improve cell proliferation and enhance the tumor killing ability of T cells. When TCR was replaced by TCR-E315 (Fig.41B) and TCR-E316, similar results were obtained. According to the above experimental results, the fusion of endodomain of costimulatory domains such as OX40, CD40 and ICOS at the C-terminal of TCR can significantly enhance the proliferation ability and the ability to kill tumor cells of TCR T cells, among which, by adding the endodomain of costimulatory domain to the constant region of mut-ohm TCR, the improvement on the proliferation and killing ability of the corresponding TCR T cells was the most significant.

### 6. Effects of different costimulatory signals on TCR T cell function

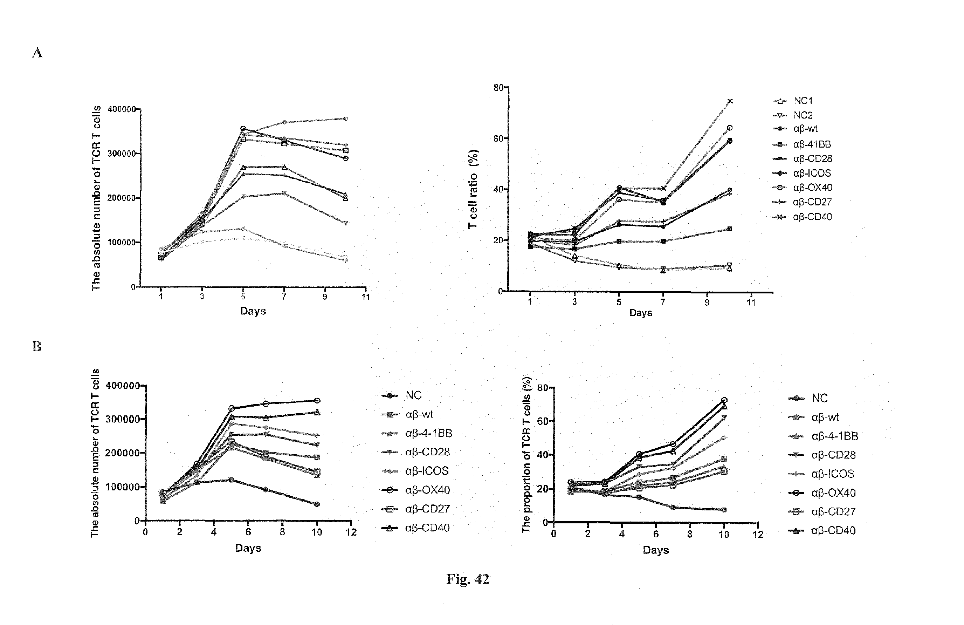

In order to compare the performance of TCR T cells modified by different costimulatory signals, the endodomains of 4-1BB, CD28, ICOS, OX40, OX40, CD27 and CD40 molecules were added to the C terminal of mut-ohmE141 TCR constant region, respectively, then the constructed mut-ohmE141-αβ-4-1BB, mut-ohmE141-αβ-CD28, mut-ohmE141-αβ-ICOS, mut-ohmE141-αβ-OX40, mut-ohmE141-αβ-CD27, mut-ohmE141-αβ-CD27, mut-ohmE141-αβ-CD40 TCR T cells were co-cultured with Raji-HLA-A*1101-LMP2-luciferase cells according to the co-culture scheme in section 5, and the number and proportion of T cells in the system were analyzed by flow cytometry. It can be seen from the results of proliferation curve and E:T ratio of absolute number of T cells (Fig. 42A) that, TCR T cells with CD40, OX40, ICOS or CD28 added at the C terminal of TCR constant region showed excellent activation and proliferation ability and significantly improved killing function, as compared with mut-ohmE141-αβ-wt without costimulatory signal modification. Among them, TCR T cells added with CD40 and OX40 showed more significant activation, proliferation and tumor clearance. In addition, similarly, TCR-E315 and TCR-E316 plasmids with the C-terminals of both α and β chains thereof connected to the costimulatory molecules described in this example were constructed, and the effects of different costimulatory molecules on the killing of target cells by either TCR T cell were analyzed. The results showed that results similar to TCR-E141 were also obtained from TCR-E315 (Figure 42B) and TCR-E316, indicating that the proliferation and killing ability of the corresponding TCR T cells could be significantly increased by selectively chimerizing costimulatory signals at the C-terminal of any of the functional TCR constant regions. Based on this, TCR T cells can be customized according to the requirements to improve the performance of TCR T cells by chimerizing costimulatory signals or other functional domains at the TCR C-terminal.

### 7. Effects of (G4S)n linker and endodomain sequence of TCR molecule on the function of armored-TCR T cells

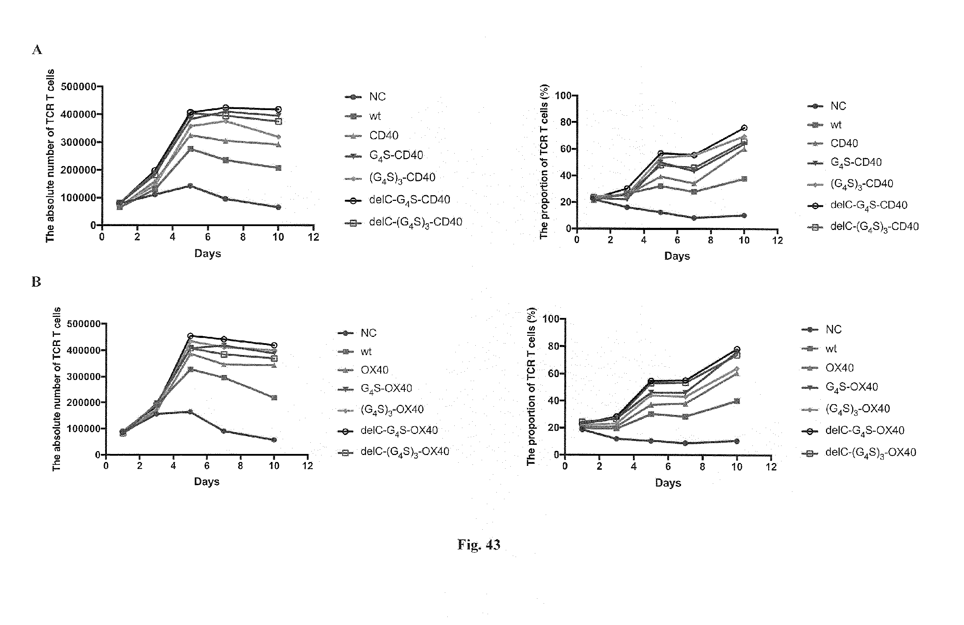

(G₄S)ₙ linker was widely used in protein engineering, which can endow fusion molecules with better flexibility to improve the function thereof. In order to further improve the function of TCR molecule integrating the costimulatory receptor endodomain, effects of (G4S)n linker on the function of the fusion protein armored-TCR molecules were studied. mut-ohmE141-αβ-G₄S-OX40 and mut-ohmE141-αβ-(GaS)₃-OX40 plasmids were constructed by introducing one or three G₄S linkers between the costimulatory receptor domain and the C-terminal of mut-ohmE141 TCR molecule, in addition, mut-ohmE141-αβ-delC-GaS-OX40 and mut-ohmE141-αβ-delC-(G₄S)₃-OX40 plasmids were also designed by deleting the endodomain at the C-terminal of the mut-ohmTCR αβ constant region and then connecting to the costimulatory receptor domain via one or three G₄S linkers. Likewise, the linker was introduced to mut-ohmE141-αβ-CD40, mut-ohmE141-αβ-ICOS, as well as TCR-E315 and TCR-E316 with OX40, CD40 and ICOS modifications, and the corresponding plasmids were constructed. After transfection of Lenti-X 293T with the second-generation packaging plasmid, the virus was obtained, which was used to infect primary T cells, and the positive cells were sorted. The sorted TCR T cells were co-cultured with Raji-HLA-A*1101-LMP2-luciferase cells according to the scheme in section 5, with counting as day 0, and then cells were collected on day 1, day 3, day 5, day 7 and day 10, respectively, for flow analysis. It can be seen from the results of proliferation curve and E:T ratio of absolute number of T cells (Fig. 43A) that, introduction of one or three G₄S between the C terminal of TCR constant region and OX40 endodomain can increase the proliferation and killing ability of TCR T cells, and deletion of the intracellular amino acids at the C terminal of TCR constant region can further improve the function of TCR T cells, as compared with mut-ohmE141-αβ-wt without a linker added. After OX40 was further replaced with CD40 or ICOS, the proliferation and killing ability of TCR T cells were also significantly enhanced after the modification described above, as compared with the control groups mut-ohmE141-αβ-CD40 and mut-ohmE141-αβ-ICOS. Meanwhile, similar results were also obtained from TCR-E315 (Fig. 43B) and TCR-E316.

### 8. Effects of connection of costimulatory structures to different TCR chains on the function of TCRT cells

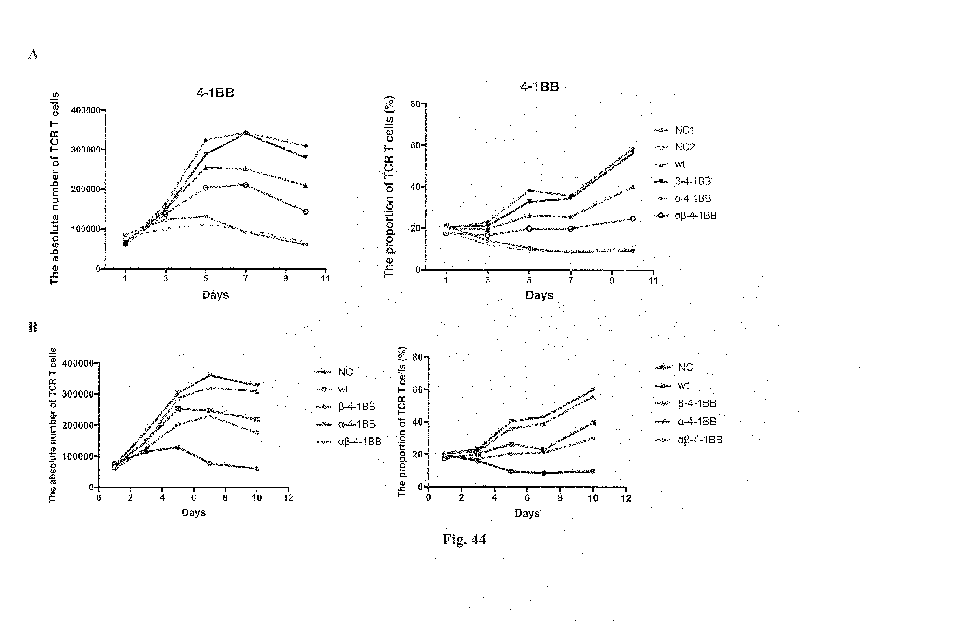

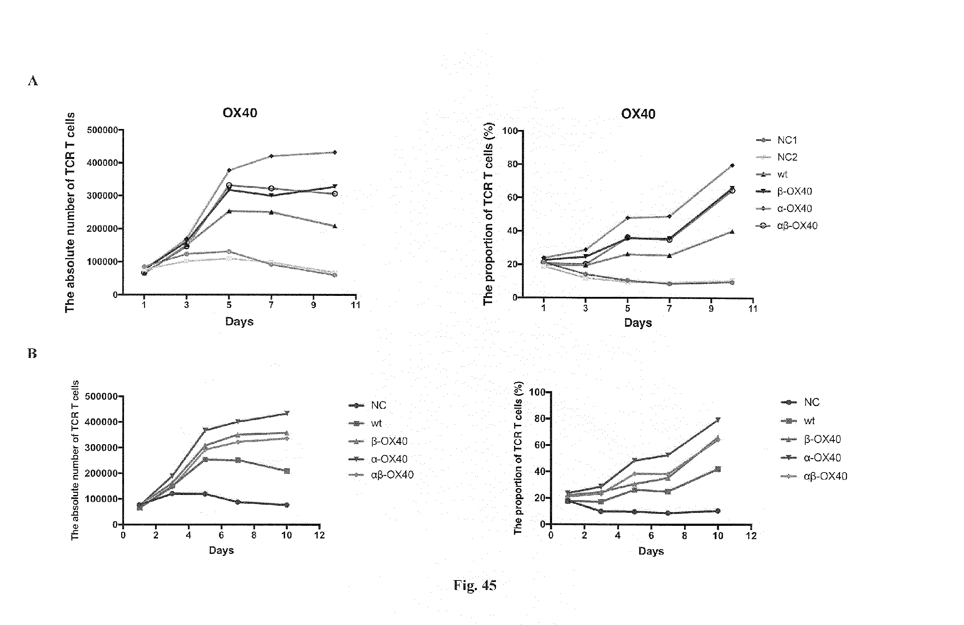

To investigate the effects of connection of costimulatory domains to different TCR chains on the T cell function, the endodomain of 4-IBB and OX40 were added to the C-terminal of TCR α chain, or TCR β chain or both TCR α and β chains to construct plasmids as follows: mut-ohmE141-α-4-1BB, mut-ohmE141-β-4-1BB, mut-ohmE141-αβ-4-1BB, mut-ohmE141-α-OX40, mut-ohmE141-β-ox40, and mut-ohmE141-αβ-ox 40. Likewise, plasmids with OX40 or 4-1BB endodomain added to different chains of TCR-E315 and TCR-E316 were also constructed. The virus was packaged with the second-generation packaging plasmid, which was then used for infecting the primary T cells. The TCR-positive T cells were co-cultured with Raji-HLA-A*1101-LMP2-luciferase cells according to the scheme in section 5, with counting as day 0, and then cells were collected on day 1, day 3, day 5, day 7 and day 10, respectively, for flow analysis. As seen from the results of proliferation curve and E:T ratio of absolute number of T cells (Fig. 44, Figure 45; A is E141, B is E315), as compared with mut-ohmE141-wt without a costimulatory signal added, TCR T cells with introduction of 4-1BB domain at the C-terminal of only one of TCR α or β chain constant region showed better activation and proliferation than that with such introduction at the C-terminal of both TCR α and β chains, and such introduction occurred only at TCR α chain showed a slight better effect than that occurred only at TCR β chain. By contrast, the design of introducing OX40 domain to the C-terminal of only TCR α chain constant region was significantly superior to the design of either introducing only in TCR β chain or introducing in both the two chains (Fig. 45). Meanwhile, similar results were also obtained from TCR-E315 and TCR-E316. The above experimental results showed that the introduction of costimulatory endodomain at the C terminal of TCR α chain facilitated best activation and proliferation of TCR T cells after their recognization of the antigenic epitopes of target cells, showing an improvement of robust killing function, as compared with chimerizing a costimulatory domain either only in TCR β chain or in both TCR α and β chains at the same time.

### 9. Effects of connection of costimulatory endodomain comprising G₄S linker to the C-terminal of TCR α chain on the function of TCR T cells

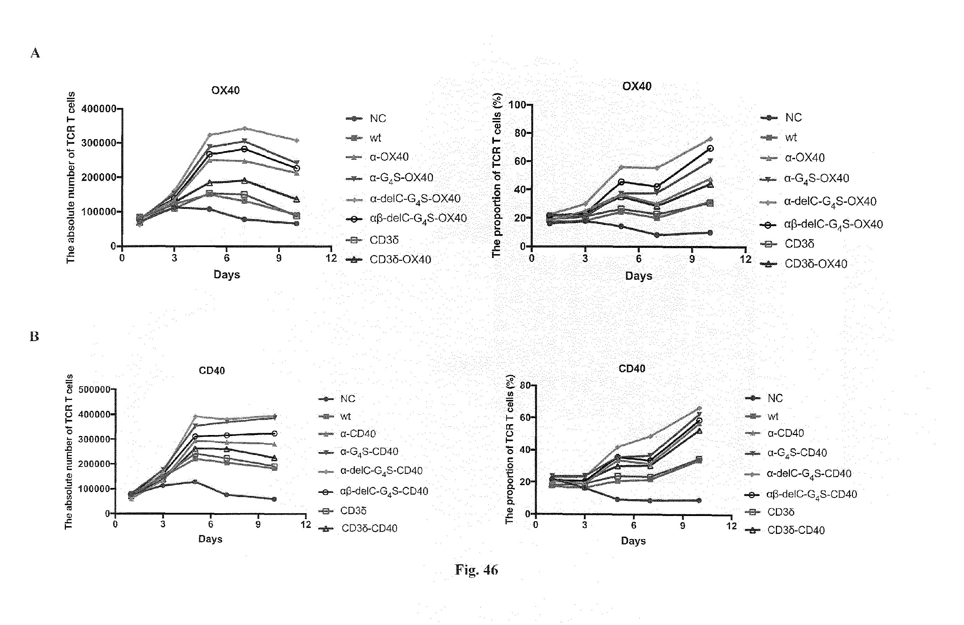

In order to further improve the function of armored-TCR T cells integrating the costimulatory receptor endodomain, combining the results of subsections 6, 7 and 8, the effects on the function of armored-TCR T cells by connecting the costimulatory receptor endodomain directly to the C-terminal of TCR α chain or to the C-terminal of TCR α chain after removing intracellular amino acids were studied. Plasmids: wtE141-α-OX40, wtE141-α-G₄S-OX40, wtE141-α-delC-G₄S-OX40, wtE141-αβ-delC-G₄S-OX40, hmE141-α-OX40, hmE141-α-G₄S-OX40, hmE141-α-delC-G₄S-OX40, hmE141-αβ-delC-G₄S-OX40, cysE141-α-OX40, cysE141-α-G₄S-OX40, cysE141-α-delC-G₄S-OX40, cysE141-αβ-delC-G₄S-OX40, mut-ohmE141-α-OX40, mut-ohmE141-α-G₄S-OX40, mut-ohmE141-α-delC-G₄S-OX40 and mut-ohmE141-αβ-delC-G₄S-OX40 were constructed. Plasmids: TCR-E141 with CD40 and ICOS added, and TCR-E315, TCR-E316 with OX40, CD40, and ICOS added, were also constructed. The virus was packaged with the second-generation packaging plasmid, which was then used for infecting the primary T cells. The TCR-positive T cells were co-cultured with Raji-HLA-A*1101-LMP2-luciferase cells according to the scheme in section 5, with counting as day 0, and then cells were collected on day 1, day 3, day 5, day 7 and day 10, respectively, for flow analysis. The results (Fig. 46) showed that, introduction of one G₄S between the C terminal of TCR α chain and a costimulatory molecule increased the proliferation and killing ability of TCR T cells, and deletion of the intracellular amino acids at the C terminal of TCR constant region could further improve the TCR T cell function, as compared with the control group without a linker added. As compared with the group with chimeric molecules added to both TCR α and β chains at the same time, the same modification on the α chain showed a significantly better effect than that on both chains at the same time. In addition, after OX40 was further replaced by CD40 or ICOS, compared with the control group, the proliferation and killing ability of TCR T linked to CD40 or ICOS via G4S after removing intracellular amino acids in the α chain were also significantly enhanced. Meanwhile, similar results were also obtained from TCR-E315 and TCR-E316.

### 10. Evaluation of in vivo proliferation and anti-tumor ability of armored-TCR T cells

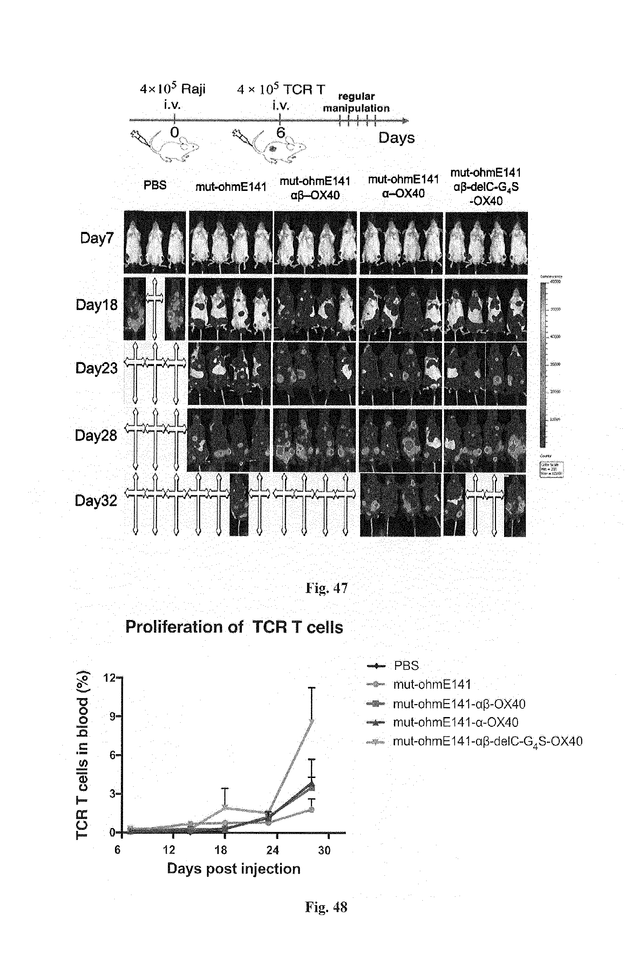

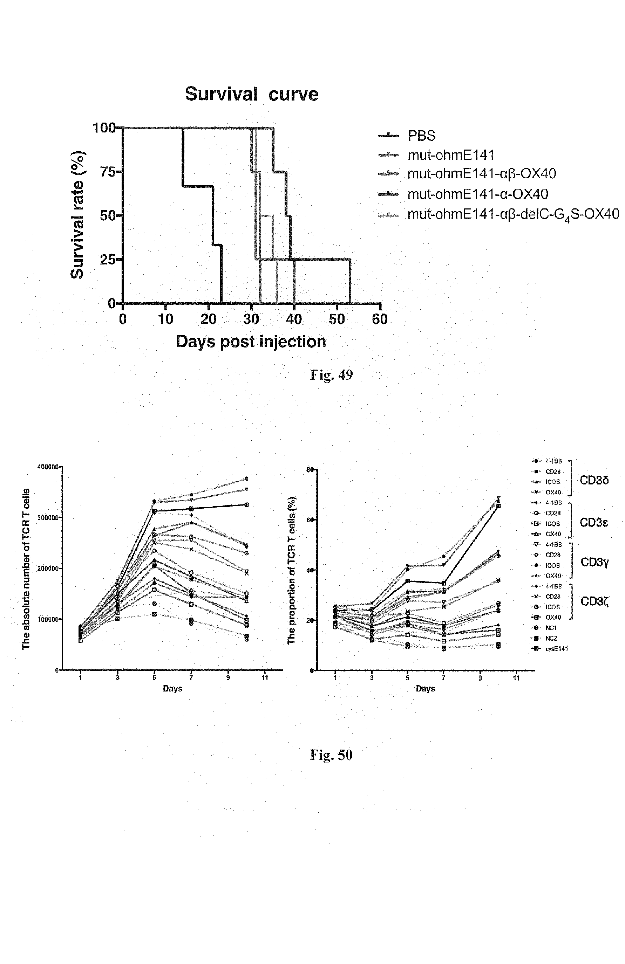

To validate the proliferation and anti-tumor effects of TCR T cells containing a costimulatory domain in vivo, 4 × 10⁵ Raji-HLA-A*1101-LMP2-luciferase tumor cells were inoculated by tail vein to NOD/Scid IL-2R γ null (NCG) female mice aged 5 to 6 weeks to construct a mouse tumor model (see Fig. 47). On the 6th day, the mice were divided into five groups as follows: A: PBS injection group (injected with equal volume of PBS); B: mut-ohmE141 TCR T cell injection group; C: mut-ohmE141-αβ-ox40 TCR T cell injection group, D: mut-ohmE141-α-OX40 TCR T cell injection group, and E: mut-ohmE141-αβ-delC-G₄S-OX40 TCR T cell injection group, wherein mice in group B/C/D/E were injected with 4 × 10⁵ TCR T cells by tail vein, while group A were injected with equal volume of 200 µL PBS. In the next few weeks, tumor cell growth in mice, human TCR T cell proliferation in mice and mouse survival were monitored. As shown in Fig. 48, TCR T cells containing a costimulatory molecule constructed in this example showed better proliferation in mice compared with the control group. In addition, as shown in Figs. 47 and 49, compared with the control group, themut-ohmE141-α-OX40 TCR T cells constructed in this example significantly prolonged the survival time of tumor-bearing mice, thus improving the survival rate of mice.

### 11. Evaluation of function of TCR T cells expressing armored-CD3 molecule

### (1) Evaluation of function in vitro

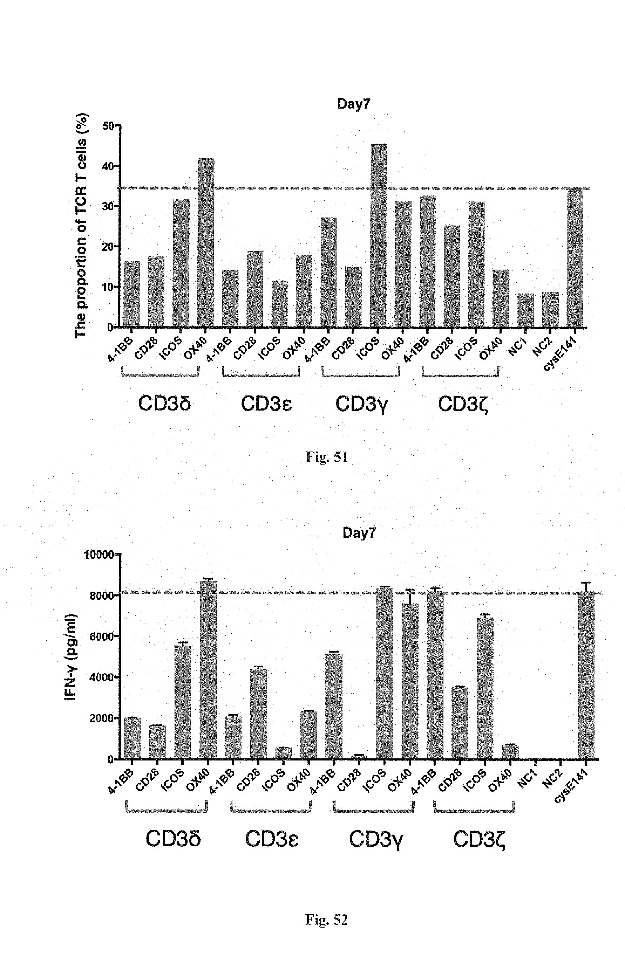

In order to compare the effects of introducing a human costimulatory receptor endodomain to the C-terminal of different CD3 subunits including δ chain, ε chain, y chain and ζ chain on the function of TCR T cells, according to the present invention, the CD3 molecule was modified to construct the following plasmids: CD3δ-4-1BB, CD3δ-CD28, CD3δ-ICOS, CD3δ-OX40, CD3ε-4-1BB, CD3ε-CD28, CD3ε-ICOS, CD3ε-OX40, CD3γ-4-1BB, CD3γ-CD28, CD3γ-ICOS, CD3γ-OX40, CD3ζ-4-1BB, CD3ζ-CD28, CD3ζ-ICOS, and CD3ζ-OX40, wherein CD3 was connected to the costimulatory receptor domains via a (G₄S)₃ linker. The modified armored-CD3 molecules and cysE141 TCR molecules were simultaneously expressed in T cells, then double positive cells obtained by flow sorting were co-cultured with Raji-HLA-A* 1101-LMP2-luciferase according to section 5, to evaluate the function of TCR T cells. As seen from the results of T cell proliferation curve in vivo and E:T ratio shown in Fig. 50 and the E:T ratio on Day 7 shown in Fig. 51, cysE141 TCR T cells expressing CD3δ-OX40 molecule or CD3γ-ICOS molecule were significantly superior to other armored-CD3 TCR T cells or cysE141 TCR T control group without armored-CD3 molecule in terms of proliferation and killing ability. At the same time, the cytokine IFNy level in the co-culture supernatant collected on the 7th day (Fig. 52) showed that, compared with cysE141 without armored-CD3 molecule, expression of CD3 δ-OX40 or CD3 γ-ICOS facilitated a better activation of cysE141 TCR T cells. Meanwhile, similar results were also obtained from TCR-E315 and TCR-E316.

### (2) Evaluation of function in vivo

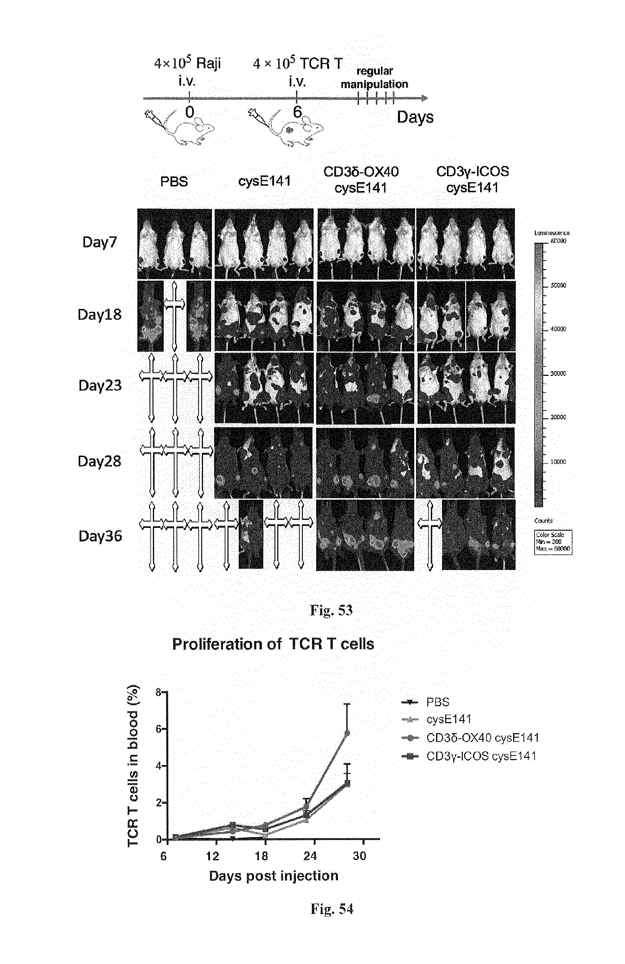

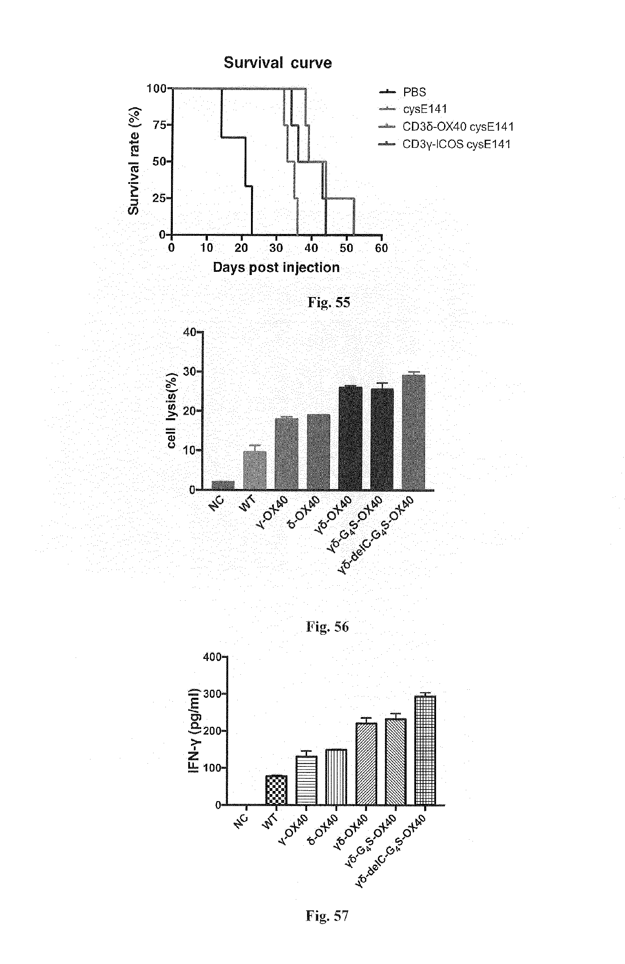

According to the results of the above in vitro function, 4×10⁵ Raji-HLA-A*1101-LMP2-luciferase tumor cells were inoculated into NCG female mice aged 5-6 weeks by tail vein, to construct a mouse tumor model (Fig. 53), and the function in vivo of cysE141 TCR T cells expressing CD3δ-OX40 or CD3γ-ICOS molecules was further evaluated. On day 6, the tumor-bearing mice were divided into four groups as follows: A: PBS injection group (injected with equal volume of PBS); B: cysE141 TCR T cell injection group; C: CD3δ-OX40 cysE141 TCR T cell injection group; and D: CD3γ-ICOS cysE141 TCR T cell injection group, wherein mice in group B/C/D/E were injected with 4 × 10⁵ TCR T cells by tail vein, while mice in group A were injected with equal volume of 200 µL PBS. In the next few weeks, tumor cell growth, TCR T cell proliferation in vivo and mouse survival were monitored. As shown in Figs. 53 and 55, compared with the control group, the CD3δ-OX40 cysE141 TCR T cells constructed in this example significantly prolonged the survival time of tumor-bearing mice. In addition, as shown in Fig. 54, compared with the control group, cysE141 TCR T cells expressing CD3δ-OX40 molecules showed better proliferation in mice.

### 12. Effects of costimulatory signals on TCR T cell function

In order to investigate whether costimulatory molecules affect the function of yδ TCR T cells, the endodomain sequence of a human costimulatory molecule was introduced to the C-terminal of TCR γ chain, TCR δ chain, both TCR γ chain and TCR δ chain constant regions, respectively. In order to further improve the function of γδ TCR T cells integrating the costimulatory receptor endodomain, the costimulatory receptor domain was connected directly via a G₄S linker to the C-terminal of TCR γ chain and δ chain with or without intracellular amino acids thereof removed, to construct plasmids as follows: TCR-G115-γ-OX40, TCR-G115-δ-OX40, TCR-G115-γδ-OX40, TCR-G115-γδ-G₄S-OX40, TCR-G115-γδ-delC-G₄S-OX40. The virus was packaged with the second-generation packaging plasmid, which was then used for infecting the primary T cells and sorting for the positive cells. The sorted γδ TCR T cells were used as effector cells, with human Daudi cells (Burkitt's lymphoma) as target cells. Co-culture was carried out for 24 hours at a 5: 1 E:T ratio, with 1640 complete medium without IL2 serving as the medium. Operation was performed according to the instructions of lactate dehydrogenase (LDH) release method (Promega). The cell killing rate was calculated as follows: the cell killing rate (%) = [(A ₑₓₚₑᵣᵢₘₑₙₜₐₗ cell -A effector cell spontaneous release well - A target cell spontaneous well)/ (A target cell spontaneous maxium release well -A target _{cell spontaneous well})] × 100%. In addition, the supernatant after 24 hours of co-culture was collected and operated according to the instructions of ELISA kit (Invitrogen), and the IFN-γ level in the supernatant was detected. The results (Figs. 56 and 57) showed that, compared with the control group, γδ T cells with the endodomain of costimulatory molecule added at the C-terminal of TCR had stronger tumor clearance ability, and the design by introducing OX40 to the C-terminal of both TCR γ and δ chains was significantly superior to the design by introducing OX40 to the C-terminal of only one of TCR γ chain and δ chain. Compared with the control group without such introduction, the design by introducing G₄S between the C-terminal of the TCR Constant region with the deletion of intracellular amino acids therein and a costimulatory molecule increased the killing ability of γδ TCR T cells.

### Example 3 Effects on STAR by modifying the N-terminal of constant region thereof

### 1. Design of STAR receptor constant region domain

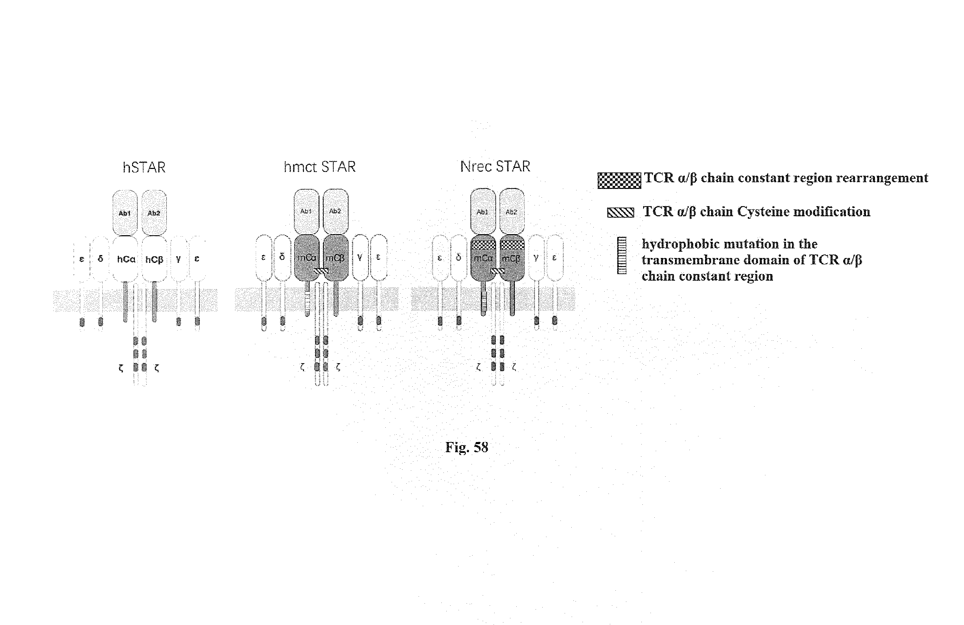

In this example, hSTAR referred to STAR comprising a human TCR constant region, hmct STAR referred to a STAR comprising a constant region with cysteine substitution and transmembrane domain modification as shown in Example 1, wherein the murine TCR α chain constant region was hmct STAR TCR α (SEQ ID NO: 41), and the murine TCR β chain constant region was hmct STAR TCRb (SEQ ID NO: 6), with the specific structures shown in Fig. 58.

In order to further optimize the design of STAR molecule, on the basis of constant region murinization, cysteine site mutation and hydrophobic amino acid mutation in the α chain constant region, the N-terminal of constant region of the STAR molecule was specifically rearranged to obtain better results. Rearrangement meant that some sequences were deleted, meanwhile humanized mutation of some sequences were performed. The significance of humanized mutation lied in reducing the non-human sequence in a STAR molecule as far as possible while ensuring the function of the STAR molecule, so as to avoid the possibility of STAR-T cells being rejected by receptors in clinical application to the greatest extent.

The schedule of 18 amino acid rearrangements (the murine sequence was DIQNPEPAVYQLKDPRSQ) at the N-terminal of TCR α chain constant region was analyzed based on the amino acid properties, which was found that E6D, K13R, R16K and Q18S in the murine and humanized sequences belonged to homologous amino acid substitution, while P15S substitution belonged to the non-polar amino acid to polar amino acid substitution, therefore, it could be considered that the proteins near this site were not conservative in nature, and could be modified without affecting its function. To sum up, the amino acid sequences at positions 1 to 14 were retained and humanized, and amino acids at positions 15 to 18 were deleted.

The schedule of 25 amino acid rearrangements (the murine sequence was DLRNVTPPKVSLFEPSKAEIANKQK) at the N-terminal of TCR β chain constant region was analyzed based on the amino acid properties, which was found that only R3K and L12V in the murine and humanized sequences were homologous amino acid substitutions, T6F, K9E, S11A, K17E, A21S, N22H and K23T were heterogeneous amino acid substitutions, therefore, it could be considered that the proteins near these sites were not conservative in nature, and could be modified without affecting its function. To sum up, the amino acid sequences at positions 1 to 16 were retained and humanized, and amino acids at positions 17 and 21 to 25 were deleted.

After N- terminal arrangement, the obtained TCR α chain constant region was Nrec STAR TCRa (SEQ ID NO: 42), and the obtained TCR β chain constant region was Nrec STAR TCRb (SEQ ID NO: 43), with the specific structures shown in Fig. 58.

### 2. Design of STAR costimulatory factor

### 1) Combination of different optimization methods of STAR

In order to validate the effects of costimulatory factors on different STAR functions, an original unoptimized STAR structure (human TCR α/β STAR, hSTAR), hmct STAR based on hSTAR with C-region murinization, cystine modification and transmembrane modification, and Nrec STAR based on hmct STAR with N-terminal modification were selected.

### 2) Design of STAR structure comprising costimulatory factors

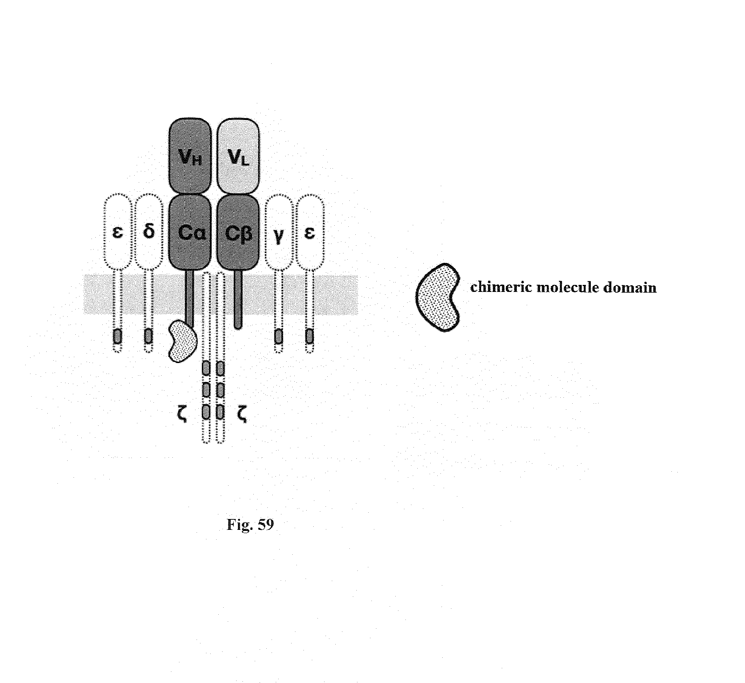

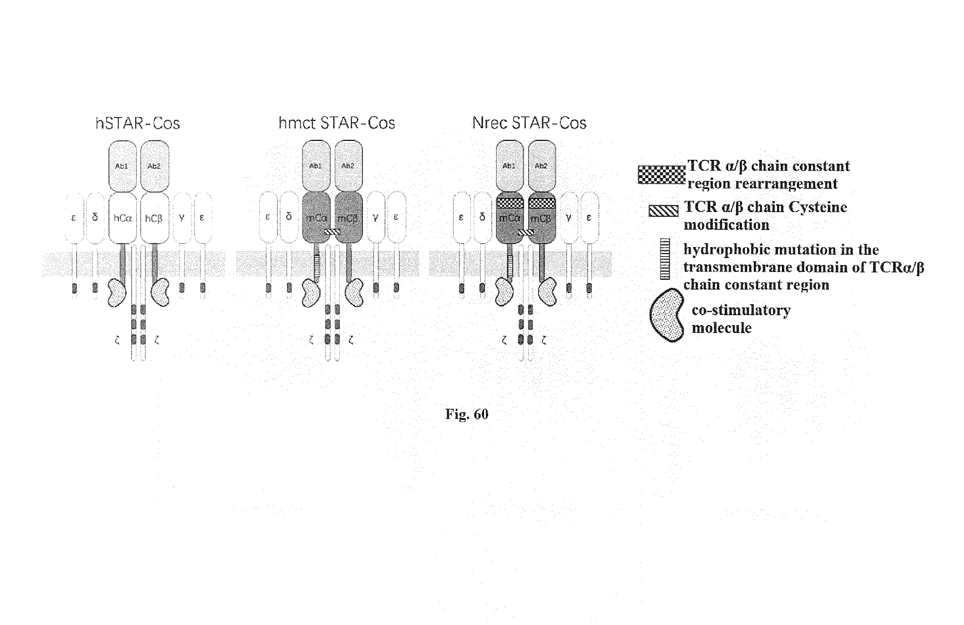

In order to enhance the toxicity of STAR-T cells and T cell proliferation persistence, according to the present invention, an endodomain sequence of a humanized costimulatory receptor was introduced to the C-terminal of the STAR constant region (Fig. 59), to study the effects on STAR - T cell functions. the above three structures: the original unoptimized STAR structure (human TCR α/β STAR, hSTAR), hmct STAR modified based on hSTAR, and Nrec STAR based on hmct STAR with N-terminal modification, were selected as the STAR constant region structure of the present invention. The costimulatory signal transduction structure comprised the intracellular signal transduction domains of CD40, OX40, ICOS, CD28, 4-1BB and CD27. In the invention, the modification could occur in TCR α chain, β chain, and α and β chains. In the invention, the costimulatory molecular modification occurred in TCR α and β chains, as shown in Fig. 60.

### 3. GPC39-targeting GC33-STAR-T function

### 1) Determination of CD19-targeting antibody sequence

The published scFv sequence FMC63 was selected as GPC3-targeting antibody heavy chain variable region (anti-GPC3 GC33 VH, SEQ ID NO: 53) and antibody light chain variable region ((anti-GPC3 GC33-VL, SEQ ID NO: 52).

### 2) Construction of CD19-targeting STAR and vector comprising costimulatory factors

STAR comprised two polypeptide chains: a fisrt polypeptide chain, which was formed by fusing anti-GPC3 FMC63-VL with the TCR bC chain of hSTAR/hmct STAR/Nrec STAR, respectively, and a second polypeptide chain, which was formed by fusing anti-GPC3 GC33 VH with the TCR aC chain of hSTAR/hmct STAR/Nrec STAR, respectively. GM-CSF signal peptide was used in both chains. The two chain sequences of hSTAR/hmct STAR/Nrec STAR were connected by the polypeptide fragment of Furin-SGSG-p2A protease cleavage site, and transcribed and translated into a protein together, then cleaved by the proteases corresponding to furin and p2A, finally producing two independent protein chains. The whole gene was inserted into a lentivirus expression vector pHAGE through restriction endonuclease sites NheI and NotI. The vector was carried with ampicin resistance gene, EF 1 α promoter and IRES-RFP fluorescent reporter gene. The following plasmids were obtained by cloning, assembling, transforming, sequencing and plasmid extraction of gene fragments: GC33-hSTAR, GC33-hmct STAR, and GC33-Nrec STAR.

Construction of STAR vector comprising costimulatory factors: on the basis of three GPC3-targeting STAR vectors: GC33-hSTAR, GC33-hmct STAR and GC33-Nrec STAR, comprising costimulatory factors CD40, OX40, ICOS, CD28, 41BB and CD27 were constructed thereon, and the above sequences were obtained by gene synthesis. The costimulatory factors were added to GC33-hSTAR, GC33-hmct STAR and GC33-Nrec STAR vectors by PCR and homologous recombination by adding the same costimulatory factor to TCR α and β chains at the same time. GC33-hSTAR- CD40, GC33-hSTAR-OX40, GC33-hSTAR-ICOS, GC33-hSTAR-CD28, GC33-hSTAR-41BB, GC33-hSTAR-CD27, GC33-hmct STAR-CD40, GC33-hmct STAR- OX40, GC33-hmct STAR-ICOS, GC33-hmct STAR-CD28, GC33-hmct STAR-41BB, GC33-hmct STAR-CD27, GC33-Nrec STAR-CD40, GC33-Nrec STAR-OX40, GC33-Nrec STAR-ICOS, GC33-Nrec STAR-CD28, GC33-Nrec STAR-41BB and GC33-Nrec STAR-CD27 were finally constructed.

### 3) Detection of killing ability of CD19-targeting STAR and STAR-T comprising a costimulatory factor

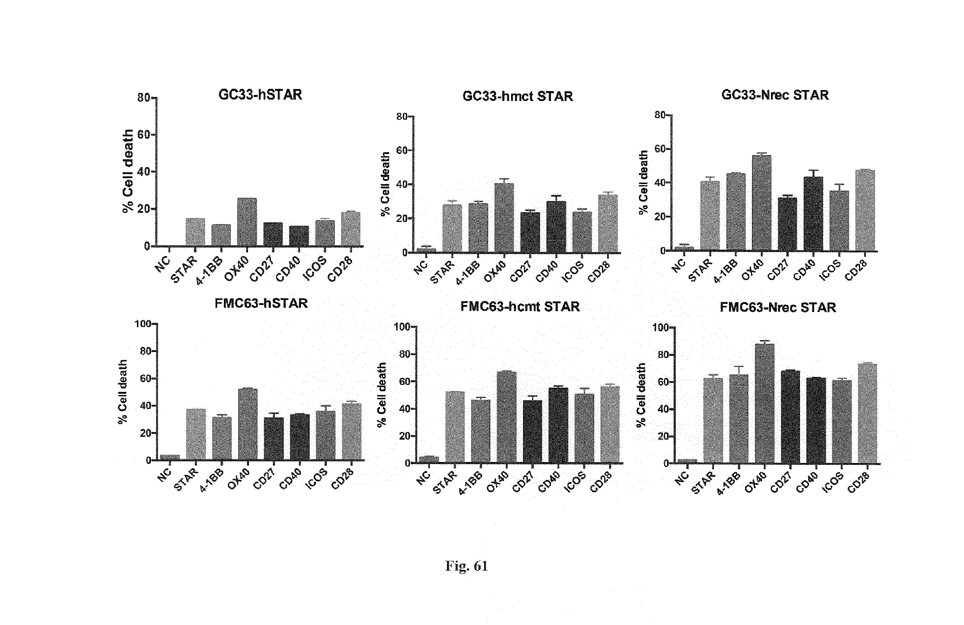

Uninfected T cells (NC group), and T cells expressing GC33-hSTAR, GC33-hmct STAR, GC33-Nrec STAR GC33-hSTAR-CD40, GC33-hSTAR-OX40, GC33-hSTAR-ICOS, GC33-hSTAR-CD28, GC33-hSTAR-41BB, GC33-hSTAR-CD27, GC33-hmct STAR-CD40, GC33-hmct STAR- OX40, GC33-hmct STAR-ICOS, GC33-hmct STAR-CD28, GC33-hmct STAR-41BB, GC33-hmct STAR-CD27, GC33-Nrec STAR-CD40, GC33-Nrec STAR-OX40, GC33-Nrec STAR-ICOS, GC33-Nrec STAR-CD28, GC33-Nrec STAR-41BB and GC33-Nrec STAR-CD27 were co-cultured with HUH-7-luc cells for 24 h in a 24-well plate. The positive number of T cells was adjusted according to RFP positive rate to 4E5 each, and the number of RAJI-luc cells was 4E5, with a total of 1 mL co-culture system. After co-culture for 24 h, the cells were centrifuged at 1500 rpm and room temperature for 5 min, after discarding the supernatant gently, added with 400 microliters of protein lysate at room temperature for lysis under shaking for 10 min, then transferred to an EP tube, centrifuged at 12000 rpm at 4 °C for 10 min, 2 multiple wells were taken for each sample, with 20 microliters per well, and added to a white 96-well plate, 50 µL luciferase substrate was added to detect a fluorescence value by a multifunctional microplate reader, and calculate the killing of target cells in each group. The results showed that the killing efficiency of hSTAR was significantly lower than that of hmct STAR and Nrec STAR, among them, Nrec STAR was the highest. The results of the same STAR comprising costimulatory factors showed that OX40 and ICOS could significantly increase the killing effect of STAR, while other costimulatory factors had no significant effect on the killing ability of STAR, 41BB reduced the killing function of STAR, however, without significant difference (Figure 61).

Fig. 4. Detection of nuclear RelB level of GPC3-targeting STAR and STAR-T comprising a costimulatory factor

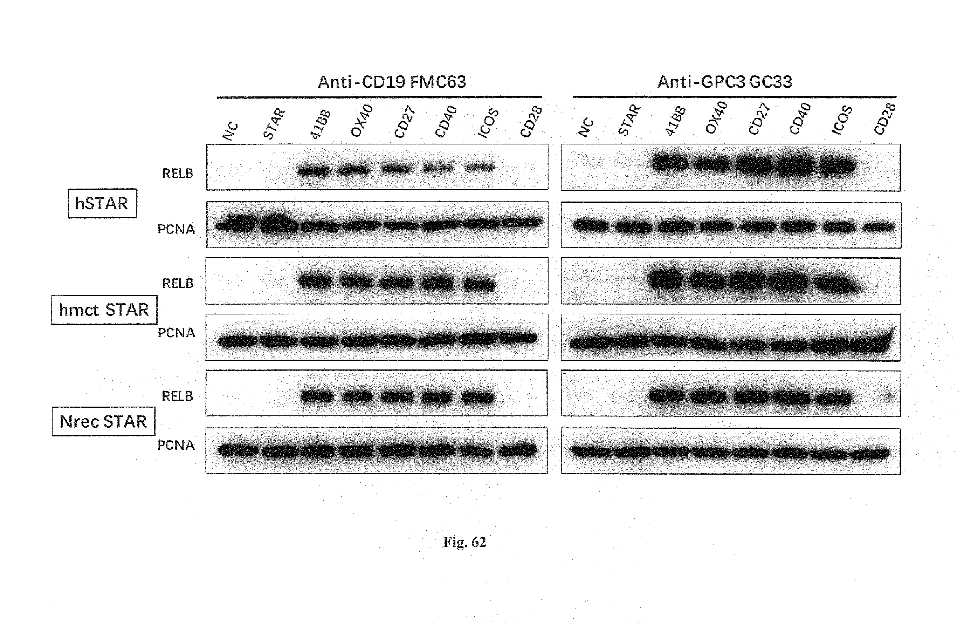

The obtained following viruses: GC33-hSTAR, GC33-hmct STAR, GC33-Nrec STAR, GC33-hSTAR-CD40, GC33-hSTAR-OX40, GC33-hSTAR-ICOS, GC33-hSTAR-CD28, GC33-hSTAR-41BB, GC33-hSTAR-CD27, GC33-hmct STAR-CD40, GC33-hmct STAR- OX40, GC33-hmct STAR-ICOS, GC33-hmct STAR-CD28, GC33-hmct STAR-41BB, GC33-hmct STAR-CD27, FMC63-Nrec STAR-CD40, FMC63-Nrec STAR-OX40, FMC63-Nrec STAR-ICOS, FMC63-Nrec STAR-CD28, FMC63-Nrec STAR-41BB and FMC63-Nrec STAR-CD27 were infected with Jurkat cells at a titer of MOI=1, after 4 days of infection, T cell lines were co-cultured with HUH-7-luc cell line in a 12-well plate, in which the STAR-T cell was 4E6 and the target cell was 2E5 (target cells were inoculated to 12-well plate one day in advance), after co-culture for 6 hours, the cells were collected for extraction of nuclear proteins, which were detected by western blotting for the nuclear RelB level. The results showed that, the nuclear RelB level was very low in GC33-hSTAR, GC33-hmct STAR and GC33-Nrec STAR groups without costimulatory factors, which was consistent with uninfected STAR-T group, after adding costimulatory factors, other costimulatory factors except CD28 significantly increased the nuclear RelB level, among which, 41BB improved the nuclear RelB level most, as shown in Figure 62.

### 4. CD19-targeting FMC63-STAR-T function

### 1) Determination of CD19-targeting antibody sequence

The published scFv sequence FMC63 was selected as CD19-targeting antibody heavy chain variable region (anti-CD19 FMC63-VH, SEQ ID NO: 50) and antibody light chain variable region (anti-CD19 FMC63-VL, SEQ ID NO: 51).

### 2) Construction of CD19-targeting STAR and vector comprising costimulatory factors

STAR comprised two polypeptide chains: a first polypeptide chain, which was formed by fusing anti-CD19 FMC63-VL with the TCR bC chain of hSTAR/hmct STAR/Nrec STAR, respectively, and a second polypeptide chain, which was formed by fusing anti-CD19 FMC63 VH with the TCR aC chain of hSTAR/hmct STAR/Nrec STAR, respectively. GM-CSFR signal peptide was used in both chains. The two chain sequences of hSTAR/hmct STAR/Nrec STAR were connected by the polypeptide fragment of Furin-SGSG-p2A protease cleavage site, and transcribed and translated into a protein together, then cleaved by the protease corresponding to furin and p2A, finally producing two independent protein chains. The whole gene was inserted into a lentivirus expression vector pHAGE through restriction endonuclease sites NheI and NotI. The vector was carried with ampicin resistance gene, EF 1 α promoter and IRES-RFP fluorescent reporter gene. The following plasmids were obtained by cloning, assembling, transforming, sequencing and plasmid extraction of gene fragments: FMC63-hSTAR, FMC63-hmct STAR, FMC63-Nrec STAR.

Construction of STAR vector comprising costimulatory factors: on the basis of three CD19-targeting STAR vectors, FMC63-hSTAR, FMC63-hmct STAR and FMC63-Nrec STAR, the costimulatory factors CD40, OX40, ICOS, CD28, 41BB and CD27 were constructed thereon, and the above sequences were obtained by gene synthesis. The costimulatory factors were added to FMC63-hSTAR, FMC63-hmct STAR and FMC63-Nrec STAR vectors by PCR and homologous recombination by adding the same costimulatory factor to TCR α and β chains at the same time. FMC63-hSTAR-CD40, FMC63-hSTAR-OX40, FMC63-hSTAR-ICOS, FMC63-hSTAR-CD28, FMC63-hSTAR-41BB, FMC63-hSTAR-CD27, FMC63-hmct STAR-CD40, FMC63-hmct STAR-OX40, FMC63-hmct STAR-ICOS, FMC63-hmct STAR-CD28, FMC63-hmct STAR-41BB, FMC63-hmct STAR-CD27, FMC63-Nrec STAR-CD40, FMC63-Nrec STAR-OX40, FMC63-Nrec STAR-ICOS, FMC63-Nrec STAR-CD28, FMC63-Nrec STAR-41BB and FMC63-Nrec STAR-CD27 were finally constructed.

### 3) Detection of killing ability of CD19-targeting STAR and STAR-T comprising a costimulatory factor

Uninfected T cells (NC group), FMC63-hSTAR, FMC63-hmct STAR, FMC63-Nrec STAR, FMC63-hSTAR-CD40, FMC63-hSTAR-OX40, FMC63-hSTAR-ICOS, FMC63-hSTAR-CD28, FMC63-hSTAR-41BB, FMC63-hSTAR-CD27, FMC63-hmct STAR-CD40, FMC63-hmct STAR-OX40, FMC63-hmct STAR-ICOS, FMC63-hmct STAR-CD28, FMC63-hmct STAR-41BB, FMC63-hmct STAR-CD27, FMC63-Nrec STAR-CD40, FMC63-Nrec STAR-OX40, FMC63-Nrec STAR-ICOS, FMC63-Nrec STAR-CD28, FMC63-Nrec STAR-41BB and FMC63-Nrec STAR-CD27 were co-cultured with RAJI-luc cells for 24 h in a 24-well plate. The poistive cell number of T cells were adjusted according to RFP positive rate to 4E5 each, and the number of RAJI-luc cells was 4E5, with a total of 1 mL co-culture system. After 24 hours of co-culture, co-cultured cells were mixed evenly, 150 µL suspension was sucked out and added with 70 µL luciferase substrate, after shaking for 10 min at low speed in dark, the fluorescence value was detected by a multifunctional microplate reader, and the target cell-killing effect of each group was calculated. The results showed that the killing efficiency of hSTAR was significantly lower than that of hmct STAR and Nrec STAR, among them, Nrec STAR was the highest. The results of the same STAR comprising costimulatory factors showed that OX40 and ICOS could significantly increase the killing effect of STAR, while other costimulatory factors had no significant effect on the killing ability of STAR, 41BB reduced the killing function of STAR, however, without significant difference (Figure 61).

### 4) Detection of nuclear RelB level of CD19-targeting STAR and STAR-T comprising a costimulatory factor

The obtained following viruses: FMC63-hSTAR, FMC63-hmct STAR, FMC63-Nrec STAR, FMC63-hSTAR-CD40, FMC63-hSTAR-OX40, FMC63-hSTAR-ICOS, FMC63-hSTAR-CD28, FMC63-hSTAR-41BB, FMC63-hSTAR-CD27, FMC63-hmct STAR-CD40, FMC63-hmct STAR-OX40, FMC63-hmct STAR-ICOS, FMC63-hmct STAR-CD28, FMC63-hmct STAR-41BB, FMC63-hmct STAR-CD27, FMC63-Nrec STAR-CD40, FMC63-Nrec STAR-OX40, FMC63-Nrec STAR-ICOS, FMC63-Nrec STAR-CD28, FMC63-Nrec STAR-41BB and FMC63-Nrec STAR-CD27 were infected with Jurkat cells at a titer of MOI=1, after 4 days of infection, T cell lines and CD19 protein were co-cultured in a 12-well plate (2 µ g/mL, 500 µL was coated on the 12-well plate and left overnight in a 4 °C refrigerator), in which the STAR-T cell was 4E6 and the target cell was 2E5 (target cells were inoculated to 12-well plate one day in advance) were cultured for 6 hours, then the cells were collected for extraction of nuclear proteins, which were detected by western blotting for the nuclear RelB level. The results showed that, the nuclear RelB level was very low in FMC63-hSTAR, FMC63-hmct STAR and FMC63-Nrec STAR groups without costimulatory factors, which was consistent with uninfected STAR-T group, after adding costimulatory factors, other costimulatory factors except CD28 significantly increased the nuclear RelB level, among which, 41BB improved the nuclear RelB level most, as shown in Figure 62.

### 5. CD19-targeting 334-STAR-T function

### 1) Determination of CD19-targeting antibody sequence

A 334 antibody sequence developed by the present inventors was selected as CD19-targeting antibody heavy chain variable region (anti-CD19 334-VH, SEQ ID NO: 54) and antibody light chain variable region (anti-CD19 334-VL, SEQ ID NO: 55).

### 2) Construction of CD19-targeting STAR and vector comprising costimulatory factors

STAR comprised two polypeptide chains: a fisrt polypeptide chain, which was formed by fusing anti-CD19 334-VL with the TCR bC chain of hSTAR/hmct STAR/Nrec STAR, respectively, and a second polypeptide chain, which was formed by fusing anti-CD19 334 VH with the TCR aC chain of hSTAR/hmct STAR/Nrec STAR, respectively. GM-CSFR signal peptide was used in both chains. The two chain sequences of hmct STAR/Nrec STAR were connected by the polypeptide fragment of Furin-SGSG-p2A protease cleavage site, and transcribed and translated into protein together, then cleaved by the protease corresponding to furin and p2A, finally producing two independent protein chains. The whole gene was inserted into a lentivirus expression vector pHAGE through restriction endonuclease sites NheI and NotI. The vector was carried with ampicin resistance gene, EF 1 α promoter and IRES-RFP fluorescent reporter gene. The following plasmids were obtained by cloning, assembling, transforming, sequencing and plasmid extraction of gene fragments: 334-hmct STAR, 334-Nrec STAR.

Construction of STAR vector comprising costimulatory factors: on the basis of CD19-targeting STAR vectors, 334-hmct STAR and 334-Nrec STAR, a costimulatory factor OX40 was constructed thereon, and the above sequences were obtained by gene synthesis. The costimulatory factor was added to 334-hmct STAR and 334-Nrec STAR vectors by PCR and homologous recombination by adding the same costimulatory factor to TCR α and β chains at the same time. Finally, 334-hmct STAR-OX40 and 334-Nrec STAR-OX40 were constructed.

### 3) Detection of killing ability of CD19-targeting STAR and STAR-T comprising a costimulatory factor

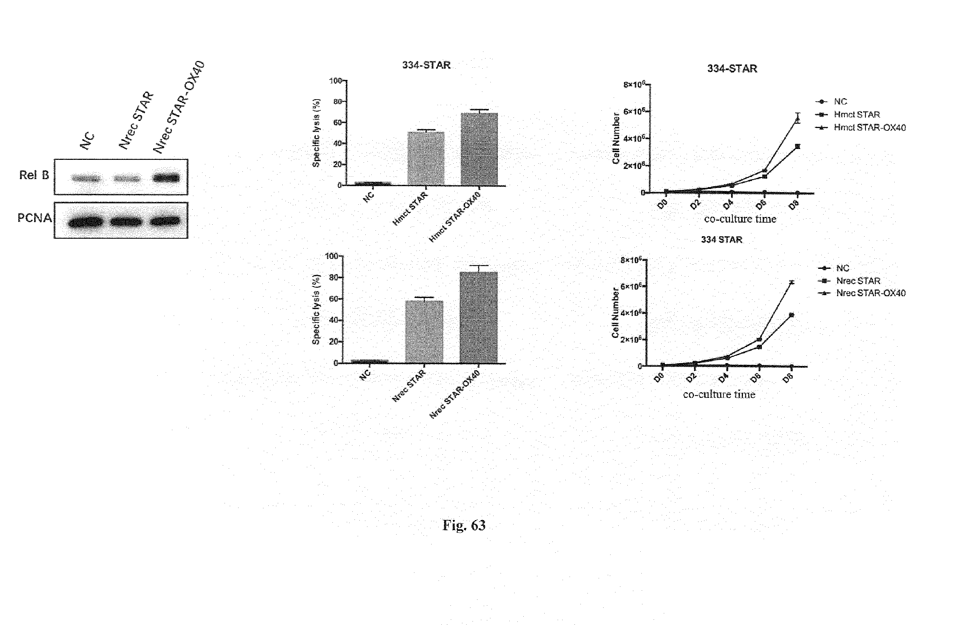

Uninfected T cells (NC group), 334-hmct STAR, 334-Nrec STAR, 334-hmct STAR- OX40 and 334-Nrec STAR-OX40 were co-cultured with RAJI-luc cells for 24 h in a 24-well plate. The poistive cell number of T cells were adjusted according to RFP positive rate to 4E5 each, and the number of RAJI-luc cells was 4E5, with a total of 1 mL co-culture system. After 24 hours of co-culture, co-cultured cells were mixed evenly, 150 µL suspension was sucked out and added with 70 µL luciferase substrate, after shaking for 10 min at low speed in dark, the fluorescence value was detected by a multifunctional microplate reader, and the target cell-killing effect of each group was calculated. The results showed that the killing efficiency of hmct STAR was significantly lower than that of Nrec STAR, which was the highest. In addition, OX40 can significantly increase the killing effect of STAR and the nuclear RelB level. See Fig. 63.

### 6. CD19 and CD 20 -double targeting STAR-T function

### 1) Determination of CD19 and CD 20-targeting antibody sequence

CD19-targeting antibody heavy chain variable region (anti-CD19 FMC63-VH, SEQ ID NO: 50) and antibody light chain variable region (anti-CD19 FMC63-VL, SEQ ID NO: 51); CD 20-targeting antibody heavy chain variable region (anti-CD20 2C6-VH, SEQ ID NO: 62) and antibody light chain variable region (anti-CD20 2C6-VL, SEQ ID NO: 63).

### 2) Construction of CD19 and CD 20-targeting STAR and vector comprising costimulatory factors

STAR comprised two polypeptide chains: a fisrt polypeptide chain, which was formed by fusing anti-CD20 2C6 VL-(G4S)3-VH with the TCR bC chain of hmct STAR/Nrec STAR, respectively, and a second polypeptide chain, which was formed by fusing nti-CD19 FMC63 VL-(G4S)3-VH with the TCR aC chain of hmct STAR/Nrec STAR, respectively. GM-CSFR signal peptide was used in both chains. The two chain sequences of hmct STAR/Nrec STAR were connected by the polypeptide fragment of Furin-SGSG-p2A protease cleavage site, and transcribed and translated into protein together, then cleaved by the protease corresponding to furin and p2A, finally producing two independent protein chains. The whole gene was inserted into a lentivirus expression vector pHAGE through restriction endonuclease sites NheI and NotI. The vector was carried with ampicin resistance gene, EF 1 α promoter and IRES-RFP fluorescent reporter gene. The following plasmids were obtained by cloning, assembling, transforming, sequencing and plasmid extraction of gene fragments: FMC63-2C6-hmct STAR and FMC63-2C6-Nrec STAR.

Construction of STAR vector comprising costimulatory factors: on the basis of CD19 and CD20-targeting STAR vectors, FMC63-2C6-hmct STAR and FMC63-2C6-Nrec STAR, a costimulatory factor OX40 was constructed thereon, and the above sequences were obtained by gene synthesis. The costimulatory factors were added to FMC63-2C6-hmct STAR and FMC63-2C6-Nrec STAR vectors by PCR and homologous recombination by adding the same costimulatory factor to TCR α and β chains at the same time. Finally, FMC63-2C6-hmct STAR-OX40 and FMC63-2C6-Nrec STAR-OX40 were constructed.

### 3) Detection of killing ability of CD19 and CD20-targeting STAR and STAR-T comprising a costimulatory factor

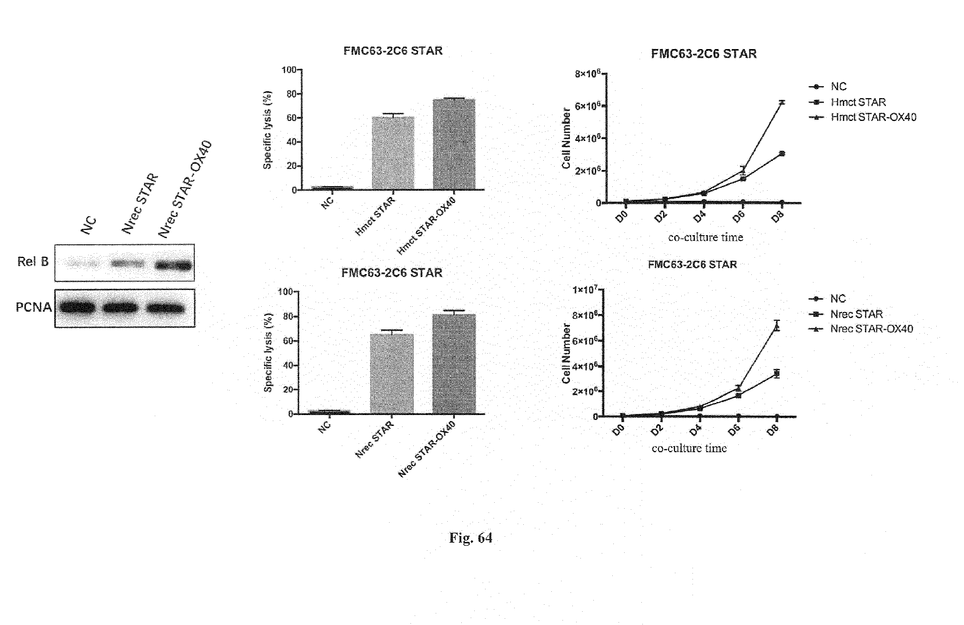

Uninfected T cells (NC group), FMC63-2C6-hmct STAR, FMC63-2C6-Nrec STAR, FMC63-2C6-hmct STAR-OX40 and FMC63-2C6-Nrec STAR-OX40 were co-cultured with RAJI-luc cells for 24 h in a 24-well plate. The positive number of T cells was adjusted according to RFP positive rate to 4E5 each, and the number of RAJI-luc cells was 4E5, with a total of 1 mL co-culture system. After 24 hours of co-culture, co-cultured cells were mixed evenly, 150 µL suspension was sucked out and added with 70 µL luciferase substrate, after shaking for 10 min at low speed in dark, the fluorescence value was detected by a multifunctional microplate reader, and the target cell-killing effect of each group was calculated. The results showed that the killing efficiency ofhmct STAR was significantly lower than that of Nrec STAR, which was the highest. In addition, OX40 can significantly increase the killing effect of STAR and the nuclear RelB level, see Fig. 64.

### Sequence Listing

SEQ ID NO: 1 Amino acid sequence of wild-type human TCR α constant region 
DIQNPDPAVYQLRDSKSSDKSVCLFTDFDSQTNVSQSKDSDVYITDKTVLDMRSMDF
KSNSAVAWSNKSDFACANAFNNSIIPEDTFFPSPESSCDVKLVEKSFETDTNLNFQNLS
VIGFRILLLKVAGFNLLMTLRLWSS*
SEQ ID NO: 2 Amino acid sequence of wild-type human TCR β constant region 
DLKNVFPPEVAVFEPSEAEISHTQKATLVCLATGFFPDHVELSWWVNGKEVHSGVST
DPQPLKEQPALNDSRYCLSSRLRVSATFWQNPRNHFRCQVQFYGLSENDEWTQDRAK
PVTQIVSAEAWGRADCGFTSVSYQQGVLSATILYEILLGKATLYAVLVSALVLMAMVK
RKDF*
SEQ ID NO: 3 Amino acid sequence of wild-type mouse TCR α constant region 
DIQNPEPAVYQLKDPRSQDSTLCLFTDFDSQINVPKTMESGTFITDKTVLDMKAMDSKSN
GAIAWSNQTSFTCQDIFKETNATYPSSDVPCDATLTEKSFETDMNLNFQNLSVMGLRILLL
KVAGFNLLMTLRLWSS
SEQ ID NO: 4 Amino acid sequence of wild-type mouse TCR β constant region 
DLRNVTPPKVSLFEPSKAEIANKQKATLVCLARGFFPDHVELSWWVNGKEVHSGVSTDPQ
AYKESNYSYCLSSRLRVSATFWHNPRNHFRCQVQFHGLSEEDKWPEGSPKPVTQNISAEA
 WGRADCGITSASYQQGVLSATILYEILLGKATLYAVLVSTLVVMAMVKRKNS
SEQ ID NO: 5 Mouse T cell receptor α chain constant region with cysteine substitution (mouse TCRaC- Cys) 
DIQNPEPAVYQLKDPRSQDSTLCLFTDFDSQINVPKTMESGTFITDKCVLDMKAMDSKSN
GAIAWSNQTSFTCQDIFKETNATYPSSDVPCDATLTEKSFETDMNLNFQNLSVMGLRILLL
KVAGFNLLMTLR LWSS
SEQ ID NO: 6 Mouse T cell receptor β chain constant region with cysteine substitution (mouse TCRβC- Cys) 
DLRNVTPPKVSLFEPSKAEIANKQKATLVCLARGFFPDHVELSWWVNGKEVHSGVCTDP
QAYKESNYSYCLSSRLRVSATFWHNPRNHFRCQVQFHGLSEEDKWPEGSPKPVTQNISAE
AWGRADCGITSASYQQGVLSATILYEILLGKATLYAVLVSTLVVMAMVKRKNS
SEQ ID NO: 7 Mouse T cell receptor α chain constant region with hydrophobic amino acid substitution (mouse TCRaC-TM9) 
DIQNPEPAVYQLKDPRSQDSTLCLFTDFDSQINVPKTMESGTFITDKTVLDMKAMDSKSN
GAIAWSNQTSFTCQDIFKETNATYPSSDVPCDATLTEKSFETDMNLNFQNLLVIVLRILLLK
VAGFNLLMTLRL WSS
SEQ ID NO: 8 Mouse T cell receptor α chain constant region with cysteine substitution in transmembrane domain (mouse TCRaC-Arg mut) 
DIQNPEPAVYQLKDPRSQDSTLCLFTDFDSQINVPKTMESGTFITDKCVLDMKAMDSKSN
GAIAWSNQTSFTCQDIFKETNATYPSSDVPCDATLTEKSFETDMNLNFQNLLVIVLRILLLR
VAGFNLLMTLRLWSS
SEQ ID NO: 9 Mouse T cell receptor β chain constant region with cysteine substitution in endodomain (Mouse TCRβC-Arg mut) 
DLRNVTPPKVSLFEPSKAEIANKQKATLVCLARGFFPDHVELSWWVNGKEVHSGVCTDP
QAYKESNYSYCLSSRLRVSATFWHNPRNHFRCQVQFHGLSEEDKWPEGSPKPVTQNISAE
AWGRADCGITSASYQQGVLSATILYEILLGRATLYAVLVSTLVVMAMVRRRNS
SEQ ID NO: 10 Amino acid sequence of CD40 endodomain
   KKVAKKPTNKAPHPKQEPQEINFPDDLPGSNTAAPVQETLHGCQPVTQEDGKESRISV
SEQ ID NO: 11 Amino acid sequence of OX40 endodomain
   RRDQRLPPDAHKPPGGGSFRTPIQEEQADAHSTLAKI
SEQ ID NO:12 Amino acid sequence of ICOS endodomain
   KKKYSSSVHDPNGEYMFMRAVNTAKKSRLTDVTL
SEQ ID NO: 13 Amino acid sequence of CD28 endodomain
   RSKRSRLLHSDYMNMTPRRPGPTRKHYQPYAPPRDFAAYRS
SEQ ID NO: 14 Amino acid sequence of 4-1BB endodomain
   KRGRKKLLYIFKQPFMRPVQTTQEEDGCSCRFPEEEEGGCEL
SEQ ID NO: 15 Amino acid sequence of CD27 endodomain
   QRRKYRSNKGESPVEPAEPCRYSCPREEEGSTIPIQEDYRKPEPACSP
SEQ ID NO: 16 Amino acid sequence of G4S linker
   GGGGS
SEQ ID NO: 17 Amino acid sequence of (G4S)2 linker
   GGGGSGGGGS
SEQ ID NO: 18 Amino acid sequence of (G4S)3 linker
   GGGGSGGGGSGGGGS
SEQ ID NO: 19 Amino acid sequence of (G4S)4 linker
   GGGGSGGGGSGGGGSGGGGS
SEQ ID NO: 20 Amino acid sequence of (G4S)5 linker
   GGGGSGGGGSGGGGSGGGGSGGGGS
SEQ ID NO: 21 Amino acid sequence of (G4S)6 linker
   GGGGSGGGGSGGGGSGGGGSGGGGSGGGGS
SEQ ID NO: 22 Amino acid sequence of (G4S)7 linker
   GGGGSGGGGSGGGGSGGGGSGGGGSGGGGSGGGGS
SEQ ID NO: 23 Amino acid sequence of (G4S)8 linker
   GGGGSGGGGSGGGGSGGGGSGGGGSGGGGSGGGGSGGGGS
SEQ ID NO: 24 Amino acid sequence of (G4S)9 linker
   GGGGSGGGGSGGGGSGGGGSGGGGSGGGGSGGGGSGGGGSGGGGS
SEQ ID NO: 25 Amino acid sequence of (G4S)10 linker
   GGGGSGGGGSGGGGSGGGGSGGGGSGGGGSGGGGSGGGGSGGGGSGGGGS
SEQ ID NO: 26 Endodomain-deleted mouse T cell receptor α chain constant region with cysteine substitution and hydrophobic modification (mouse TCRαC-del mut) 
DIQNPEPAVYQLKDPRSQDSTLCLFTDFDSQINVPKTMESGTFITDKCVLDMKAMDSKSN
GAIAWSNQTSFTCQDIFKETNATYPSSDVPCDATLTEKSFETDMNLNFQNLLVIVLRILLLK
VAGFNLLMTLRLW
SEQ ID NO: 27 Endodomain-deleted mouse T cell receptor β chain constant region with cysteine substitution (mouse TCRβC-del mut) 
DLRNVTPPKVSLFEPSKAEIANKQKATLVCLARGFFPDHVELSWWVNGKEVHSGVCTDP
QAYKESNYSYCLSSRLRVSATFWHNPRNHFRCQVQFHGLSEEDKWPEGSPKPVTQNISAE
AWGRADCGITSASYQQGVLSATILYEILLGKATLYAVLVSTLVVMAM
SEQ ID NO: 28 Amino acid sequence of human CD3γ MEQGKGLAVLILAIILLQGTLAQSIKGNHLVKVYDYQEDGSVLLTCDAEAKNITWFKDGK 
MIGFLTEDKKKWNLGSNAKDPRGMYQCKGSQNKSKPLQVYYRMCQNCIELNAATISGFL
FAEIVSIFVLAVGVYFIAGQDGVRQSRASDKQTLLPNDQLYQPLKDREDDQYSHLQGNQL
RRN
SEQ ID NO: 29 Amino acid sequence of human CD3δ 
MEHSTFLSGLVLATLLSQVSPFKIPIEELEDRVFVNCNTSITWVEGTVGTLLSDITRLDLGKR
ILDPRGIYRCNGTDIYKDKESTVQVHYRMCQSCVELDPATVAGIIVTDVIATLLLALGVFCF
AGHETGRLSGAADTQALLRNDQVYQPLRDRDDAQYSHLGGNWARNK
SEQ ID NO: 30 Amino acid sequence of human CD3ε 
MQSGTHWRVLGLCLLSVGVWGQDGNEEMGGITQTPYKVSISGTTVILTCPQYPGSEILWQ
HNDKNIGGDEDDKNIGSDEDHLSLKEFSELEQSGYYVCYPRGSKPEDANFYLYLRARVCE
NCMEMDVMSVATIVIVDICITGGLLLLVYYWSKNRKAKAKPVTRGAGAGGRQRGQNKE
RPPPVPNPDYEPIRKGQRDLYSGLNQRRI
SEQ ID NO: 31 Amino acid sequence of human CD3ζ 
MKWKALFTAAILQAQLPITEAQSFGLLDPKLCYLLDGILFIYGVILTALFLRVKFSRSADAP
AYQQGQNQLYNELNLGRREEYDVLDKRRGRDPEMGGKPRRKNPQEGLYNELQKDKMA
EAYSEIGMKGERRRGKGHDGLYQGLSTATKDTYDALHMQALPPR
SEQ ID NO: 32 Amino acid sequence of human IL-2β receptor endodomain 
NCRNTGPWLKKVLKCNTPDPSKFFSQLSSEHGGDVQKWLSSPFPSSSFSPGGLAPEISPLE
VLERDKVTQLLPLNTDAYLSLQELQGQDPTHLV
SEQ ID NO: 33 Amino acid sequence of human IL-7α receptor endodomain 
KKRIKPIVWPSLPDHKKTLEHLCKKPRKNLNVSFNPESFLDCQIHRVDDIQARDEVEGFLQ
DTFPQQLEESEKQRLGGDVQSPNCPSEDVVITPESFGRDSSLTCLAGNVSACDAPILSSSRS
LDCRESGKNGPHVYQDLLLSLGTTNSTLPPPFSLQSGILTLNPVAQGQPILTSLGSNQEEAY
VTMSSFYQNQ
SEQ ID NO: 34 Amino acid sequence of human IL-21 receptor endodomain 
SLKTHPLWRLWKKIWAVPSPERFFMPLYKGCSGDFKKWVGAPFTGSSLELGPWSPEVPST
LEVYSCHPPRSPAKRLQLTELQEPAELVESDGVPKPSFWPTAQNSGGSAYSEERDRPYGLV
SIDTVTVLDAEGPCTWPCSCEDDGYPALDLDAGLEPSPGLEDPLLDAGTTVLSCGCVSAG
SPGLGGPLGSLLDRLKPPLADGEDWAGGLPWGGRSPGGVSESEAGSPLAGLDMDTFDSG
FVGSDCSSPVECDFTSPGDEGPPRSYLRQWVVIPPPLSSPGPQAS
SEQ ID NO: 35 Amino acid sequence of human STATS activation moiety
   YRHQ
SEQ ID NO: 36 Amino acid sequence of human IL-2β receptor endodomain and human STATS activation moiety NCRNTGPWLKKVLKCNTPDPSKFFSQLSSEHGGDVQKWLSSPFPSSSFSPGGLAPEISPLE VLERDKVTQLLPLNTDAYLSLQELQGQDPTHLVGGGGSYRHQ
SEQ ID NO: 37 Amino acid sequence of human IL-7α receptor endodomain and human STATS activation moiety 
KKRIKPIVWPSLPDHKKTLEHLCKKPRKNLNVSFNPESFLDCQIHRVDDIQARDEVEGFLQ
DTFPQQLEESEKQRLGGDVQSPNCPSEDVVITPESFGRDSSLTCLAGNVSACDAPILSSSRS
LDCRESGKNGPHVYQDLLLSLGTTNSTLPPPFSLQSGILTLNPVAQGQPILTSLGSNQEEAY
VTMSSFYQNQGGGGSYRHQ
SEQ ID NO: 38 Amino acid sequence of anti-CD20 OFA ScFv 
EIVLTQSPATLSLSPGERATLSCRASQSVSSYLAWYQQKPGQAPRLLIYDASNRATGIPARFS
GSGSGTDFTLTISSLEPEDFAVYYCQQRSNWPITFGQGTRLEIKGGGGSGGGGSGGGGSEV
QLVESGGGLVQPGRSLRLSCAASGFTFNDYAMHWVRQAPGKGLEWVSTISWNSGSIGYA
DSVKGRFTISRDNAKKSLYLQMNSLRAEDTALYYCAKDIQYGNYYYGMDVWGQGTTVT
VSS
SEQ ID NO: 39 Amino acid sequence of anti-CD19 FMC63 ScFv 
DIQMTQTTSSLSASLGDRVTISCRASQDISKYLNWYQQKPDGTVKLLIYHTSRLHSGVPSR
FSGSGSGTDYSLTISNLEQEDIATYFCQQGNTLPYTFGGGTKLEITGGGGSGGGGSGGGGS
EVKLQESGPGLVAPSQSLSVTCTVSGVSLPDYGVSWIRQPPRKGLEWLGVIWGSETTYYN
SALKSRLTIIKDNSKSQVFLKMNSLQTDDTAIYYCAKHYYYGGSYAMDYWGQGTSVTVS
S
SEQ ID NO: 40 Amino acid sequence of red fluorescent protein 
MASSEDVIKEFMRFKVRMEGSVNGHEFEIEGEGEGRPYEGTQTAKLKVTKGGPLPFAWDI
LSPQFQYGSKAYVKHPADIPDYLKLSFPEGFKWERVMNFEDGGVVTVTQDSSLQDGEFIY
KVKLRGTNFPSDGPVMQKKTMGWEASTERMYPEDGALKGEIKMRLKLKDGGHYDAEV
KTTYMAKKPVQLPGAYKTDIKLDITSHNEDYTIVEQYERAEGRHSTGA*
SEQ ID NO: 41 Mouse TCRaC- Cys- TM9 (hmct STAR TCRaC) 
DIQNPEPAVYQLKDPRSQDSTLCLFTDFDSQINVPKTMESGTFITDKCVLDMKAMDSKSN
GAIAWSNQTSFTCQDIFKETNATYPSSDVPCDATLTEKSFETDMNLNFQNLLVIVLRILLLK
VAGFNLLMTLRLWSS
SEQ ID NO: 42 Mouse TCRaC- Cys-TM9-N.Rec (Nrec STAR TCRaC) 
DIQNPDPAVYQLRDDSTLCLFTDFDSQINVPKTMESGTFITDKCVLDMKAMDSKSNGAIA
WSNQTSFTCQDIFKETNATYPSSDVPCDATLTEKSFETDMNLNFQNLLVIVLRILLLKVAGF
NLLMTLRLWSS
SEQ ID NO: 43 Mouse T cell receptor β chain constant region with N-terminal modification and cysteine substitution (Mouse TCRbC-Cys-N.Rec, Nrec STAR TCRbC) DLKNVFPPEVAVFEPSAEIATLVCLARGFFPDHVELSWWVNGKEVHSGVCTDPQAYKESN 
YSYCLSSRLRVSATFWHNPRNHFRCQVQFHGLSEEDKWPEGSPKPVTQNISAEAWGRAD
CGITSASYQQGVLSATILYEILLGKATLYAVLVSTLVVMAMVKRKNS
> SEQ ID NO: 44 Amino acid sequence of TCR-E141 α variable region 
MKRILGALLGLLSAQVCCVRGIQVEQSPPDLILQEGANSTLRCNFSDSVNNLQWFHQNPW
GQLINLFYIPSGTKQNGRLSATTVATERYSLLYISSSQTTDSGVYFCAVLNNNDMRFGAGTR
LTVKP
> SEQ ID NO: 45 Amino acid sequence of CR-E141 β variable region 
MGCRLLCCAVLCLLGAVPIDTEVTQTPKHLVMGMTNKKSLKCEQHMGHRAMYWYKQK
AKKPPELMFVYSYEKLSINESVPSRFSPECPNSSLLNLHLHALQPEDSALYLCASSQGRWY
EQYFGPGTRLTVT
> SEQ ID NO: 46 Amino acid sequence of TCR-E315 α variable region 
METLLGLLILWLQLQWVSSKQEVTQIPAALSVPEGENLVLNCSFTDSAIYNLQWFRQDPG
KGLTSLLLIQSSQREQTSGRLNASLDKSSGRSTLYIAASQPGDSATYLCAGKTSYDKVIFGP
GTSLSVIP
> SEQ ID NO: 47 Amino acid sequence of TCR-E315 β variable region 
MGTRLLCWAALCLLGAELTEAGVAQSPRYKIIEKRQSVAFWCNPISGHATLYWYQQILGQ
GPKLLIQFQNNGVVDDSQLPKDRFSAERLKGVDSTLKIQPAKLEDSAVYLCASSVFPTSVE
QYFGPGTRLTVT
> SEQ ID NO: 48 Amino acid sequence of TCR-E316 α variable region 
MSLSSLLKVVTASLWLGPGIAQKITQTQPGMFVQEKEAVTLDCTYDTSDQSYGLFWYKQP
SSGEMIFLIYQGSYDEQNATEGRYSLNFQKARKSANLVISASQLGDSAMYFCAMVSGAGG
GADGLTFGKGTHLIIQP
> SEQ ID NO: 49 Amino acid sequence of TCR-E316 β variable region 
MSNQVLCCVVLCLLGANTVDGGITQSPKYLFRKEGQNVTLSCEQNLNHDAMYWYRQDP
GQGLRLIYYSQIVNDFQKGDIAEGYSVSREKKESFPLTVTSAQKNPTAFYLCASSIGVGLSN
TEAFFGQGTRLTVV
SEQ ID NO:50 Anti-CD19 FMC63 VH 
EVKLQESGPGLVAPSQSLSVTCTVSGVSLPDYGVSWIRQPPRKGLEWLGVIWGSETTYYN
SALKSRLTIIKDNSKSQVFLKMNSLQTDDTAIYYCAKHYYYGGSYAMDYWGQGTSVTVS
S
SEQ ID NO:51 Anti-CD19 FMC63 VL 
DIQMTQTTSSLSASLGDRVTISCRASQDISKYLNWYQQKPDGTVKLLIYHTSRLHSGVPSR
FSGSGSGTDYSLTISNLEQEDIATYFCQQGNTLPYTFGGGTKLEIT
SEQ ID NO:52 Anti-GPC3 GC33 VL DVVMTQSPLSLPVTPGEPASISCRSSQSLVHSNRNTYLHWYLQKPGQSPQLLIYKVSNRFS GVPDRFSGSGSGTDFTLKISRVEAEDVGVYYCSQNTHVPPTFGQGTKLEIKR
SEQ ID NO:53 Anti-GPC3 GC33 VH 
QVQLVQSGAEVKKPGASVKVSCKASGYTFTDYEMHWVRQAPGQGLEWMGALDPKTGD
TAYSQKFKGRVTLTADESTSTAYMELSSLRSEDTAVYYCTRFYSYTYWGQGTLVTVSS
SEQ ID NO:54 Anti-CD19 334 VH 
QVQLQQSGAELVRPGASVKLSCKALGFIFTDYEIHWVKQTPVHGLEWIGAFHPGSGGSAY
NQKFKGKATLTADKSSSTAYMELSSLTFEDSAVYHCTRQLGPDWGQGTLVTVS
SEQ ID NO:55 Anti-CD19 334 VL 
DVVMTQTPLTLSVTIGQPASISCKSSQSLLESDGKTYLNWLLQRPGQSPKRLIYLVSKLDSG
VPDRFTGSGSGTDFTLRISRVEAEDLGVYYCWQGTQFPWTFGGGTKLEIK
SEQ ID NO: 56 Endodomain-deleted mouse T cell receptor α chain constant region with N-terminal modification, cysteine substitution and hydrophobic modification in transmembrane domain 
DIQNPDPAVYQLRDDSTLCLFTDFDSQINVPKTMESGTFITDKCVLDMKAMDSKSNGAIA
WSNQTSFTCQDIFKETNATYPSSDVPCDATLTEKSFETDMNLNFQNLLVIVLRILLLKVAGF
NLLMTLRLW
SEQ ID NO: 57 Endodomain-deleted mouse T cell receptor β chain constant region with N-terminal modification and cysteine substitution 
DLKNVFPPEVAVFEPSAEIATLVCLARGFFPDHVELSWWVNGKEVHSGVCTDPQAYKESN
YSYCLSSRLRVSATFWHNPRNHFRCQVQFHGLSEEDKWPEGSPKPVTQNISAEAWGRAD
CGITSASYQQGVLSATILYEILLGKATLYAVLVSTLVVMAM
SEQ ID NO: 58 Amino acid sequence of wild-type human of TCR γ chain constant region: 
DKQLDADVSPKPTIFLPSIAETKLQKAGTYLCLLEKFFPDVIKIHWQEKKSNTILGSQEGNT
MKTNDTYMKFSWLTVPEKSLDKEHRCIVRHENNKNGVDQEIIFPPIKTDVITMDPKDNCS
KDANDTLLLQLTNTSAYYMYLLLLLKSVVYFAIITCCLLRRTAFCCNGEKS
SEQ ID NO: 59 Amino acid sequence of wild-type mouse TCR γ chain constant region: 
XKRLDADISPKPTIFLPSVAETNLHKTGTYLCLLEKFFPDVIRVYWKEKDGNTILDSQEGDT
LKTNDTYMKFSWLTVPERAMGKEHRCIVKHENNKGGADQEIFFPSIKKVAVSTKPTTCWQ
DKNDVLQLQFTITSAYYTYLLLLLKSVIYLAIISFSLLRRTSVCGNEKKS
SEQ ID NO: 60 Amino acid sequence of wild-type human of TCR δ chain constant region: 
XSQPHTKPSVFVMKNGTNVACLVKEFYPKDIRINLVSSKKITEFDPAIVISPSGKYNAVKLG
KYEDSNSVTCSVQHDNKTVHSTDFEVKTDSTDHVKPKETENTKQPSKSCHKPKAIVHTE
KVNMMSLTVLGLRMLFAKTVAVNFLLTAKLFFL*
SEQ ID NO: 61 Amino acid sequence of wild-type mouse TCR δ chain constant region XSQPPAKPSVFIMKNGTNVACLVKDFYPKEVTISLRSSKKIVEFDPAIVISPSGKYSAVKLGQ 
YGDSNSVTCSVQHNSETVHSTDFEPYANSFNNEKLPEPENDTQISEPCYGPRVTVHTEKVN
MMSLTVLGLRLLFAKTIAINFLLTVKLFF*
SEQ ID NO: 62 Anti CD20 2C6 VH 
AVQLVESGGGLVQPGRSLRLSCAASGFTFGDYTMHWVRQAPGKGLEWVSGISWNSGSIG
YADSVKGRFTISRDNAKNSLYLQMNSLRAEDTALYYCTKDNQYGSGSTYGLGVWGQGTL
VTVSS
SEQ ID NO: 63 Anti CD20 2C6 VL

EIVLTQSPATLSLSPGERATLSCRASQSVSSYLAWYQQKPGQAPRLLIYDASNRATGIPARFS
GSGSG TDFTLTISSLEPEDFAVYYCQQRSNWPLTFGGGTKVEIK

## Claims

1. A modified T cell receptor (TCR) complex, wherein
i) the TCR complex comprises a TCR α chain, a TCR β chain, CD3 ε, CD3 γ, CD3 δ and CD3 ζ; wherein at least one functional domain, such as a costimulatory molecule endodomain, is connected to the C-terminal of at least one of the TCR α chain, TCR β chain, CD3 ε, CD3 γ, CD3 δ and CD3 ζ; wherein the TCR α chain comprises a first target binding region and a first constant region, and the TCR β chain comprises a second target binding region and a second constant region; or
ii) the TCR complex comprises a TCR γ chain, a TCR δ chain, CD3 ε, CD3 γ, CD3 δ and CD3 ζ; wherein at least one functional domain is connected to the C-terminal of at least one of the TCR γ chain, TCR δ chain, CD3 ε, CD3 γ, CD3 δ and CD3 ζ; wherein the TCR γ chain comprises a first target binding region and a first constant region, and the TCR δ chain comprises a second target binding region and a second constant region,
preferably, the target binding region is an antigen-binding region.

2. The modified T cell receptor complex of claim 1, wherein the antigen-binding region is derived from
i) an antibody;
ii) a receptor, such as a native T cell receptor; or
iii) a ligand.

3. The modified T cell receptor complex of claim 1 or 2, wherein the natural endodomain of at least one of the TCR α chain, the TCR β chain, CD3 ε, CD3 γ, CD3 δ and CD3 ζ in the complex of i) is deleted, or the natural endodomain of at least one of the TCR γ chain, the TCR δ chain, CD3 ε, CD3 γ, CD3 δ and CD3 ζ in the complex of ii) is deleted.

4. The modified T cell receptor complex of claim 3, wherein in the complex of i), the functional domain is connected directly or via a linker to the C-terminal of at least one of the TCR α chain, the TCR β chain, CD3 ε, CD3 γ, CD3 δ and CD3 ζ in which the natural endodomain is deleted; or in the complex of ii), the functional domain is connected directly or via a linker to the C-terminal of at least one of the TCR γ chain, the TCR δ chain, CD3 ε, CD3 γ, CD3 δ and CD3 ζ in which the natural endodomain is deleted.

5. The modified T cell receptor complex of claim 4, wherein the linker is (G₄S)n, where n represents an integer from 1 to 10, preferably n is 3.

6. The modified T cell receptor complex of any one of claims 1 to 5, wherein at least one functional domain, such as the endodomain of a costimulatory molecule, is connected to the C-terminal of one of the TCR α chain, the TCR β chain, CD3 ε, CD3 γ, CD3 δ and CD3 ζ in the complex of i); or
at least one functional domain is connected to the C-terminal of one of the TCR γ chain, the TCR δ chain, CD3 ε, CD3 γ, CD3 δ and CD3 ζ in the complex of ii).

7. The modified T cell receptor complex of claim 6, wherein at least one functional domain is connected to the C-terminal of the TCR α chain in the complex of i).

8. The modified T cell receptor complex of claim 7, wherein the natural endodomain of the TCR α chain is deleted.

9. The modified T cell receptor complex of claim 8, wherein the functional domain is connected directly or via a linker to the C-terminal of the TCR α chain in which the natural endodomain is deleted.

10. The modified T cell receptor complex of claim 9, wherein the linker is, for example, (G₄S)n, where n represents an integer from 1 to 10, preferably, n is 3.

11. The modified T cell receptor complex of claim 6, wherein at least one functional domain is connected to the C-terminal of TCR β chain in the complex of i).

12. The modified T cell receptor complex of claim 11, wherein the natural endodomain of the TCR β chain is deleted.

13. The modified T cell receptor complex of claim 12, wherein the functional domain is connected directly or via a linker to the C-terminal of the TCR β chain in which the natural endodomain is deleted.

14. The modified T cell receptor complex of claim 13, wherein the linker is, for example, (G₄S)n, where n represents an integer from 1 to 10, preferably, n is 3.

15. The modified T cell receptor complex of claim 6, wherein at least one functional domain is connected to the C-terminal of the TCR γ chain in the complex of ii).

16. The modified T cell receptor complex of claim 15, wherein the natural endodomain of the TCR γ chain is deleted.

17. The modified T cell receptor complex of claim 16, wherein the functional domain is connected directly or via a linker to the C-terminal of the TCR γ chain in which the natural endodomain is deleted.

18. The modified T cell receptor complex of claim 17, wherein the linker is, for example, (G₄S)n, where n represents an integer from 1 to 10, preferably, n is 3.

19. The modified T cell receptor complex of claim 6, wherein at least one functional domain is connected to the C-terminal of TCR δ chain in the complex of ii).

20. The modified T cell receptor complex of claim 19, wherein the natural endodomain of the TCR δ chain is deleted.

21. The modified T cell receptor complex of claim 20, wherein the functional domain is connected directly or via a linker to the C-terminal of the TCR δ chain in which the natural endodomain is deleted.

22. The modified T cell receptor complex of claim 21, wherein the linker is, for example, (G₄S)n, where n represents an integer from 1 to 10, preferably, n is 3.

23. The modified T cell receptor complex of any one of claims 1 to 5, wherein at least one functional domain is connected to the C-terminals of two of the TCR α chain, the TCR β chain, CD3 ε, CD3 γ, CD3 δ and CD3 ζ in the complex of i); or at least one functional domain is connected to the C-terminals of two of the TCR γ chain, the TCR δ chain, CD3 ε, CD3 γ, CD3 δ and CD3 ζ in the complex of ii).

24. The modified T cell receptor complex of any one of claims 1 to 5, wherein at least one functional domain is connected to the respective C-terminals of the TCR α chain and the TCR β chain in the complex of i).

25. The modified T cell receptor complex of claim 24, wherein the natural endodomain of each of the TCR α chain and TCR β chain is deleted.

26. The modified T cell receptor complex of claim 25, wherein the functional domain is connected directly or via a linker to the C-terminals of the TCR α chain and TCR β chain in which the natural endodomain is deleted each.

27. The modified T cell receptor complex of claim 26, wherein the linker is, for example, (G₄S)n, where n represents an integer from 1 to 10, preferably, n is 3.

28. The modified T cell receptor complex of claim 23, wherein at least one functional domain is connected to the respective C-terminals of the TCR γ chain and TCR δ chain in the complex of ii).

29. The modified T cell receptor complex of claim 28, wherein the natural endodomain of both the TCR γ chain and TCR δ chain is deleted.

30. The modified T cell receptor complex of claim 29, wherein the functional domain is connected directly or via a linker to the C-terminals of TCR γ chain and TCR δ chain in which the natural endodomain is deleted each.

31. The modified T cell receptor complex of claim 30, wherein the linker is, for example, (G₄S)n, where n represents an integer from 1 to 10, preferably, n is 3.

32. The modified T cell receptor complex of any one of claims 1 to 5, wherein the same or different functional domains are connected to the C-terminals of two of the TCR α chain, the TCR β chain, CD3 ε, CD3 γ, CD3 δ and CD3 ζ in the complex of i); or the same or different functional domains are connected to the C-terminals of two of the TCR γ chain, the TCR δ chain, CD3 ε, CD3 γ, CD3 δ and CD3 ζ in the complex of ii).

33. The modified T cell receptor complex of any one of claims 1 to 32, wherein 1, 2, 3, 4, 5, 6, 7, 8, 9, 10 or more functional domains are connected to the C-terminal of at least one of the TCR α chain, the TCR β chain, CD3 ε, CD3 γ, CD3 δ and CD3 ζ in the complex of i); or 1, 2, 3, 4, 5, 6, 7, 8, 9, 10 or more functional domains are connected to the C-terminal of at least one of the TCR γ chain, the TCR δ chain, CD3 ε, CD3 γ, CD3 δ and CD3 ζ in the complex of ii).

34. The modified T cell receptor complex of any one of claims 1 to 33, wherein the at least one functional domain is selected from the endodomain of a costimulatory molecule, such as CD40, OX40, ICOS, CD28, 4-1BB, CD27, and CD137, or the endodomain of a co-inhibitory molecule, such as T1M3, PD1, CTLA4, and LAG3, or the endodomain of a cytokine receptor such as an interleukin receptor (such as IL-2 receptor), an interferon receptor, a tumor necrosis factor superfamily receptor, a colony-stimulating factor receptor, a chemokine receptor, a growth factor receptor, or other membrane proteins, or the domain of an intracellular protein such as NIK.

35. The modified T cell receptor complex of any one of claims 1 to 34, wherein the endodomain of the costimulatory molecule is OX40 or ICOS, preferably OX40.

36. The modified T cell receptor complex of any one of claims 1 to 35, wherein the first constant region is a natural TCR α chain constant region, for example, a natural human TCR α chain constant region or a natural mouse TCR α chain constant region; or the first constant region is a natural TCR γ chain constant region, for example, a natural human TCR γ chain constant region or a natural mouse TCR γ chain constant region.

37. The modified T cell receptor complex of any one of claims 1 to 35, wherein the first constant region is a modified TCR α chain constant region or a modified TCR γ chain constant region.

38. The modified T cell receptor complex of claim 37, wherein the modified TCR α chain constant region is derived from a mouse TCR α chain constant region, in which the amino acid at position 48, such as threonine (T), is mutated to cysteine (C), as compared to the wild-type mouse TCR α chain constant region.

39. The modified T cell receptor complex of claim 37, wherein the modified TCR α chain constant region is derived from a mouse TCR α chain constant region, in which the amino acid at position 112, such as serine (S), is mutated to leucine (L), the amino acid at position 114, such as methionine (M), is mutated to isoleucine (I), and the amino acid at position 115, such as glycine (G), is mutated to valine(V), as compared to the wild-type mouse TCR α chain constant region.

40. The modified T cell receptor complex of claim 37, wherein the modified TCR α chain constant region is derived from a mouse TCR α chain constant region, in which the amino acid at position 6, such as E is substituted by D, K at position 13 is substituted by R, and amino acids at positions 15 to 18 are deleted, as compared to the wild-type mouse TCR α chain constant region.

41. The modified T cell receptor complex of claim 37, wherein the modified TCR α chain constant region is derived from a mouse TCR α chain constant region, in which the amino acid at position 48, such as threonine (T), is mutated to cysteine (C), the amino acid at position 112, such as serine (S), is mutated to leucine (L), the amino acid at position 114, such as methionine (M), is mutated to isoleucine (I), and the amino acid at position 115, such as glycine (G), is mutated to valine(V), as compared to the wild-type mouse TCR α chain constant region,

42. The modified T cell receptor complex of claim 37, wherein the modified TCR α chain constant region is derived from a mouse TCR α chain constant region, in which the amino acid at position 6, such as E, is substituted by D, K at position 13 is substituted by R, the amino acids at positions 15 to 18 are deleted, the amino acid at position 48, such as threonine (T), is mutated to cysteine (C), the amino acid at position 112, such as serine (S), is mutated to leucine (L), the amino acid at position 114, such as methionine (M), is mutated to isoleucine (I), and the amino acid at position 115, such as glycine (G), is mutated to valine(V), as compared to the wild-type mouse TCR α chain constant region.

43. The modified T cell receptor complex of any one of claims 1 to 35, wherein the first constant region comprises the nucleotide sequence shown in one of SEQ ID NOs: 1, 3, 5, 7, 8, 26, 41, 42 and 56.

44. The modified T cell receptor complex of any one of claims 1 to 43, wherein the second constant region is a natural TCR β chain constant region, for example, a natural human TCR β chain constant region or a natural mouse TCR β chain constant region; or wherein the second constant region is a natural TCR δ chain constant region, for example, a natural human TCR δ chain constant region or a natural mouse TCR δ chain constant region.

45. The modified T cell receptor complex of any one of claims 1 to 43, wherein the second constant region is a modified TCR β chain constant region, or a modified TCR δ chain constant region.

46. The modified T cell receptor complex of claim 45, wherein the modified TCR β chain constant region is derived from a mouse TCR β chain constant region, in which the amino acid at position 56, such as serine (S), is mutated to cysteine (C), as compared to the wild-type mouse TCR β chain constant region.

47. The modified T cell receptor complex of claim 45, wherein the modified TCR β chain constant region is derived from a mouse TCR β chain constant region, in which the amino acid at position 3, such as R, is substituted by K, the amino acid at position 6, such as T, is substituted by F, K at position 9 is substituted by E, S at position 11 is substituted by A, L at position 12 is substituted by V, and the amino acids at positions 17 and 21 to 25 are deleted, as compared to the wild-type mouse TCR β chain constant region.

48. The modified T cell receptor complex of claim 45, wherein the modified TCR β chain constant region is derived from the mouse TCR β chain constant region, in which the amino acid at position 56, such as serine (S), is mutated to cysteine (C), the amino acid at position 3, such as R, is substituted by K, the amino acid at position 6, such as T, is substituted by F, K at position 9 is substituted by E, S at position 11 is substituted by A, L at position 12 is substituted by V, and the amino acids at positions 17 and 21 to 25 are deleted, as compared to the wild-type mouse TCR β chain constant region.

49. The modified T cell receptor complex of any one of claims 1 to 43, wherein the modified TCR β chain constant region comprises the amino acid sequence shown in one of SEQ ID NOs: 2, 4, 6, 9, 27, 43 and 57.

50. The modified T cell receptor complex of any one of claims 1 to 49, wherein the antigen-binding region and the second antigen-binding region specifically bind to the target antigen independently or in combination.

51. The modified T cell receptor complex of claim 50, wherein the target antigen is a disease-associated antigen, preferably a cancer-associated antigen, such as a cancer-associated antigen selected from the group consisting of: phosphatidylinositol proteoglycan 3 (GPC3), CD16, CD64, CD78, CD96, CLL1, CD116, CD117, CD71, CD45, CD71, CD123, CD138, ErbB2 ( HER2/neu), carcinoembryonic antigen (CEA), epithelial cell adhesion molecule (EpCAM), epidermal growth factor receptor (EGFR), EGFR Variant III ( EGFRvIII), CD19, CD20, CD30, CD40, disialoganglioside GD2, ductal epithelial mucin, gp36, TAG-72, glycosphingolipids, glioma-associated antigen, β-human chorionic gonadotropin, α fetal globulin (AFP), exogenous lectin reactive AFP, thyroglobulin, RAGE-1, MN- CA IX, human telomerase reverse transcriptase, RU1, RU2 ( AS), intestinal carboxylesterase, mut hsp70- 2. M-CSF, prostase, prostate-specific antigen (PSA), PAP, NY-ESO-1, LAGA-1a, p53, Prostein, PSMA, survival and telomerase, prostate cancer tumor antigen-1 (PCTA-1), MAGE, ELF2M, neutrophil elastase, ephrin B2, CD22, insulin growth factor (IGF1)-I, IGF-II, IGFI receptor, mesothelin, major histocompatibility complex (MHC) molecule presenting tumor-specific peptide epitopes, 5T4, ROR1, Nkp30, NKG2D, tumor matrix antigen, extradomain A (EDA) and extradomain B (EDB) of fibronectin, A1 domain of tenascin-C (TnC A1), fibroblast associated protein (fap), CD3, CD4, CD8, CD24, CD25, CD33, CD34, CD133, CD138, Foxp3, B7-1 (CD80), B7-2 (CD86), GM-CSF, cytokine receptor, endothelial factor, major histocompatibility complex(MHC) molecules, BCMA (CD269, TNFRSF17), TNFRSF17 (UNIPROT Q02223), SLAMF7 (UNIPROT Q9NQ25), GPRC5D (UNIPROT Q9NZD1), FKBP11 (UNIPROT Q9NYL4), KAMP3, ITGA8 (UNIPROT P53708), and FCRL5 (UNIPROT Q68SN8).

52. The modified T cell receptor complex of any one of claims 1 to 51, wherein the first antigen-binding region comprises a heavy chain variable region of an antibody that specifically binds to a target antigen, and the second antigen binding region comprises a light chain variable region of the antibody; alternatively, the first antigen-binding region comprises a light chain variable region of an antibody that specifically binds to a target antigen, and the second antigen-binding region comprises a heavy chain variable region of the antibody.

53. The modified T cell receptor complex of any one of claims 1 to 52, wherein the first antigen-binding region comprises a single chain antibody or a single domain antibody that specifically binds to a target antigen; and/or the second antigen-binding region comprises a single chain antibody or a single domain antibody that specifically binds to a target antigen,
for example, the single chain antibody comprises a heavy chain variable region and a light chain variable region linked by a linker, such as (G₄S)n, where n represents an integer from 1 to 10, preferably n is 1 or 3.

54. The modified T cell receptor complex of claim 53, wherein the first antigen-binding region and the second antigen-binding region bind to the same target antigen.

55. The modified T cell receptor complex of claim 54, wherein the first antigen-binding region and the second antigen-binding region bind to different regions (e.g. different epitopes) of the same target antigen.

56. The modified T cell receptor complex of claim 54, wherein the first antigen-binding region and the second antigen-binding region bind to different target antigens.

57. The modified T cell receptor complex of any one of claims 1 to 56, wherein CD3y, CD38, CD3ε and/or CD3ζ are human-derived.

58. The modified T cell receptor complex of any one of claims 1 to 57, wherein the human CD3γ comprises the amino acid sequence shown in SEQ ID NO: 28.

59. The modified T cell receptor complex of any one of claims 1 to 58, wherein the human CD3δ comprises the amino acid sequence shown in SEQ ID NO: 29.

60. The modified T cell receptor complex of any one of claims 1 to 59, wherein the human CD3ε comprises the amino acid sequence shown in SEQ ID NO: 30.

61. The modified T cell receptor complex of any one of claims 1 to 60, wherein the CD3ζ comprises the amino acid sequence shown in SEQ ID NO: 31.

62. An isolated therapeutic immune cell comprising the modified T cell receptor complex of any one of claims 1 to 61.

63. The therapeutic immune cell of claim 62, wherein the therapeutic immune cell is a T cell or NK cell.

64. A pharmaceutical composition, comprising the therapeutic T cell of claim 62 or 63, and a pharmaceutically acceptable carrier.

65. Use of the therapeutic T cell of claim 62 or 63 or the pharmaceutical composition of claim 64 in the preparation of a drug for treating a disease, such as cancer, in a subject.
